# EUROPÄISCHE PATENTSCHRIFT

(11) **EP 2 916 885 B1**
(45) Veröffentlichungstag und Bekanntmachung des Hinweises auf die Patenterteilung: **30.11.2016**
(21) Anmeldenummer: 13780316.9
(22) Anmeldetag: 21.10.2013
(51) Int. Cl.: A61M 5/00, B65B 35/38, B65B 43/59, B65B 3/00, B65B 3/28, B65B 1/32, G01G 17/06, B01L 9/06, B65B 43/46, B65B 1/46, B65B 7/28, B65D 51/24, F26B 5/06, G01N 35/04

(54) **VERFAHREN UND VORRICHTUNG ZUR BEHANDLUNG ODER VERARBEITUNG VON BEHÄLTERN FÜR SUBSTANZEN FÜR MEDIZINISCHE, PHARMAZEUTISCHE ODER KOSMETISCHE ANWENDUNGEN**
DEVICE AND METHOD FOR TREATMENT OR PROCESSING OF CONTAINERS FOR SUBSTANCES FOR MEDICAL, PHARMACEUTICAL OR COSMETIC APPLICATIONS
DISPOSITIF ET PROCÉDÉ DESTINÉS AU TRAITEMENT OU MANIPULATION DES RÉCIPIENTS POUR LES SUBSTANCES POUR LES APPLICATIONS MÉDICALES, PHARMACEUTIQUES OU COSMÉTIQUES

(30) Priorität: 12.11.2012 DE 102012110866
(43) Veröffentlichungstag der Anmeldung: 16.09.2015
(60) Teilanmeldung: 15166614.6
(73) Patentinhaber: SCHOTT AG, 55122 Mainz (DE)
(72) Erfinder: WISSNER, Kai, D-69493 Hirschberg (DE); WASSENBERG, Jörn, D-55130 Main (DE); DEUTSCHLE, Fritz Gregor, 65185 Wiesbaden (DE); WANSEL, Alexander, D-65193 Wiesbaden (DE)
(74) Vertreter: 2K Patentanwälte Blasberg Kewitz & Reichel
(86) Internationale Anmeldenummer: PCT/EP2013/003164
(87) Internationale Veröffentlichungsnummer: WO 2014/072019

(56) Entgegenhaltungen:
- WO-A1-2011/110872
- WO-A2-2007/061987
- DE-A1- 10 028 823
- DE-A1- 10 242 118
- DE-A1-102004 035 061
- DE-A1-102005 014 116
- DE-A1-102010 060 308

## Beschreibung

### Gebiet der Erfindung

Die vorliegende Erfindung betrifft allgemein die gleichzeitige Halterung einer Mehrzahl von Behältern zur Aufbewahrung von Substanzen für medizinische, pharmazeutische oder kosmetische Anwendungen, insbesondere von Fläschchen (Vials), Ampullen oder Karpullen, und betrifft insbesondere ein Verfahren und eine Vorrichtung zur Behandlung oder Verarbeitung bzw. Prozessierung von Fläschchen an oder in einer Bearbeitungsstation, insbesondere während diese von einer Haltestruktur gehalten oder zumindest geführt sind, sowie eine Verstelleinrichtung zu diesem Zweck.

### Hintergrund der Erfindung

Als Behälter zur Aufbewahrung von und Lagerung von medizinischen, pharmazeutischen oder kosmetischen Präparaten mit Verabreichung in flüssiger Form, insbesondere in vordosierten Mengen, werden in großem Umfang Medikamentenbehälter, wie beispielsweise Fläschchen, Ampullen oder Karpullen, eingesetzt. Diese weisen generell eine zylindrische Form auf, können aus Kunststoffen oder aus Glas hergestellt werden und sind kostengünstig in großen Mengen erhältlich. Für eine möglichst wirtschaftliche Befüllung der Behälter unter sterilen Bedingungen werden in zunehmendem Maße Konzepte eingesetzt, bei denen die Behälter gleich beim Hersteller der Behälter in Transport- und Verpackungsbehälter steril verpackt werden, die bei einem Pharmaunternehmen dann unter sterilen Bedingungen, insbesondere in einem sog. Steriltunnel, ausgepackt und dann weiter verarbeitet werden.

Zu diesem Zweck sind aus dem Stand der Technik div. Transport- und Verpackungsbehälter bekannt, in denen gleichzeitig eine Mehrzahl von Medikamentenbehältern in einer regelmäßigen Anordnung, beispielsweise in einer Matrixanordnung entlang von Reihen und sich rechtwinklig dazu erstreckenden Spalten, angeordnet sind. Dies hat Vorteile bei der automatisierten Weiterverarbeitung der Behälter, da die Behälter an kontrollierten Positionen und in vorgegebener Anordnung an Bearbeitungsstationen übergeben werden können, beispielsweise an Prozessautomaten, Roboter oder dergleichen. Hierzu werden Haltestrukturen eingesetzt, in welchen gleichzeitig eine Mehrzahl von Behältern in einer vorbestimmten regelmäßigen Anordnung gehalten werden können. Zur Übergabe an eine Bearbeitungsstation braucht einfach nur der Transport- und Verpackungsbehälter geeignet positioniert und geöffnet zu werden. Die nachgeordnete Bearbeitungsstation weiß dann, in welcher Position und Anordnung die weiter zu verarbeitenden Behälter angeordnet sind.

Ein solcher Transport- und Verpackungsbehälter und ein entsprechendes Verpackungskonzept sind beispielsweise in der US 8,118,167 B2 offenbart. Die Weiterverarbeitung der Behälter erfolgt jedoch stets in der Weise, dass die die Haltestruktur aus dem Transport- und Verpackungsbehälter, die Behälter aus der Haltestruktur entnommen und vereinzelt werden und auf einer Fördereinrichtung, insbesondere einem Förderband, einzeln an die Bearbeitungsstationen übergeben und dort weiterverarbeitet werden. Dies begrenzt die erzielbare Geschwindigkeit bei der Weiterverarbeitung. Insbesondere bei der Vereinzelung der Behälter mit Hilfe von Zellenrädern oder dergleichen kommt es immer wieder dazu, dass einzelne Behälter unkontrolliert aneinanderstoßen, was zu einem unerwünschten Abrieb und in der Folge zu einer Verunreinigung des Behälterinnenraums oder der Prozessanlage sowie zu einer Beeinträchtigung des äußeren Erscheinungsbildes der Behälter führt, was unerwünscht ist.

US 8,100,263 B2 offenbart einen steril verpackbaren und transportierbaren Transport- und Verpackungsbehälter, in welchen eine plattenförmige Haltestruktur eingesetzt werden kann, in der eine Mehrzahl von Medikamentenbehältern in einer regelmäßigen Anordnung gehalten werden. Die einzelnen Medikamentenbehälter werden zunächst lose in Aufnahmen, die in der Haltestruktur ausgebildet sind, angeordnet. Anschließend wird die Haltestruktur in den Transport- und Verpackungsbehälter eingesetzt und dieser mit einem gasundurchlässigen Kunststoffschlauch umgeben. Beim anschließenden Evakuieren der so ausgebildeten Verpackungseinheit wird der Kunststoffschlauch aufgrund des in dem Schlauch vorherrschenden Unterdrucks in die Zwischenräume zwischen den Medikamentenbehältern hineingedrückt, was so einerseits zu einer Stabilisierung der Position der Medikamentenbehälter in der Haltestruktur führt und andererseits eine weitere unkontrollierte Kollision von benachbarten Medikamentenbehältern verhindert. Beim Evakuieren und beim Anschließenden Öffnen des Kunststoffschlauchs können jedoch die Medikamentenbehälter seitlich verrutschen, was den Automatisierungsaufwand zur Weiterverarbeitung der Medikamentenbehälter erhöht. Ferner können die Medikamentenbehälter nach dem Öffnen des Kunststoffschlauchs dennoch unkontrolliert kollidieren, was die vorgenannten Nachteile mit sich bringt. Die Medikamentenbehälter können nicht in dem Transport- oder Verpackungsbehälter oder in der Haltestruktur weiter verarbeitet werden, sondern müssen zunächst in der herkömmlichen Weise vereinzelt und an nachgeordnete Bearbeitungsstationen übergeben werden.

Weitere vergleichbare Transport- und Verpackungsbehälter und Haltestrukturen werden in WO 2011/135085 A1 und WO 2009/015862 A1 offenbart. Zur Weiterverarbeitung müssen die Medikamentenbehälter jedoch stets vereinzelt werden. Eine chargenweise Weiterverarbeitung der Medikamentenbehälter, während diese in einer plattenförmigen Haltestruktur, wie vorstehend ausgeführt, aufgenommen sind, ist nicht möglich.

Die Figuren 1 bis 4 der WO 2009/015862 A1 offenbaren eine Haltestruktur, bei der elastische Haltezungen fest gegen den verengten Halsabschnitt am oberen Ende der Fläschchen drücken, um die Fläschchen reibschlüssig zu fixieren. Die Haltestruktur ist somit nur sehr eingeschränkt für Fläschchen mit hohen Toleranzen oder anderen Außendurchmessern geeignet. Ferner können die Fläschchen in der Haltestruktur nicht spannungsfrei gehalten werden, was insbesondere bei der Prozessierung, beispielsweise in einem Gefriertrockenschrank, zu einer unerwünschten Aufwölbung der Haltestruktur führen kann. Die Fläschchen können auch nicht von oben her in die Öffnungen der Haltestruktur eingeführt werden.

Die DE 10 2005 014 116 A1 beschreibt den Transport einer Gruppe von Gebinden, z.B. Spritzen, Vials, etc., in einem als Halterung ausgebildetem Nest. Die Verarbeitung der Gebinde im Nest erfolgt in der Regel reihenweise, wobei eine Reihe in einem oder mehreren Takten bearbeitet werden kann. Ein nicht dargestellter Greifer kann an den Wänden der Gebinde mit einer Saugeinrichtung angreifen und eine Anzahl von Gebinden aus der Halterung heraus heben. Dieser Greifer bringt die Gebinde dann in eine Wiegestation mit mehreren Wägezellen und lässt sie dort los. Sie werden dann gewogen. Anschließend ergreift der Greifer die Gebinde wieder und hält sie unter die Füllnadeln der Füllstation. Die Gebinde dieser Reihe werden dann gemeinsam befüllt. Anschließend setzt der Greifer die Gebinde dieser Reihe wieder in die Wägezellen ab, lässt sie los, und nimmt sie nach dem zweiten Wiegen wieder auf. Er setzt sie dann in exakt die gleiche Reihe von zylindrischen Ansätzen bzw. Löchern, aus denen er sie entnommen hatte.

Bei den vorgenannten Haltestukturen wird der Außendurchmesser der Fläschchen quasi als Hilfskontur zur Fixierung der Fläschchen an der Haltestruktur verwendet. Derartige Haltestrukturen sind deshalb nicht flexibel genug für Fläschchen mit größeren Toleranzen und/oder anderen Außendurchmessern einsetzbar.

Jedenfalls ist ein unmittelbarer Kontakt der Böden der Medikamentenbehälter, insbesondere der Böden von Fläschchen, bei den herkömmlichen Haltestrukturen nicht möglich. Dies erschwert jedoch die Weiterverarbeitung der Medikamentenbehälter insbesondere dann, wenn deren Inhalt einer Gefriertrocknung (auch als Lyophilisation oder Sublimationstrocknung bezeichnet) unterzogen werden soll. Ferner ist eine Weiterverarbeitung der Medikamentenbehälter unmittelbar in den Haltestrukturen nicht möglich, da diese dort entweder starr gehalten werden oder für die Weiterverarbeitung nicht in ausreichendem Maß zugänglich sind, weshalb die Medikamentenbehälter für eine Weiteverarbeitung herkömmlich stets aus den Haltestrukturen entnommen werden müssen, was zeitaufwendig und teuer ist.

### Zusammenfassung der Erfindung

Aufgabe der vorliegenden Erfindung ist es, ein einfaches und zuverlässiges Verfahren zur Behandlung oder Verarbeitung bzw. Prozessierung von Behältern, insbesondere Fläschchen, an oder in einer Bearbeitungsstation bereitzustellen, insbesondere unter Verwendung einer Haltestruktur zum gleichzeitigen Halten einer Mehrzahl von Behältern. Dabei sollen die Behälter einfach und zuverlässig gehalten sowie kostengünstig steril verpackt, wieder entpackt und weiterverarbeitet werden können.

Diese Aufgaben werden gemäß der vorliegenden Erfindung durch ein Verfahren nach Anspruch 1, durch eine Vorrichtung nach Anspruch 12 und durch eine Verwendung nach Anspruch 17 gelöst. Weitere vorteilhafte Ausführungsformen sind Gegenstand der rückbezogenen Unteransprüche.

Somit wird ein Verfahren zur Behandlung oder Verarbeitung von Behältern bereitgestellt, die zur Aufbewahrung von Substanzen für medizinische, pharmazeutische oder kosmetische Anwendungen dienen oder diese enthalten, wobei die Behälter mittels einer Fördereinrichtung zur Behandlung oder Verarbeitung automatisiert an zumindest einer Prozessstation vorbeigeführt werden oder diese durchlaufen. Bei dem Verfahren werden eine Mehrzahl von Behältern, insbesondere Fläschchen (vials), von der Fördereinrichtung gefördert, während diese an einem Träger bzw. einer Haltestruktur, wie nachfolgend beispielhaft beschrieben, gemeinsam in einer regelmäßigen Anordnung gehalten werden, werden die an dem Träger gehaltenen Behälter zur Behandlung oder Verarbeitung an oder in der jeweiligen Prozessstation in eine angehobene Position verschoben und werden die Behälter nach der Behandlung oder Verarbeitung abgesenkt, um an dem Träger erneut in der regelmäßigen Anordnung gehalten zu werden.

Somit kann erfindungsgemäß auf die Behälter ausschließlich von deren Unterseite und/oder Seitenfläche eingewirkt werden, um eine gewünschte axiale Verstellung zur Bearbeitung oder Verarbeitung der Behälter und deren Inhalte zu gewährleisten. Somit kann die Gefahr des Eindringens von Verunreinigungen von oben her in das Innere der Behälter erfindungsgemäß minimiert werden. Da die Behälter beim Einwirken auf deren Unterseite und/oder Seitenfläche auch zuverlässiger geführt und verstellt werden können, eignet sich das erfindungsgemäße Verfahren insbesondere auch zur Ausführung in sterilen Prozessumgebungen, beispielsweise in einem Steriltunnel oder dergleichen, wo Wartungs- und Montagearbeiten einen Betrieb der Anlage unterbrechen würden und somit so weit als möglich vermieden werden können.

Weil die Behälter gemeinsam an dem Träger in einer vorbestimmten regelmäßigen Anordnung gehalten sind, ist der Aufwand zur Automatisierung des Verfahrens geringer. Insbesondere können auch eine Mehrzahl von Behältern gleichzeitig bearbeitet oder verarbeitet werden, insbesondere reihen- oder chargenweise. Zum Anheben der Behälter in die angehobene Stellung genügt erfindungsgemäß ein berührender Kontakt einer Vertikal-Verstelleinrichtung mit der Unterseite und/oder Seitenfläche der Behälter, um die Behälter mechanisch zu verschieben. Das Absenken der Behälter in die Ausgangsstellung erfolgt dabei durch Saugen, also mittels eines Unterdrucks.

Gemäß einer weiteren Ausführungsform verhindert die Vertikal-Verstelleinrichtung eine seitliche Ausweichbewegung der Behälter zumindest beim Anheben der Behälter in die angehobene Position. Dies kann insbesondere dadurch realisiert werden, dass die Vertikal-Verstelleinrichtung zum Anheben der Behälter ausschließlich eine Bewegung exakt in vertikale Richtung ausführt (was durch eine einfache Geradführung der Vertikal-Verstelleinrichtung realisiert werden kann), dabei jedoch die Behälter so fixiert, beispielsweise durch Greifen oder Klemmen des Bodenbereichs und/oder der Seitenfläche der Behälter, dass ein Verrutschen der Behälter auf der Vertikal-Verstelleinrichtung verhindert ist.

Gemäß einer weiteren Ausführungsform umgreift die Vertikal-Verstelleinrichtung zum Anheben der Behälter einen unteren Rand der Behälter vorübergehend, was beispielsweise durch einfache mechanische Greifer oder durch eine pneumatisch betriebene Manschette bewirkt werden kann.

Gemäß einer weiteren Ausführungsform wird der untere Rand der jeweiligen Behälter während des Anhebens mittels einer Mehrzahl von Haltearmen umgriffen. Da diese Haltearme normalerweise seitlich von der Vertikal-Verstelleinrichtung abragen, sind Öffnungen oder Aufnahmen in dem Träger, in welchem die Behälter in deren Ausgangsposition an dem Träger gehalten werden und die von der Vertikal-Verstelleinrichtung beim Anheben der Behälter in die angehobene Position durchgriffen werden, bevorzugt korrespondierend ausgebildet. Insbesondere können diese korrespondierende Aussparungen aufweisen, die auch einer weiteren Zentrierung und Führung der Vertikal-Verstelleinrichtung beim Anheben der Behälter dienen können.

Gemäß einer weiteren Ausführungsform weist die die Vertikal-Verstelleinrichtung eine Mehrzahl von Hubstangen auf, die entlang einer Linie fluchtend angeordnet sind und eine Reihe von Behältern gleichzeitig in die angehobene Position verschieben können. Somit ist eine chargen- oder reihenweise Verarbeitung oder Bearbeitung der Behälter in der angehobenen Position möglich. Die Hubstangen können dabei mechanisch, pneumatisch oder hydraulisch verstellt werden. Eine so ausgelegte Vertikal-Verstelleinrichtung kann insbesondere innerhalb der Prozessstation angeordnet sein, also insbesondere auch in einer sterilen Prozessumgebung.

Gemäß einer weiteren Ausführungsform weist das Verfahren weiterhin eine Relativ-Positionierung des Trägers bzw. der Haltestruktur mit den daran gehaltenen Behältern und der Vertikal-Verstelleinrichtung auf. Hierzu erfolgt eine Relativ-Positionierung des Trägers bzw. der Haltestruktur und/oder der Vertikal-Verstelleinrichtung, beispielsweise mit Hilfe von formschlüssig zusammenwirkenden, korrespondierend zueinander ausgebildeten Elementen.

Gemäß der Erfindung werden die Behälter zum Absenken mittels eines auf einen Bodenbereich der Behälter einwirkenden Unterdrucks an den Träger zurückgezogen. Somit wird während des gesamten Verfahrens ausschließlich von unten her auf die Behälter eingewirkt, was die Gefahr des Eindringens von Verunreinigungen in die Behälter weitestgehend minimiert.

Gemäß einer weiteren Ausführungsform werden die Behälter zum Absenken seitlich geklemmt, um nach der Behandlung oder Verarbeitung in die regelmäßige Anordnung an dem Träger zurückgezogen zu werden.

Gemäß einer weiteren Ausführungsform werden die Behälter zum Absenken nach unten gedrückt. Dies kann beispielsweise mit Hilfe von Greifern realisiert werden, die auf die Seitenwände oder den verschmälerten Nackenabschnitt der Behälter einwirken.

Gemäß einer weiteren Ausführungsform sind an dem Träger, der bevorzugt flächig, insbesondere rechteckförmig ausgebildet ist, als Haltemittel zumindest zwei Haltezungen vorgesehen, die am Rand einer jeweiligen Öffnung oder Aufnahme vorgesehen sind und von einer Oberseite des Trägers abragen, um den jeweiligen Behälter in der Öffnung oder Aufnahme zu halten. Dabei sind die Haltezungen erfindungsgemäß so ausgelegt, dass diese beim Einführen der Behälter in die Öffnungen oder Aufnahmen elastisch weggeschwenkt oder weggeklappt werden, und ferner so auf die Behälter abgestimmt sind, dass diese mit radialem Spiel von den Haltezungen gehalten sind. Das radiale Spiel ermöglicht, dass Behälter mit unterschiedlichen radialen Toleranzen und/oder Außenabmessungen von derselben Haltestruktur zuverlässig gehalten werden können. Das radiale Spiel ist zweckmäßig so ausgelegt und auf die Außenkontur und -abmessung der Behälter abgestimmt, dass niemals gleichzeitig sämtliche Haltezungen den verengten Halsabschnitt am oberen Ende der Behälter, insbesondere Fläschchen, berühren. Gleichzeitig verhindert das radiale Spiel auch ein unerwünschtes Verspannen oder gar Aufwölben des Trägers beim Halten von Behältern mit unterschiedlichen radialen Toleranzen und/oder Außenabmessungen, was erhebliche Vorteile insbesondere bei der gleichzeitigen Prozessierung einer Mehrzahl von Behältern, während diese von der Haltestruktur gehalten sind, bietet, beispielsweise bei der Gefriertrocknung bei Prozessierung bei sehr niedrigen Temperaturen.

Selbst wenn sich der Träger dennoch bei der Prozessierung verziehen oder aufwölben sollte, kann dennoch ein gleichmäßiger Bodenkontakt zu sämtlichen von der Haltestruktur gehaltenen Behälter realisiert werden, insbesondere wenn diese ergänzend mit einem ausreichendem axialen Spiel von den Haltezungen an der Haltestruktur gehalten sind, da das axiale Spiel zusätzlich auch einen Längentoleranzausgleich ermöglicht.

Die Haltezungen sind dabei ausreichend elastisch ausgebildet oder gelagert, sodass die Behälter axial, d.h. in Richtung der Längsachse der Behälter und senkrecht zur Ebene des Trägers, von der Ober- oder Unterseite des Trägers her in die Öffnungen oder Aufnahmen eingeschoben werden können, insbesondere unter elastischer Verformung der Haltezungen, beispielsweise unter Wegbiegen derselben. Die Bestückung des Trägers mit Behältern kann somit einfach automatisiert werden, was durch eine regelmäßige Anordnung der Öffnungen oder Aufnahmen, bevorzugt in einer zweidimensionalen Matrix, noch weiter begünstigt wird.

Als bevorzugte Stelle, an der die Behälter an den Haltezungen gehalten oder abgestützt sind, hat sich die Unterseite eines verbreiterten oberen Randabschnittes der Behälter erwiesen, wie diese insbesondere bei Fläschchen typischerweise als sog. Rollrand oder Schulter vorgesehen sind. In diesem Bereich steht eine Abstütz- oder Lagerfläche zum Halten oder Abstützen der Behälter mit einer ausreichenden Erstreckung in Radialrichtung der Öffnungen oder Aufnahmen zur Verfügung, um das vorgenannte radiale Spiel bei der Halterung der Behälter ohne Weiteres zu realisieren.

Weil die Behälter in den Öffnungen oder Aufnahmen mit sehr geringem Kraftaufwand angehoben oder bewegt, beispielsweise gedreht, werden können, können diese, während diese sich in der Haltestruktur befinden und von dieser gehalten oder zumindest geführt werden, ohne weiteres bearbeitet werden. Als besonders vorteilhaft hat sich diese Art der Halterung z.B. beim Verschließen der Behälter durch Bördeln eines Metalldeckels erwiesen. Die hierzu erforderlichen Vorgänge können an dem Metalldeckel ausgeführt werden, während der Behälter in der Öffnung oder Aufnahme der Haltestruktur gehalten oder zumindest geführt ist. Als besonders vorteilhaft hat sich diese Art der Halterung auch bei der Prozessierung von Behältern erwiesen, während diese in der Haltestruktur gehalten bzw. aufgenommen sind. Beispielsweise können die Haltestrukturen mit den darin aufgenommenen bzw. gehaltenen Behältern in einen Gefriertrockenschrank eingebracht werden. Aufgrund der Halterung der Behälter mit einem gewissen Spiel in den Haltestrukturen kann gewährleistet werden, dass die Böden von sämtlichen Behältern auf einer kühlenden Unterlage, beispielsweise einem Kühlfinger des Gefriertrockenschranks, gleichmäßig aufliegen. Oder die Behälter können ohne größeren Kraftaufwand in den Öffnungen oder Aufnahmen der Haltestruktur angehoben und zur Prozessierung gehandhabt werden.

Gemäß einer weiteren Ausführungsform werden die Behälter vollständig aus Öffnungen oder Aufnahmen, die in dem Träger vorgesehen sind und die regelmäßige Anordnung vorgeben, herausgehoben, um an oder in der Prozessstation behandelt oder verarbeitet zu werden. Die Behälter sind also vollständig von den Haltemitteln der Haltestruktur freigegeben, was eine anderweitige Positionierung der Behälter bedingt, um ein seitliches Verrutschen der Behälter bei der Bearbeitung oder Verarbeitung der Behälter zu unterbinden. Diese Positionierung kann insbesondere durch geeignete Ausgestaltung der Vertikal-Verstelleinrichtung realisiert werden, beispielsweise durch Umgreifen des unteren Rands der Behälter durch Abschnitte der Vertikal-Verstelleinrichtung.

Gemäß einer bevorzugten Ausführungsform sind die Haltezungen als elastische Haltezungen ausgebildet, verfügen jedoch über eine ausreichende Elastizität, um beim Einführen der Behälter in die Öffnungen oder Aufnahmen ausreichend elastisch weggeschwenkt oder weggeklappt zu werden, um den Behältern den Weg in die Öffnungen oder Aufnahmen freizugeben. Dies lässt sich durch geeignete Dimensionierung, Materialwahl und Auslegung der Materialstärke der Haltezungen ohne weiteres erreichen. Bevorzugt sind die Haltezungen somit aus einem Kunststoff ausgebildet.

Gemäß einer Ausführungsform sind die Haltezungen elastisch gegen eine Haltestellung vorgespannt, bevorzugt mittels eines elastischen Rückstellelements, beispielsweise einer Rückstellfeder oder eines Kunststoffblättchens oder elastischen Kunststoffgebildes, das mit der zugeordneten Haltezunge geeignet zusammenwirkt und auf der Oberseite des Trägers vorgesehen oder ausgebildet ist.

Gemäß einer Ausführungsform sind die Haltezungen so auf die Behälter abgestimmt, dass die Behälter mit einem verbreiterten Rand, der an einem oberen Ende der Behälter ausgebildet ist, also insbesondere mit dem vorgenannten Rollrand, lose auf Oberseiten der Haltezungen aufliegen. Die Behälter können somit ohne Widerstand nach oben wieder aus den Öffnungen oder Aufnahmen entnommen werden.

Gemäß einer Ausführungsform umgreifen die Haltezungen den verbreiterten Rand dergestalt, dass die Behälter mit radialem Spiel oder mit radialem und axialem Spiel von den Haltezungen gehalten sind. Auf diese Weise können die Behälter in den Öffnungen oder Aufnahmen axial verliersicher gehalten werden. Zum Entnehmen der Behälter aus den Öffnungen oder Aufnahmen brauchen die Haltezungen nur wiederum in der Art, wie beim Einführen der Behälter, zurückgeschwenkt oder zurück geklappt werden.

Gemäß einer Ausführungsform sind die Haltezungen so verteilt auf der Oberseite des Trägers angeordnet, dass diese beim Wegschwenken oder Wegklappen einander nicht unmittelbar berühren und eine unmittelbar benachbarte Öffnung oder Aufnahme nicht versperren. Somit kann die Packungsdichte der Behälter an dem Träger noch weiter erhöht werden. Insbesondere sind die Haltezungen so ausgelegt, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer Ausführungsform sind am oberen Ende der Haltezungen Einführschrägen ausgebildet, welche jeweils in eine von den Haltezungen radial einwärts vorstehende Haltenase zum Halten der Behälter übergehen. Die Behälter können somit noch einfacher und kraftärmer in die Öffnungen oder Aufnahmen eingeführt werden. Insbesondere geraten beim Einführen der Behälter von oben her in die Öffnungen oder Aufnahmen zunächst die Böden bzw. unteren Enden der Behälter in Anlage zu den Einführschrägen. Beim weiteren Einführen der Behälter gleitet das untere Ende bzw. der Boden der Behälter entlang den Einführschrägen abwärts und spreizt dabei die Haltezungen auseinander oder klappt bzw. schwenkt diese zurück. Beim weiteren Einführen der Behälter gerät schließlich die zylindrische Seitenwand in Anlage zu den Haltenasen und gleitet an diesen entlang, solange bis schließlich die Unterseite des vorgenannten Rollrands lose auf den Haltenasen der Haltezungen aufliegt.

Gemäß einer Ausführungsform sind die einer jeweiligen Öffnung oder Aufnahme zugeordneten Haltezungen oder deren Einführschrägen gleichsinnig und um einen Winkel kleiner 90° verdrillt ausgebildet, sodass die Haltezungen beim Einführen der Behälter von der Oberseite des Trägers her in die Öffnungen oder Aufnahmen, in Draufsicht betrachtet, radial und mit einer Bewegungskomponente in Umfangsrichtung weggeschwenkt oder weggeklappt werden. Dies kann je nach der Anordnung und Verteilung der Haltezungen auf dem Träger ermöglichen, dass unmittelbar benachbarte Haltezungen, wenn diese beim Einführen der Behälter in die zugeordneten Öffnungen oder Aufnahmen zum Träger hin geschwenkt oder geklappt werden, einander nicht berühren.

Gemäß einer weiteren Ausführungsform sind die Öffnungen oder Aufnahmen auf einer der Oberseite gegenüberliegenden Unterseite des Trägers zumindest abschnittsweise von einer jeweiligen Seitenwand begrenzt, um eine Berührung von Behältern in unmittelbar benachbarten Öffnungen oder Aufnahmen zu verhindern, wobei die Seitenwände ganz besonders bevorzugt so ausgebildet sind, dass die Behälter von der Unterseite des Trägers her frei zugänglich sind. Die Seitenwände von benachbarten Öffnungen oder Aufnahmen sind bevorzugt miteinander verbunden, was vorteilhaft zu einer weiteren Versteifung des Trägers beiträgt. Bevorzugt sind die Seitenwände einstückig mit dem Träger ausgebildet, was beispielsweise in Kunststoff-Spritzgusstechnik einfach realisiert werden kann.

Die Böden bzw. unteren Enden der in den Öffnungen oder Aufnahmen aufgenommenen Behälter stehen bevorzugt von den unteren Enden der Seitenwände vor, sodass die Böden der Behälter von der Unterseite des Trägers her frei zugänglich sind. Dies ermöglicht, dass die Behälter prozessiert werden können, während diese an dem Träger in den Öffnungen oder Aufnahmen gehalten sind, wie weiter unten ausgeführt.

Gemäß einer weiteren Ausführungsform sind die Haltezungen mit dem Träger einstückig ausgebildet, was eine eine kostengünstige Herstellung ermöglicht, beispielsweise durch Spritzgießen aus einem Kunststoff. Die elastischen Haltezungen ragen dabei bogenförmig von der Oberseite des Trägers ab und, in Draufsicht betrachtet, bevorzugt ein wenig in die zugeordnete Öffnung oder Aufnahme hinein. So können die Behälter insbesondere im Bereich eines verengten Halsabschnitts und nahe dem oberen offenen Ende eines Behälters bzw. Fläschchens gehalten werden, wie nachfolgend näher ausgeführt. Die bogenförmige Ausbildung der Haltezungen erleichtert das Einschieben und wieder Herausziehen der Behälter in die bzw. aus den Öffnungen oder Aufnahmen der Trägers.

Gemäß einer weiteren Ausführungsform sind die einer Öffnung bzw. Aufnahme zugeordneten elastischen Haltezungen jeweils bezüglich einer Mittellinie der Öffnung oder Aufnahme symmetrisch angeordnet und ausgebildet. Die Behälter werden somit automatisch zentriert in den jeweiligen Öffnungen oder Aufnahmen des Trägers gehalten. Die Symmetrie verhindert auch ein versehentliches Verkippen oder Verkanten der Behälter beim Einführen oder Halten in den Öffnungen oder Aufnahmen des Trägers.

Gemäß einer weiteren Ausführungsform bilden die elastischen Haltezungen jeweils eine Dreipunkt-Lagerung zum Halten der Behälter in der jeweiligen Öffnung oder Aufnahme des Trägers aus, wodurch eine automatische Zentrierung der Behälter in den zugeordneten Öffnungen oder Aufnahmen und eine sehr präzise und stabile Festlegung der Position der Behälter an dem Träger noch mehr begünstigt ist.

Gemäß einer weiteren Ausführungsform sind die Seitenwände in einer regelmäßigen hexagonalen Anordnung auf der Unterseite und/oder Oberseite des Trägers verteilt angeordnet. Insgesamt wird so eine wabenartige Struktur ausgebildet, die vorteilhaft zu einer weiteren Versteifung des Trägers beitragen kann. Dabei sind die Seitenwände von benachbarten Öffnungen oder Aufnahmen bevorzugt miteinander verbunden.

Gemäß einer weiteren Ausführungsform sind die Seitenwände einer jeweiligen Öffnung oder Aufnahme jeweils umlaufend ausgebildet und bilden eine hexagonale Wabenstruktur auf der Unterseite des Trägers aus. Die Seitenwände von unmittelbar benachbarten Öffnungen oder Aufnahmen laufen dabei in den Eckbereichen der Öffnungen oder Aufnahmen zusammen und sind miteinander verbunden oder einstückig ausgebildet, was in einer weiteren Versteifung des Trägers resultiert.

Gemäß einer bevorzugten weiteren Ausführungsform ragen von einem Verbindungsbereich der Seitenwände jeweils drei Haltezungen in einer Anordnung mit dreizähliger Symmetrie in die jeweils zugeordneten Öffnungen oder Aufnahmen hinein, sodass in dem Verbindungsbereich vorteilhaft eine Kräfteaufhebung erzielt werden kann. Insgesamt kann der Träger so die Mehrzahl von Behältern spannungsarm halten.

Gemäß einer weiteren Ausführungsform sind die Seitenwände einer jeweiligen Öffnung oder Aufnahme jeweils kreisförmig und umlaufend ausgebildet. Bevorzugt sind die Seitenwände von unmittelbar benachbarten Öffnungen oder Aufnahmen miteinander verbunden oder einstückig ausgebildet, was ebenfalls zu einer weiteren Versteifung des Trägers führt.

Gemäß einer weiteren Ausführungsform sind die Öffnungen oder Aufnahmen in einer regelmäßigen Anordnung von Reihen und Spalten auf dem Träger verteilt angeordnet, wobei die jeweils Reihen und Spalten jeweils regelmäßig versetzt zueinander angeordnet sind und eine wiederkehrende Anordnung ausbilden. Diese regelmäßige Anordnung ist für eine automatisierte Behandlung der Behälter vorteilhaft.

Gemäß einer weiteren Ausführungsform kann die Grundfläche der Haltestruktur durch Abnehmen oder Wegklappen der an dem Rand ausgebildeten abnehmbaren oder wegschwenkbaren Elemente reduziert werden. Dies ermöglicht eine höhere Packungsdichte während der Prozessierung der in den Haltestrukturen aufgenommenen Behälter, beispielsweise in einem Steriltunnel oder einem Gefriertrockenschrank.

Gemäß einer weiteren Ausführungsform kann durch den formschlüssigen Eingriff von Aussparungen und/oder Vorsprünge, die entweder an den vorgenannten abnehmbaren oder wegschwenkbaren Elementen des Trägers oder unmittelbar im Rand des Trägers ausgebildet sind, mit korrespondierend ausgebildeten Vorsprüngen und/oder Aussparungen eines unmittelbar benachbarten Trägers eine hohe Packungsdichte und gleichzeitig eine gegenseitige Stabilisierung der Positionen der Träger realisiert werden.

Gemäß einem weiteren Gesichtspunkt der vorliegenden Erfindung können unmittelbar benachbarte Haltestrukturen so miteinander unmittelbar verbunden werden, dass diese relativ zueinander in der Längsrichtung und/oder in der Querrichtung unverschieblich sind. Mit anderen Worten: die jeweils unmittelbar benachbarten Haltestrukturen können gemeinsam, quasi als Einheit, bestehend aus mehreren (zumindest zwei) Haltestrukturen gehandhabt werden, ohne dass sich deren Lage relativ zueinander wesentlich verändern würde. Zu diesem Zweck wird erfindungsgemäß eine lösbare, vorübergehende Verbindung der unmittelbar benachbarten Haltestrukturen gewählt, wobei grundsätzlich beliebige form- oder kraftschlüssige Verbindungstechniken eingesetzt werden können, solange die durch die Verbindung erzielbare Verbindungskraft größer ist als die üblicherweise bei der Handhabung oder Prozessierung der Haltestrukturen auftretenden Kräfte, die danach trachten, die unmittelbar benachbarten Haltestrukturen wieder voneinander zu trennen.

Ein weiterer Gesichtspunkt der vorliegenden Erfindung betrifft ein Verfahren zur Behandlung oder Verarbeitung von Behältern, die zur Aufbewahrung von Substanzen für medizinische, pharmazeutische oder kosmetische Anwendungen dienen oder diese enthalten, bei dem die Behälter in noch einfacherer und wirtschaftlicher Weise gewogen werden können. Dabei werden eine Mehrzahl von Behältern von der Fördereinrichtung gefördert werden, während diese an einem Träger, wie hierin beschrieben, gemeinsam in einer regelmäßigen Anordnung gehalten werden, und werden die an dem Träger gehaltenen Behälter zur Behandlung oder Verarbeitung an oder in der jeweiligen Prozessstation in eine angehobene Position angehoben. Erfindungsgemäß werden die Behälter mittels einer Wiegeeinrichtung zwischen zwei Verfahrensschritten gewogen, insbesondere während diese an einem Träger gehalten oder in dessen Öffnungen, Aufnahmen oder Haltemitteln zumindest geführt sind, wie nachfolgend beschrieben.

Zu diesem Zweck ist die Wiegeeinrichtung bevorzugt in eine zum Anheben und Absenken der Behälter verwendete Vertikal-Verstelleinrichtung integriert, wie nachfolgend beschrieben.

Ein weiterer Gesichtspunkt der vorliegenden Erfindung betrifft eine Vorrichtung, die ausgelegt ist, um ein Verfahren, wie hierin beschrieben, zur Behandlung oder Verarbeitung von Behältern, die zur Aufbewahrung von Substanzen für medizinische, pharmazeutische oder kosmetische Anwendungen dienen oder diese enthalten, auszuführen.

Ein weiterer Gesichtspunkt der vorliegenden Erfindung betrifft eine Vertikal-Verstelleinrichtung für eine solche Vorrichtung, wie nachfolgend beschrieben.

### Figurenübersicht

Nachfolgend wird die Erfindung in beispielhafter Weise und unter Bezugnahme auf die beigefügten Zeichnungen beschrieben werden, woraus sich weitere Merkmale, Vorteile und zu lösende Aufgaben ergeben werden. Es zeigen:
- Fig. 1a und 1b: in einer perspektivischen Draufsicht und einer Draufsicht eine Haltestruktur gemäß einer ersten Ausführungsform zur Verwendung bei einem Verfahren gemäß der vorliegenden Erfindung;
- Fig. 1c: einen Teilschnitt der Haltestruktur gemäß A-A von Fig. 1b;
- Fig. 1d: einen stark vergrößerten Teilschnitt in dem Einsatz, der in der Fig. 1c dargestellt ist;
- Fig. 1e: in dem stark vergrößerten Teilschnitt nach der Fig. 1d die Halterung eines Behälters in einer der Öffnungen einer Haltestruktur gemäß der der ersten Ausführungsform;
- Fig. 1f: eine Variante der Haltestruktur gemäß der Fig. 1a, mit Vorsprüngen und Aussparungen an den abnehmbaren oder wegschwenkbaren Elementen die einer weiteren Erhöhung der Packungsdichte der Haltestruktur dienen;
- Fig. 1g: eine Haltestruktur gemäß einer weiteren Variante nach der Fig. 1a in einer perspektivischen Draufsicht;
- Fig. 1h: in einer schematischen Draufsicht die Kopplung zweier unmittelbar benachbarter Haltestrukturen gemäß der Fig. 1g;
- Fig. 2a: in einem perspektivischen Teilschnitt und in Draufsicht einen Transport- oder Verpackungsbehälter mit einer darin aufgenommenen Haltestruktur gemäß einer zweiten Ausführungsform zur Verwendung bei einem Verfahren gemäß der vorliegenden Erfindung und mit von dieser gehaltenen Behältern;
- Fig. 2b: den Transport- oder Verpackungsbehälter gemäß der Fig. 2a in einem Teilschnitt und in Draufsicht;
- Fig. 2c: in zwei vergrößerten Teilschnitten die Halterung von Behältern in der Haltestruktur gemäß der zweiten Ausführungsform sowie Details davon;
- Fig. 2d: in einer perspektivischen Draufsicht die Haltestruktur gemäß der Fig. 2a ohne Behälter;
- Fig. 2e: in einer perspektivischen Unteransicht die Haltestruktur gemäß der Fig. 2a ohne Behälter;
- Fig. 2f: einen weiteren Teilschnitt der Haltestruktur gemäß der Fig. 2a ohne Behälter;
- Fig. 2g: in einem stark vergrößerten Teilschnitt die Halterung eines Behälters in einer Haltestruktur gemäß einer weiteren Ausführungsform der vorliegenden Erfindung;
- Fig. 2h: eine stark vergrößerte Draufsicht auf eine Einführschräge einer Haltezunge gemäß einer Variante zu der Haltestruktur gemäß der Fig. 2a;
- Fig. 2i: eine weitere Variante von Haltezungen für eine Haltestruktur gemäß der Fig. 2a;
- Fig. 2j: einen Teilschnitt durch eine weitere Variante einer Haltestruktur gemäß der Fig. 2a mit darin gehaltenem Behälter;
- Fig. 2k und 2l: weitere Varianten einer Haltestruktur zur Verwendung bei einem Verfahren gemäß der vorliegenden Erfindung;
- Fig. 2m bis 2o: in einer Bewegungssequenz das Einführen eines Behälters in eine Haltestruktur gemäß einer weiteren Variante;
- Fig. 3abis 3c: eine Haltestruktur gemäß einer weiteren Ausführungsform zur Verwendung bei einem Verfahren gemäß der vorliegenden Erfindung in einer perspektivischen Draufsicht, in einer Draufsicht und in einer Schnittansicht;
- Fig. 3d: einen Teilschnitt durch die Haltestruktur gemäß der Fig. 3a;
- Fig. 3e: in einer stark vergrößerten Teildraufsicht die Verhakung von ineinander eingreifenden Vorsprüngen und Aussparungen an den Rändern zweier Haltestrukturen gemäß der Fig. 3a;
- Fig. 3f: einen Querschnitt entlang A-A gemäß der Fig. 3e;
- Fig. 4a: einen Ausschnitt aus einer Bearbeitungsstation zur Bearbeitung oder Prozessierung von Fläschchen gemäß der vorliegenden Erfindung, während die Fläschchen in einer angehobenen Stellung in einer Haltestruktur gemäß der Fig. 2d gehalten sind;
- Fig. 4b bis 4g: in schematischen Seitenansichten oder Teilschnitten eine Sequenz von Prozessschritten bei einem Verfahren gemäß der vorliegenden Erfindung in der Bearbeitungsstation gemäß der Fig. 4a, wobei Behälter ausgehend von einer Ausgangsstellung (Fig. 4b) in eine angehobene Stellung überführt werden (Fig. 4g);
- Fig. 5a bis 5f: eine entsprechende Sequenz von Prozessschritten gemäß einer weiteren Variante des Verfahrens gemäß der vorliegenden Erfindung;
- Fig. 6a: in einer Seitenansicht das Zusammenwirken eines Stößels der Bearbeitungsstation gemäß der Fig. 4a und eines von diesem gehaltenen Fläschchens;
- Fig. 6b: den Stößel gemäß der Fig. 6a vor seinem Eingriff mit dem Fläschchen, wobei der in dem Stößel geführte Unterdrucksauger sichtbar ist;
- Fig. 6c: einen Querschnitt entlang B-B gemäß der Fig. 6b;
- Fig. 7a: eine Explosionsansicht des Stößels gemäß der Fig. 6b und des Unterdrucksaugers;
- Fig. 7b bis 7d: verschiedene Detailansichten des Stößels gemäß der Fig. 7a;
- Fig. 8a: eine Lochplatte mit Führungsstruktur zum Führen und Positionieren des Stößels gemäß der Fig. 7a in einer Perspektivansicht;
- Fig. 8b: eine Draufsicht auf die Lochplatte mit Führungsstruktur gemäß der Fig. 8a;
- Fig. 8c: eine Detailansicht aus der Draufsicht gemäß der Fig. 8b;
- Fig. 8d: eine Schnittansicht entlang B-B gemäß der Fig. 8b; und
- Fig. 9a: ein schematisches Flussdiagramm eines Verfahrens zur Prozessierung oder Bearbeitung von Fläschchen mittels einer Bearbeitungsstation gemäß der Fig. 4a;
- Fig. 9b und 9c: Beispiele für die Verwendung eines solchen Verfahrens zum Bördeln oder Crimpen eines Metalldeckels auf einem oberen Rand von Fläschchen;
- Fig. 9d: ein schematisches Flussdiagramm eines weiteren Verfahrens zur Prozessierung oder Bearbeitung von Fläschchen mittels einer Bearbeitungsstation gemäß der Fig. 4a;
- Fig. 9e und 9f: ein weiteres Beispiel für die Verwendung eines solchen Verfahrens zum Verschließen von Fläschchen mit Hilfe von steril verschließenden Kappen, die unmittelbar auf den oberen Rand der Fläschchen aufgedrückt werden;
- Fig. 10a und 10b: Einzelheiten einer Vorrichtung gemäß der vorliegenden Erfindung zum Wiegen eines Behälters, während dieser in einer Haltestruktur aufgenommen oder geführt ist;
- Fig. 11a und 11b: weitere Varianten einer Haltestruktur zur Verwendung bei einem Verfahren gemäß der vorliegenden Erfindung;
- Fig. 12a bis 12i: weitere Varianten einer Vertikal-Verstelleinrichtung zur Verwendung bei einem Verfahren gemäß der vorliegenden Erfindung; und
- Fig. 13a bis 13c: in einer Sequenz die Verwendung einer weiteren Vertikal-Verstelleinrichtung gemäß der vorliegenden Erfindung zum Verstellen von Behältern, die in einer Haltestruktur der vorgenannten Art gehalten sind.

In den Figuren bezeichnen identische Bezugszeichen identische oder im Wesentlichen gleichwirkende Elemente oder Elementgruppen.

### Ausführliche Beschreibung von bevorzugten Ausführungsbeispielen

Eine Haltestruktur sowie ein Transport- und Verpackungsbehälter, der eine solche Haltestruktur aufnimmt, dienen gemäß der vorliegenden Erfindung, wie nachfolgend beschrieben, der gleichzeitigen Halterung einer Mehrzahl von Behältern zur Aufbewahrung von Substanzen für kosmetische, medizinische oder pharmazeutische Anwendungen, und zwar bevorzugt in einer regelmäßigen Anordnung, insbesondere in einer Matrixanordnung unter regelmäßigen Abständen der Behälter zueinander, entlang von zwei unterschiedlichen Raumrichtungen, bevorzugt entlang von zwei zueinander orthogonalen Raumrichtungen oder in regelmäßigen Reihen, die relativ zueinander versetzt angeordnet sind. Nachfolgend wird zunächst die Haltestruktur beschrieben, bevor das Verfahren und eine Vorrichtung gemäß der vorliegenden Erfindung beschrieben werden.

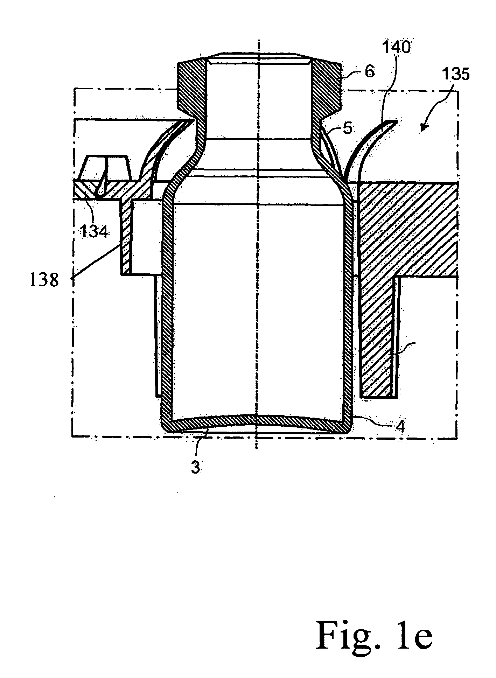

Ein Beispiel für derartige Medikamentenbehälter in Gestalt von Fläschchen ist in der Fig. 1e schematisch in einem Längsschnitt dargestellt. Diese weisen eine zylindrische Grundform auf, mit einer zylinderförmigen Seitenwand mit - im Rahmen der Toleranzen - konstantem Innen- und Außendurchmesser, die von einem flach ausgebildeten Flaschenboden 3 senkrecht abragt und nahe dem oberen offenen Ende des Fläschchens in einen verengten Halsabschnitt 5 von vergleichsweise geringer axialer Länge und anschließend in einen verbreiterten oberen Rand 6 (auch Rollrand) übergeht, der einen größeren Außendurchmesser aufweist als der zugeordnete Halsabschnitt 5 und zur Verbindung mit einem Verschlusselement ausgelegt ist. Wie man der Fig. 1e entnehmen kann, ist die Unterseite des Rollrands 6 abgeschrägt ausgebildet und erstreckt sich unter einem spitzen Winkel abwärts und hin zu dem verengten Halsabschnitt 5. Wie in der Fig. 1e dargestellt, ist in Luftspalt in radialer Richtung zwischen beispielsweise der linken Haltezunge 140 (oder mehreren oder allen Haltezungen einer Öffnung oder Aufnahme) und dem verengten Halsabschnitt 5 des Behälters ausgebildet.

Der Halsabschnitt 5 kann glattwandig ohne Außengewinde ausgebildet sein oder kann mit einem Außengewinde zum Aufschrauben eines Verschlusselements versehen sein. Beispielsweise kann in die Innenbohrung des Halsabschnitts 5 und des oberen Rands 6 ein Stopfen (nicht dargestellt) eingeführt werden, dessen oberes Ende mit dem oberen Rand 6 des Fläschchens gasdicht und geschützt gegen das Eindringen von Verunreinigungen in das Fläschchen mit dem oberen Rand 6 verbunden ist, beispielsweise durch Crimpen oder Bördeln einer nicht dargestellten Metallschutzfolie. Derartige Fläschchen sind radial symmetrisch und aus einem durchsichtigen oder eingefärbten Glas oder auch durch Blasformen oder Kunststoff-Spritzgusstechniken aus einem geeigneten Kunststoffmaterial ausgebildet, und können grundsätzlich innenbeschichtet sein, so dass das Material des Fläschchens möglichst wenig Verunreinigungen an die aufzunehmende Substanz abgibt.

Ein weiteres Beispiel für Behälter im Sinne der vorliegenden Anmeldung sind Ampullen, Karpullen oder Spritzen- oder Injektionsbehältnisse. Ampullen oder Karpullen sind Behältnisse für Arzneimittel zur meist parenteralen Applikation (Injektion), für Kosmetika und andere Substanzen und sind meist zylindrisch geformt mit einer ausgezogenen Spitze (Spieß oder Kopf) und einem flachen Boden oder auch mit zwei ausgezogenen Spitzen an beiden Enden. Diese können insbesondere als Brechampullen mit einer ringförmigen Sollbruchstelle um den Ampullenhals herum oder als OPC-Ampulle (One-Point-Cut-Ampulle) mit einem in das Glas geritzten Brechring ausgebildet sein. Spritzen- bzw. Injektionsbehältnisse, auch als Injektionsfläschchen, Stechampulle oder Mehrwegampulle bezeichnet, sind zylindrische, flaschenähnlich geformte Behältnisse aus Glas oder Kunststoff, meist in relativ kleinen Nennvolumina (z. B. 1 ml, 10 ml). Sie sind mit einem Gummistopfen mit Septum (Durchstichgummi) verschlossen. Zum Schutz des Septums und Fixierung des Gummistopfens ist noch ein äußerer Verschluss (Bördelkappe oder Krampe), oft aus Aluminiumblech, aufgebracht. Bei einer Karpule befindet sich die Flüssigkeit in einem Zylinder, der am einen Ende mit einem dicken Gummi- oder Kunststoffstopfen verschlossen ist. Dieser fungiert als Kolben, wenn der Inhalt mit einer Karpulenspritze ausgepresst wird. Am anderen Ende ist der Zylinder nur mit einer dünnen Membran verschlossen, die bei der Anwendung vom hinteren Ende der Karpulenkanüle (eine beidseitig angeschliffene Kanüle) durchstochen wird. Zylinderampullen werden häufig in der Zahnmedizin zur Lokalanästhesie verwendet. Spezielle Zylinderampullen mit besonders gestaltetem Vorderteil (z. B. Gewinde) werden zur Insulintherapie in Insulinpens verwendet.

Im Sinne der vorliegenden Erfindung dienen derartige Behälter (container) zur Aufbewahrung von Substanzen oder Wirkstoffen für kosmetische, medizinische oder pharmazeutische Anwendungen, die in einer oder auch mehrere Komponenten in fester oder flüssiger Form in dem Behälter aufbewahrt werden sollen. Gerade bei Glasbehältern können Aufbewahrungsdauem viele Jahre betragen, was insbesondere von der hydrolytischen Resistenz der verwendeten Glassorte abhängt. Während nachfolgend Behälter offenbart werden, die zylindrisch sind, sei darauf hingewiesen, dass die Behälter im Sinne der vorliegenden Erfindung auch ein anderes Profil haben können, beispielsweise ein quadratisches, rechteckförmiges oder vieleckiges Profil.

Unweigerlich weisen solche Behälter herstellungsbedingt Toleranzen auf, die gerade bei Glasbehältern einen oder mehrere Zehntel Millimeter betragen können. Um solche Fertigungstoleranzen kompensieren zu können und gleichzeitig zu gewährleisten, dass sämtliche Flaschenböden 3 in einer Ebene angeordnet werden können, werden die Behälter erfindungsgemäß an einer Halterungsstruktur fixiert. Die Halterung der Behälter wird dabei im Übergangsbereich des verengten Halsabschnitts 5 zum verbreiterten oberen Rand 6 realisiert. Insbesondere liegt die Unterseite des Rands 6 der Behälter im Übergangsbereich zum verengten Halsabschnitt 5 auf den oberen Enden von Haltezungen 140 auf, wie nachfolgend näher beschrieben. Die Haltezungen 140 sind bevorzugt aus einem ausreichend flexiblen oder elastischen Kunststoff ausgebildet. Alternativ können die Haltezungen auch relativ steif ausgebildet sein, jedoch so beweglich an der Oberseite des Trägers 134 gelagert sein, dass diese beim Einführen der Behälter elastisch aus der Öffnung 135 weggeschwenkt oder zurück geklappt werden, wie nachfolgend beschrieben. Zu diesem Zweck können die Haltezungen mittels elastischer Rückstellelemente (nicht gezeigt), beispielsweise Rückstellfedern oder elastischen Kunststoffgebilden oder -blättchen, in die in der Fig. 1e dargestellte Haltestellung elastisch vorgespannt werden.

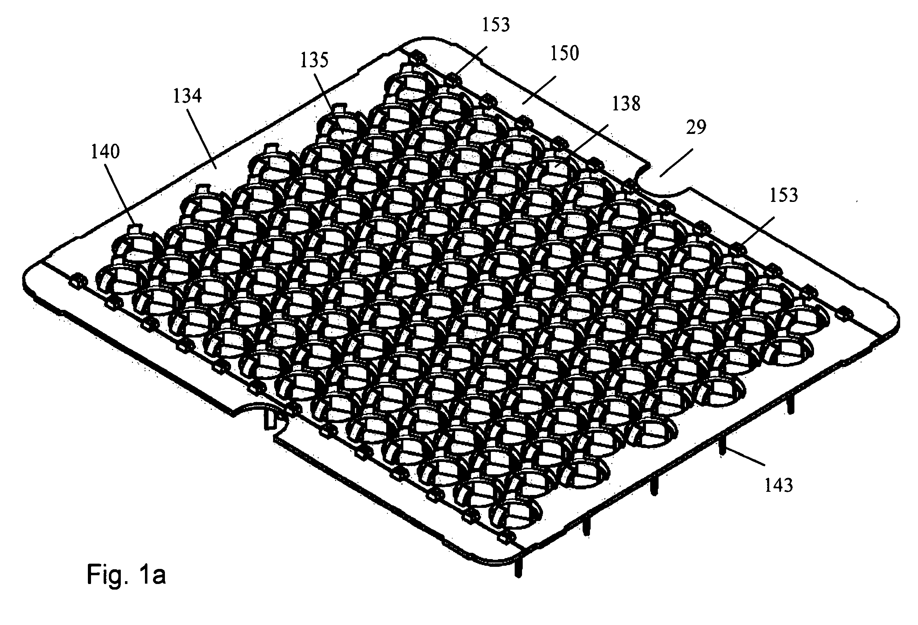

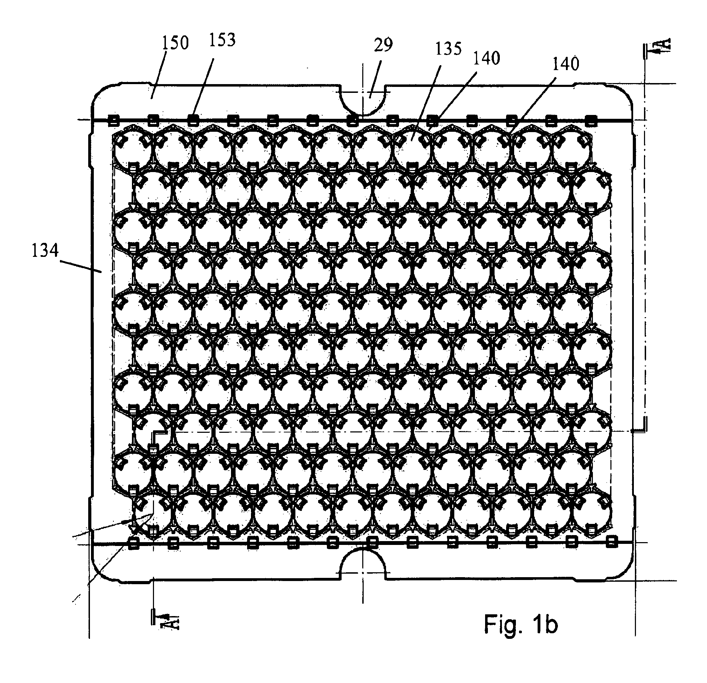

Zum gleichzeitigen Halten einer Mehrzahl von Behältern wird gemäß einer ersten Ausführungsform, wie in den Fig. 1a und 1b dargestellt, ein flächiger rechteckförmige Träger 134 bereitgestellt, der aus einem Kunststoff ausgebildet ist, beispielsweise ausgestanzt oder spritzgegossen ist, und eine Mehrzahl von Öffnungen 135 zur Aufnahme der Glasfläschchen 2 aufweist. Die Öffnungen 135 sind in einer regelmäßigen zweidimensionalen Anordnung angeordnet, bei dem dargestellten Ausführungsbeispiel in einer Matrix-Anordnung aus Reihen und sich rechtwinklig dazu erstreckenden Spalten, die unter gleichen Abständen zueinander und regemäßig zueinander versetzt in einer wiederkehrenden Anordnung angeordnet sind.

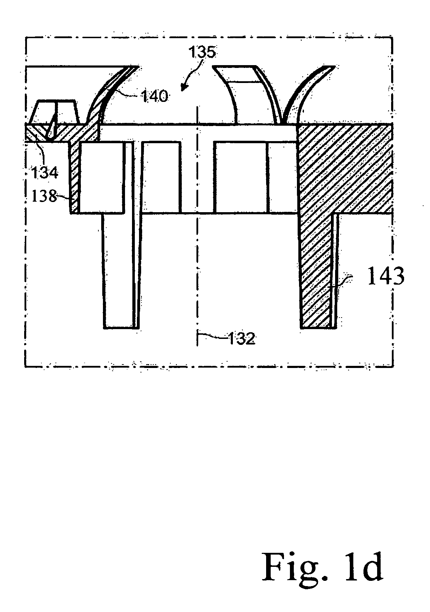

Die Öffnungen 135 sind von Seitenwänden 138 (vgl. Fig. 1d) auf der Unterseite des Trägers 134 begrenzt, um eine Kollision von Behältern, die in unmittelbar benachbarten Öffnungen 135 aufgenommen sind, zu verhindern. Gemäß der Fig. 1b ragen elastische Haltezungen 140 von der Oberseite des Trägers 134 bogenförmig ab und, in Draufsicht betrachtet, in die zugeordneten Öffnungen 135 hinein. Die elastischen Haltezungen 140 und die Seitenwände 138 sind bevorzugt einstückig mit dem flächigen Träger 134 ausgebildet sind, beispielsweise durch 1K- oder 2K-Kunststoff-Spritzgussverfahren.

Gemäß der Fig. 1b laufen die Seitenwände 138 jeweils in den Eckbereichen der Öffnungen 135 zusammen und sind dort miteinander verbunden oder einstückig ausgebildet. Von diesen Eckbereichen ragen die elastischen Haltezungen 140 in einer Anordnung mit dreizähliger Punktsymmetrie in die benachbarten Öffnungen 135 ab. Dies führt zu einer symmetrischen Kraftableitung beim Halten der Behälter über die Haltezungen 140. Die Haltezungen 140 bewirken so eine vorteilhafte Dreipunkt-Lagerung der Behälter in den Öffnungen, sodass die Behälter automatisch zentriert bezüglich einer Mittellinie 132 (vgl. Fig. 1d) einer jeweiligen Öffnung 135 gehalten werden.

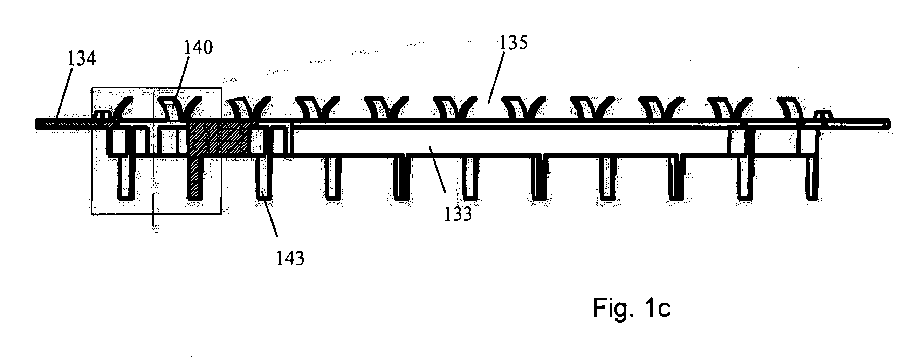

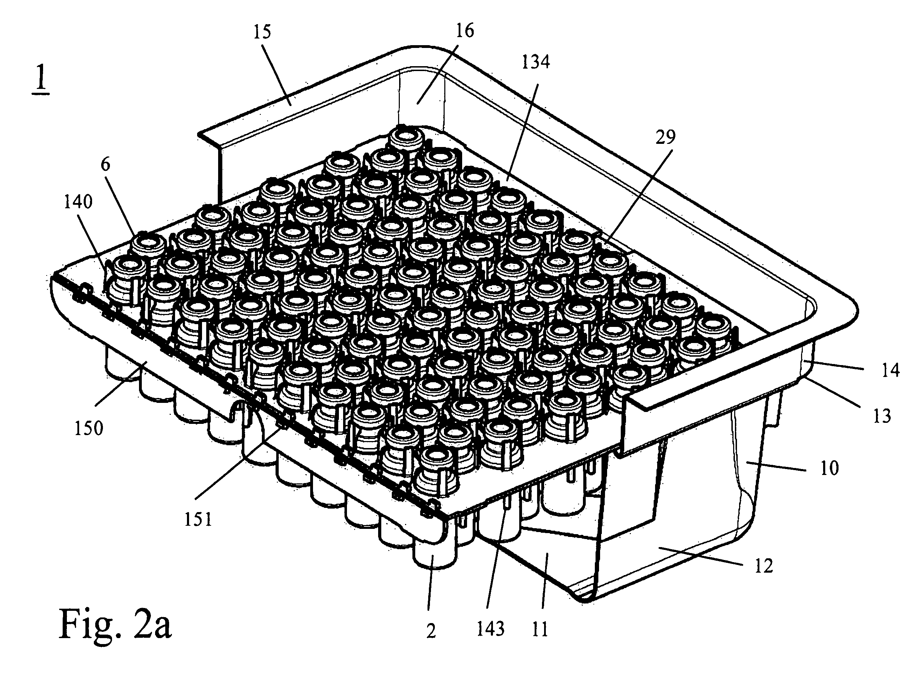

Die Fig. 1c zeigt einen Teilschnitt der Haltestruktur gemäß A-A von Fig. 1b. Erkennbar ist, dass der Träger 134 auf der Unterseite von einem umlaufenden Rand 133 begrenzt ist, auf welchem der Träger 134 auf einer umlaufenden Stufe 13 (vgl. Fig. 2a) eines Transport- oder Verpackungsbehälter 1 abgestützt werden kann.

Die Fig. 1d zeigt einen stark vergrößerten Teilschnitt in dem Einsatz, der in der Fig. 1c dargestellt ist. Erkennbar ist, dass die Behälter ohne weiteres von unten her in die Öffnungen 135 des Trägers 134 eingeführt werden können. Beim Einführen der Behälter in die Öffnungen 135 kommt es zu einem elastischen Verbiegen der elastischen Haltezungen 140.

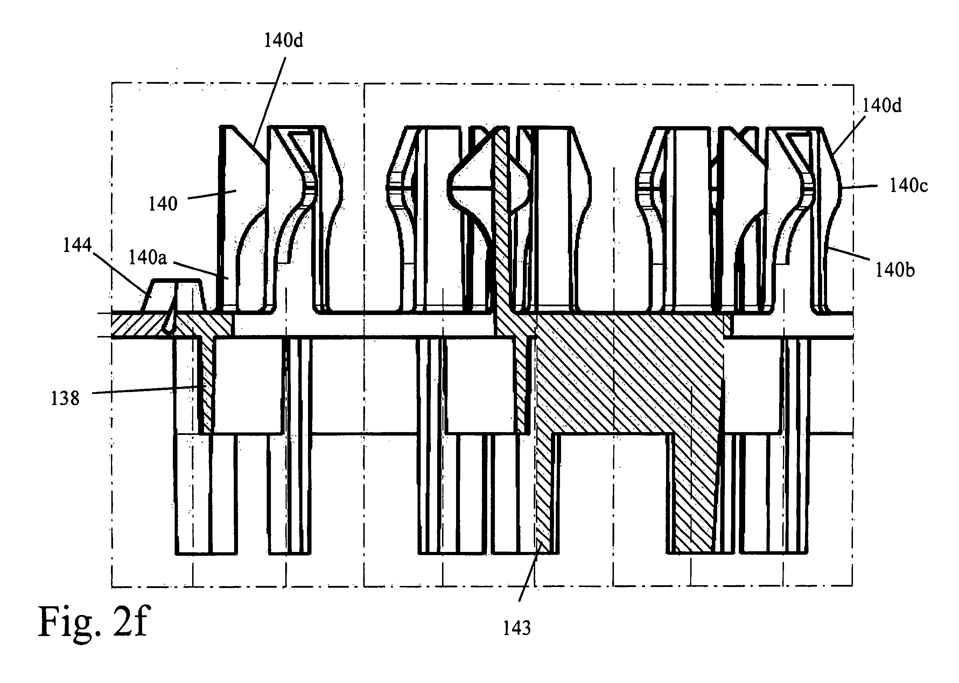

Je nach konkreter Ausgestaltung der zu haltenden Behälter können diese grundsätzlich auch von oben her in die Öffnungen 135 des Trägers 134 eingeführt werden, um an dem Träger 134 gehalten zu werden. Dies hat den Vorteil, dass das Risiko, dass Flüssigkeit oder anderer Behälterinhalt aus dem Behälterinnenraum der noch unverschlossenen Behälter unkontrolliert beim Einführen in die Öffnungen und beim Wegschwenken der Haltezungen 140 auf die Haltestruktur, insbesondere die Trägerplatte 134, gelangen können, noch weiter verringert werden kann. Zu diesem Zweck können auf der Oberseite der elastischen Haltezungen 140 Einführschrägen vorgesehen sein, wie diese nachfolgend näher anhand der Fig. 2f für eine alternative Ausführungsform beschrieben werden.

Durch Stärke, Material und Auslegung der elastischen Haltezungen 140 kann die zum Einführen und Entnehmen eines Behälters notwendige Kraft einfach vorgegeben werden.

Gemäß einer bevorzugten Ausführungsform sind die Behälter zumindest mit radialem Spiel und bevorzugt sowohl mit radialem als auch mit axialem Spiel lose auf den Haltezungen abgestützt. Auf diese Weise können auch große Toleranzen von Behältern und unterschiedliche Außendurchmesser im Bereich des Halsabschnitts 5 einfach ausgeglichen werden. Denn zur Halterung der Behälter genügt es, wenn der Rollrand 6 noch auf den Oberseiten der Haltezungen 140 aufliegt. Grundsätzlich können dadurch auch Behälter unterschiedlichen Typs, beispielsweise mit unterschiedlichen Durchmessern im Bereich des Halsabschnitts 5, von derselben Haltestruktur gehalten werden.

Die Fig. 1e verdeutlicht dies in dem gleichen stark vergrößerten Teilschnitt wie nach der Fig. 1d und stellt die Halterung eines Behälters in einer Öffnung 135 des Träges 134 dar. Gemäß der Fig. 1e liegt die Unterseite des verbreiterten Rands 6 auf dem vorderen Ende der elastischen Haltezungen 140 im Übergangsbereich zwischen dem verengten Halsabschnitt 5 und dem Rand 6 lose auf, um die Lage des Behälters zu fixieren. Wie man in der Fig. 1e erkennen kann, liegt zwischen den Haltezungen 140 (vgl. linker Bildteil) und dem verengten Halsabschnitt 5 ein Luftspalt, der ein radiales Spiel ermöglicht.. Aufgrund dieser Halterung mit radialem Spiel besteht dabei, je nach konkreter Ausbildung des Behälters, noch die Möglichkeit, den von den Haltezungen 140 gehaltenen Behälter axial zu verschieben, d.h. in Längsrichtung der Behälter, beispielsweise solange, bis die Böden 3 von allen von dem Träger 134 gehaltenen Behältern unter dem gleichen Abstand zum Träger 134 gehalten werden, um gemeinsam eine Ebene aufzuspannen.

Gemäß der Fig. 1e ist der Behälter soweit in die Öffnung 135 eingeschoben, dass der verbreiterte Rand 6 auf den vorderen Enden der Haltezungen genau an dem Übergangsbereich zwischen dem verengten Halsabschnitt 5 und dem verbreiterten oberen Rand 6 abgestützt ist. Dies lässt sich beispielsweise durch Einschieben der Behälter von unten her in die Öffnungen 135 des Trägers 134 und anschließendes Herabdrücken der Behälter bewerkstelligen, und zwar solange, bis die vorderen Enden der Haltezungen genau an dem Übergangsbereich zwischen dem verengten Halsabschnitt 5 und dem verbreiterten oberen Rand 6 anliegen. In der Haltestellung gemäß der Fig. 1e ist jedenfalls bei der großen Mehrzahl der fixierten Behälter ein gewisser radialer Abstand zwischen dem stufenartigen Übergangsbereich zwischen dem oberen Rand 6 und dem verengten Halsabschnitt 5 und vorderen Ende der Haltezungen 140 vorgesehen. Auf diese Weise können in einem gewissen Umfang Fertigungstoleranzen der Behälter in axialer Richtung und auch Fertigungstoleranzen in radialer Richtung kompensiert werden und somit auch Behälter mit unterschiedlichen Durchmessern im Bereich des verengten Halsabschnitts 5 von ein- und demselben Träger 134 gehalten werden. Damit lassen sich auch etwaige Verspannungen im Kunststoff des Trägers 134 aufgrund der Aufnahme von Behältern mit einem zu großen Außendurchmesser gering halten.

Gemäß alternativen Ausführungsformen, wie nachfolgend beschrieben, können die Behälter auch form- oder reibschlüssig an dem Träger 134 gehalten werden.

Zum Transport und zur Verpackung der vorstehend beschriebenen Haltestruktur mit den darin aufgenommenen Behältern dient ein Transport- und Verpackungsbehälter 10, wie dieser schematisch in der Fig. 2a für eine Haltestruktur bzw. einen Träger 134 gemäß einer zweiten Ausführungsform der vorliegenden Erfindung dargestellt ist. Gemäß der Fig. 2a ist der Transport- und Verpackungsbehälter 10 im Wesentlichen kasten- oder wannenförmig ausgebildet und weist einen Boden 11, eine senkrecht von diesem abragende, umlaufend ausgebildete Seitenwand 12, eine von dieser im Wesentlichen rechtwinklig abragende Stufe 13, eine umlaufend ausgebildete obere Seitenwand 14 und einen oberen Rand 15 auf, der flanschartig ausgebildet ist. Die Ecken 16 des Transport- und Verpackungsbehälters 10 sind zweckmäßig abgerundet ausgebildet. Die obere Seitenwand 14 kann unter einem geringen Neigungswinkel zur Senkrechten auf den Boden 11 geneigt ausgebildet sein, um das Einführen der Haltestruktur 134 zu erleichtern. Ein derartiger Transport- und Verpackungsbehälter 10 ist bevorzugt aus einem Kunststoff ausgebildet, insbesondere durch Kunststoff-Spritzgusstechnik, und ist bevorzugt aus einem klaren, durchsichtigen Kunststoff ausgebildet, um eine optische Sichtkontrolle der in dem Transport- und Verpackungsbehälter 10 aufgenommenen Haltestruktur 134 und der von dieser gehaltenen Behälter 2 zu ermöglichen.

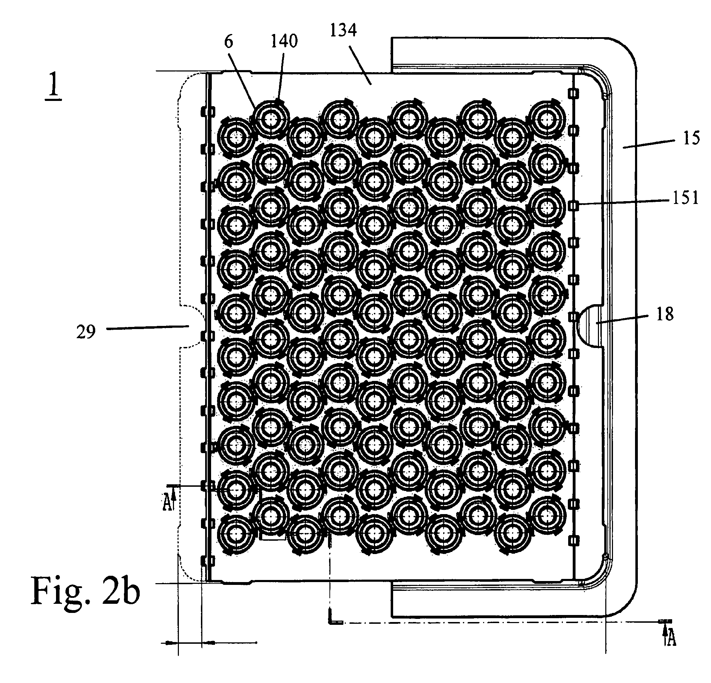

Zur Aufnahme der Haltestruktur 134 in dem Transport- und Verpackungsbehälter 10 kann diese von einem umlaufend ausgebildeten Randsteg 133 umrandet sein, wie in der Fig. 1c dargestellt. Ein solcher Randsteg kann auch entlang des Umfangsrands abschnittsweise durchgehend ausgebildet sein. Zur zuverlässigen Positionierung der Haltestruktur 134 in dem Transport- und Verpackungsbehälter 10 weisen die Haltestruktur 134 und der Transport- und Verpackungsbehälter 10 miteinander zusammenwirkende Positionierungsgebilde auf, die insbesondere formschlüssig zusammenwirken. So können an geeigneter Stelle, insbesondere auf der Stufe 13 oder auf Abstützflächen 18 (vgl. Fig. 2b) des Transport- und Verpackungsbehälters 10 Positionierungsgebilde in Form von Vorsprüngen oder Aussparungen bzw. Vertiefungen ausgebildet sein, die mit korrespondierend ausgebildeten Aussparungen bzw. Vertiefungen oder Vorsprüngen der Haltestruktur formschlüssig zusammenwirken, um die Haltestruktur 134 präzise in dem Transport- und Verpackungsbehälter 10 zu positionieren. Zu diesem Zweck können insbesondere auf der Stufe 13 des Transport- und Verpackungsbehälters 10 mehrere zapfenartige Vorsprünge (nicht gezeigt) ausgebildet sein, die in korrespondierend ausgebildete Zentrieröffnungen in einem Halterahmen der Haltestruktur 134 zusammenwirken. Gemäß der Fig. 2a ist die Stufe 13 des Transport- und Verpackungsbehälters 10 als umlaufende, ebene Abstützfläche ausgebildet, auf welcher die Haltestruktur 134 unmittelbar aufliegt. Gemäß weiteren Ausführungsformen können an den Seitenwänden 12 des Transport- und Verpackungsbehälters 10 auch weitere Abstützflächen 18 oder Abstützelemente ausgebildet sein, insbesondere in Form von Vorsprüngen, wie nachfolgend ausgeführt. Auf diese Weise kann die Haltestruktur 134 präzise in dem Transport- und Verpackungsbehälter 10 positioniert werden und die Mehrzahl von Fläschchen 2 auf diese Weise in einer regelmäßigen Anordnung und an präzise definierten Positionen in einem Transport- und Verpackungsbehälter 10 mit standardisierten Abmessungen angeordnet und gehalten werden. Insbesondere kann auf diese Weise gewährleistet werden, dass sämtliche Böden oder untere Enden der Fläschchen 2 in einer gemeinsam aufgespannten Ebene parallel zum Boden 11 oder zum oberen Rand 15 des Transport- und Verpackungsbehälters 10 angeordnet sind.

Wenngleich in der Fig. 2a der Boden 11 des Transport- und Verpackungsbehälters 10 als geschlossen und einstückig mit der Seitenwand 12 ausgebildet dargestellt ist, kann das untere Ende des Transport- und Verpackungsbehälters 10 auch in der Art des oberen Endes geöffnet ausgebildet sein, insbesondere mit einem flanschartigen unteren Rand in der Art des oberen Rands 15 versehen sein, so dass die Böden der Fläschchen 2 von der Unterseite des Transport- und Verpackungsbehälters 10 her frei zugänglich sind, beispielsweise für Verarbeitungsschritte in einem Steriltunnel oder in einem Gefriertrockenschrank, wie nachfolgend näher erläutert.

Wie nachfolgend näher erläutert, können erfindungsgemäß die Behälter 2 auch innerhalb der Haltestruktur 134 oder des Transport- und Verpackungsbehälters 10 gemeinsam weiterverarbeitet werden, insbesondere auch in einem Steriltunnel oder einem Gefriertrockenschrank.

Damit die Haltestruktur 134 leicht in den Transport- und Verpackungsbehälter 10 eingesetzt und aus diesem entnommen werden kann, sind an zwei Längsseiten der Haltestruktur 26134 Zugriffsöffnungen 29 ausgebildet, über welche Greifarme oder dergleichen die Haltestruktur 134 greifen können. Die Zugriffsöffnungen 29 können, in Längs- oder Querrichtung der Haltestruktur 134 betrachtet, versetzt zueinander angeordnet, was eine eineindeutige Positionierung der Haltestruktur 134 in dem Transport- und Verpackungsbehälter 10 weiter erleichtert.

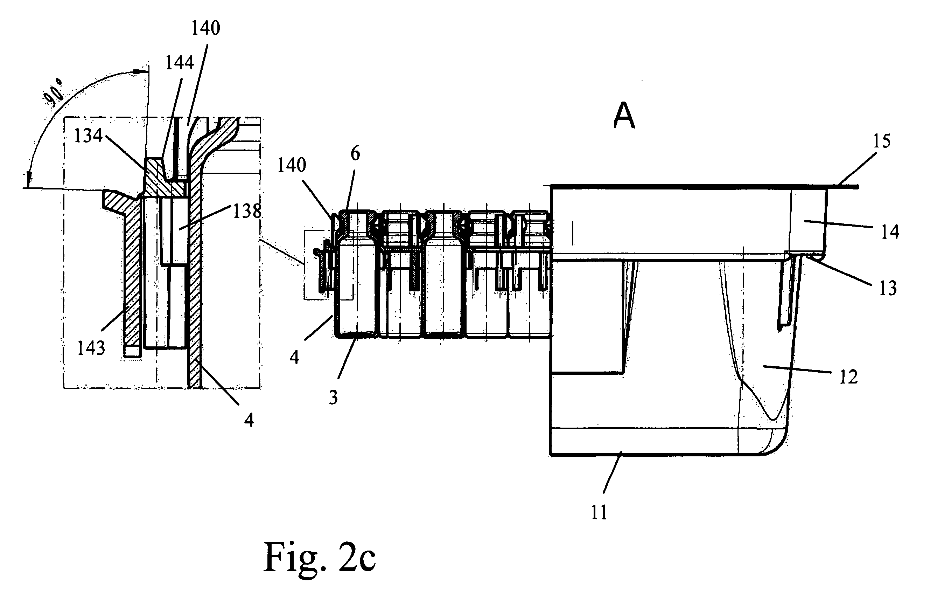

Die Fig. 2c zeigt in zwei vergrößerten Teilschnitten entlang A-A gemäß der Fig. 2b die Halterung von Behältern in der Haltestruktur gemäß der zweiten Ausführungsform sowie Details davon. Erkennbar ist insbesondere, dass auf der Oberseite des Trägers abgeschrägte Anschlagnasen 144 vorgesehen sind, welche das Zurückschwenken der elastischen Haltezungen 140 beim Einführen der Behälter begrenzen.

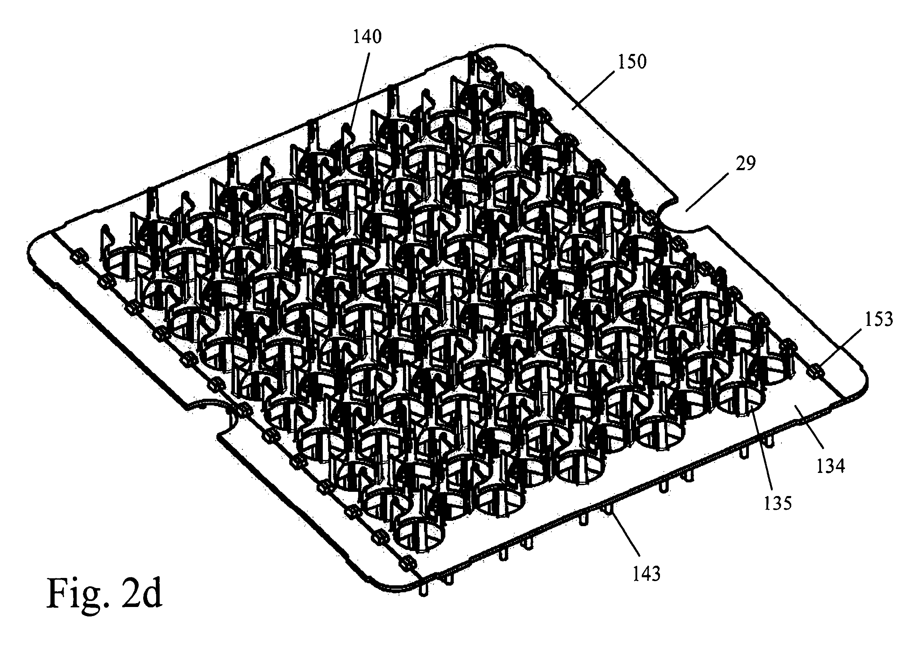

Die Fig. 2d zeigt in einer perspektivischen Draufsicht die Haltestruktur gemäß der Fig. 2a ohne Behälter. Erkennbar sind die elastischen Haltezungen 140 fähnchenartig und mit einer radial einwärts vorstehenden Haltenase ausgebildet, wie in dem stark vergrößerten Teilschnitt durch diese Haltestruktur gemäß der Fig. 2f besser dargestellt. Gemäß der Fig. 2f sind die elastischen Haltezungen 140 über eine senkrecht von der Oberseite des Trägers 134 abragende, elastische Basis 140a mit dem Träger 134 verbunden. Die Basis 140a geht über in einen radial einwärts gekrümmten Abschnitt 140b über, der schließlich in die Haltenase 140c übergeht, auf welcher der verbreiterte Rand 6 (vgl. Fig. 1e) der Behälter aufliegt, wie vorstehend anhand der Fig. 1e für die erste Ausführungsform beschrieben. Die Haltenase 140c ragt dabei in die Öffnung des Trägers 134 hinein. Die Haltenase 140c geht über in eine sich schräg aufwärts erstreckende Einführschräge 140d über, die mit dem oberen Ende der Haltezunge 140 verbindet. Aufgrund der Einführschräge 140d auf der Oberseite der Haltezunge 140 sowie des nach unten geöffneten, gekrümmten Abschnitts 140b der Haltezunge 140 können die Behälter wahlweise von oben oder von unten her in die Öffnungen des Trägers 134 eingeführt und aus diesen wieder abgezogen werden.

Beim Einführen der Behälter von oben her in die Öffnungen geraten zunächst die Böden bzw. unteren Enden der Behälter in Anlage zu den Einführschrägen 140d der Haltezungen 140. Beim weiteren Einführen der Behälter gleitet das untere Ende bzw. der Boden der Behälter entlang den Einführschrägen 140d abwärts und spreizt dabei die Haltezungen 140 zunehmend elastisch auseinander oder klappt bzw. schwenkt diese zurück. Beim weiteren Einführen der Behälter gerät schließlich die zylindrische Seitenwand der Behälter (vgl. Fig. 1e) in Anlage zu den Haltenasen 140c und gleitet an diesen entlang, solange bis schließlich die Unterseite des verbreiterten Rands der Behälter lose auf den Haltenasen 140c der Haltezungen 140 aufliegt. Die Behälter können dann entweder nach oben hin mit umgekehrtem Bewegungsablauf der Haltezungen 140 und ohne elastisches Verbiegen der Haltezungen 140 oder nach unten hin mit elastischem Verbiegen der Haltezungen 140 aus den Öffnungen des Trägers 134 entnommen werden.

Beim Einführen der Behälter von unten her in die Öffnungen gerät das obere Ende der Behälter zunächst in Anlage zu dem gekrümmten Abschnitt 140b der Haltezungen. Beim weiteren Einführen der Behälter gleitet das obere Ende der Behälter entlang den gekrümmten Abschnitten 140b aufwärts und spreizt dabei die Haltezungen 140 zunehmend elastisch auseinander oder klappt bzw. schwenkt diese zurück, bis schließlich die Haltenasen 140c erreicht sind. Beim weiteren Hochschieben der Behälter gleitet die Unterseite des verbreiterten Rands der Behälter über die Haltenasen 140c der Haltezungen 140 und liegt schließlich lose auf den Haltenasen 140c der Haltezungen 140 auf. Die Behälter können dann entweder nach unten hin mit umgekehrtem Bewegungsablauf der Haltezungen 140 und mit elastischem Verbiegen der Haltezungen 140 oder nach oben hin ohne elastisches Verbiegen der Haltezungen 140 aus den Öffnungen des Trägers 134 entnommen werden.

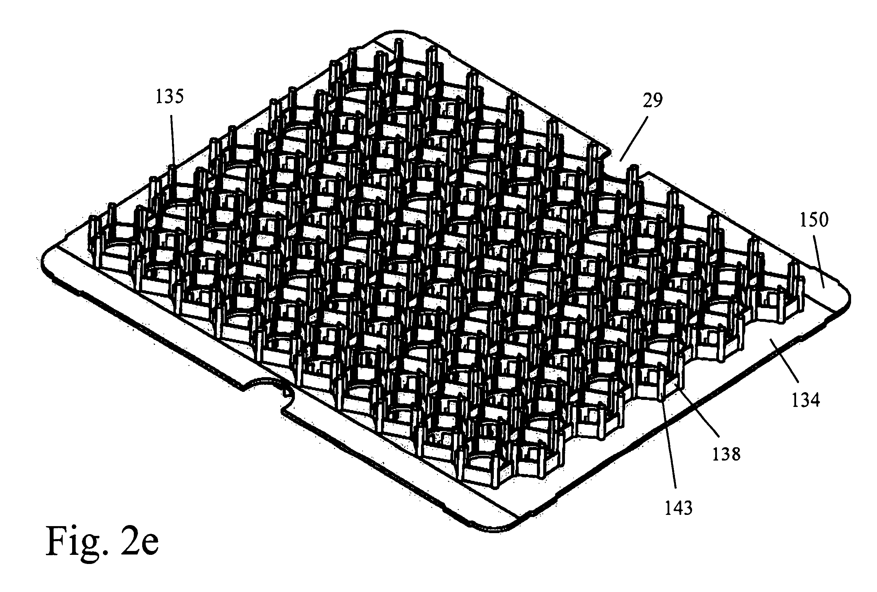

Die Fig. 2e zeigt in einer perspektivischen Unteransicht die Haltestruktur gemäß der Fig. 2a ohne Behälter. Erkennbar ist die wabenförmige, hexagonale Anordnung der umlaufenden Seitenwände 138, in deren Eckbereichen Zapfen 143 senkrecht von der Unterseite des Trägers 134 abragen. Diese Zapfen 143 dienen als Abstandshalter beim Ablegen des Trägers 134 auf einer Ablagefläche, beispielsweise dem Boden 11 eines Transport- und Verpackungsbehälters (vgl. Fig. 2a), vermeiden aber gleichzeitig auch den Kontakt der Behälter untereinander.

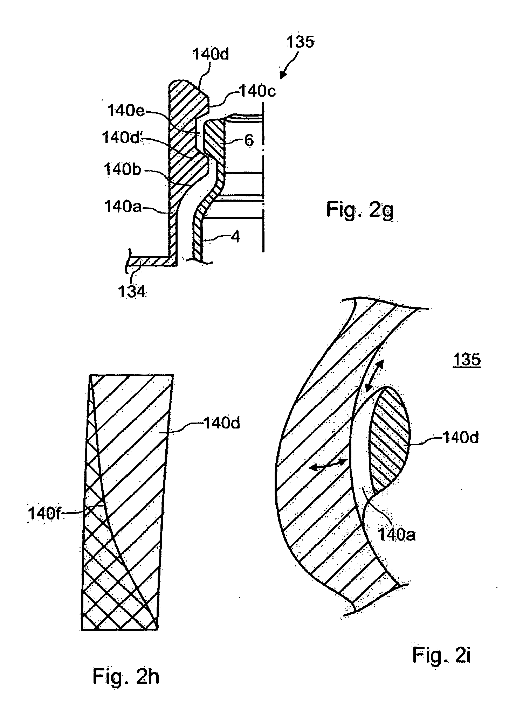

Die Fig. 2g zeigt in einem stark vergrößerten Teilschnitt die Halterung eines Behälters in einer Haltestruktur gemäß einer weiteren Ausführungsform der vorliegenden Erfindung. Abweichend zur zweiten Ausführungsform werden die Behälter hier an ihrem verbreiterten oberen Randabschnitt 6 (Rollrand) formschlüssig umgriffen, wobei ein ausreichendes radiales Spiel, wie vorstehend beschrieben, gewährleistet ist, wie durch den Luftspalt in Radialrichtung in der Fig. 2g angedeutet. Alternativ kann zusätzlich zu diesem radialen Spiel auch ein ausreichendes axiales Spiel gewährleistet sein, wie durch den Luftspalt in axialer Richtung in der Fig. 2g angedeutet. Zu diesem Zweck ist am vorderen Ende der Haltenase 140c (vgl. Fig. 2f) eine C-förmige Aussparung 140e vorgesehen, die über Schrägen 140d' in die Haltenase 140c übergeht. In der Haltestellung gemäß der Fig. 2g liegt der verbreiterte Randabschnitt 6 lose und mit radialem Spiel auf der unteren Schräge 140d' der Aussparung 140e auf. Wie in der Fig. 2g dargestellt, kann zwischen dem oberen Ende des verbreiterten Randabschnitts 6 und der oberen Schräge 140d' der Aussparung ein ausreichendes axiales Spiel bereitgestellt sein. Insgesamt wird somit der verbreiterte Randabschnitt 6 von der Haltezunge 140 klammerartig und formschlüssig umgriffen. Die Einführschräge 140d', der gekrümmte Abschnitt 140b sowie die Schrägen 140d' der Aussparung ermöglichen dabei ein Einführen und Abziehen der Behälter ohne größeren Kraftaufwand in die Öffnungen bzw. aus diesen heraus unter elastischen Wegbiegen der Haltezungen 140.

Die Fig. 2h stellt eine stark vergrößerte Draufsicht auf eine Einführschräge einer Haltezunge gemäß einer Variante zu der Haltestruktur gemäß der Fig. 2a dar. Gemäß der Fig. 2h ist die Einführschräge 140d mittels eines darauf ausgebildeten bogenförmigen Grats 140f insgesamt verdrillt ausgebildet. Diese gewundene Einführschräge 140d ist auf sämtlichen Haltezungen der Öffnungen oder Aufnahmen gleich ausgebildet. Insgesamt sind die Einführschrägen, in Draufsicht betrachtet, um einen Winkel von kleiner 90° gekrümmt ausgebildet. Im Zusammenwirken mit dem Behälter bewirkt dies beim Einführen der Behälter in die Öffnungen, dass die Haltezungen nicht nur radial auswärts weggeschwenkt oder zurück geklappt werden, sondern gleichzeitig mit einer Bewegungskomponente in Umfangsrichtung in Entsprechung zu der Geometrie der Einführschrägen 140d weggedreht werden, und zwar um einen Winkel von kleiner 90°. Je nach der Geometrie der Anordnung der Haltezungen auf dem Träger kann so eine Kollision von Haltezungen von unmittelbar benachbarten Öffnungen oder Aufnahmen beim Zurückschwenken oder Zurückklappen vermieden werden. Auf diese Weise kann die Packungsdichte der Behälter an der Haltestruktur noch weiter erhöht werden.

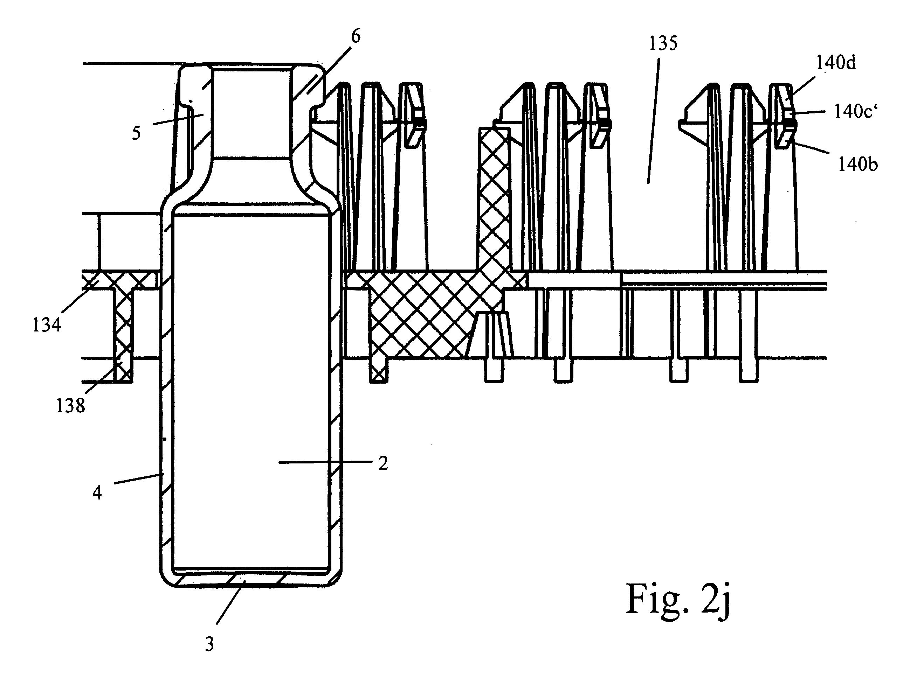

Die Fig. 2i zeigt in einer Draufsicht eine weitere Variante von Haltezungen für eine Haltestruktur gemäß der Fig. 2a, bei der die Basis 140a, in Axialrichtung betrachtet, verdrillt ausgebildet ist, was im Zusammenwirken der Einführschräge 140d mit dem Behälter beim Einführen des Behälters von oben her in die Öffnung oder Aufnahme sowohl eine radiale Komponente als auch eine Komponente in Umfangsrichtung beim elastischen Wegschwenken der Haltezungen ergibt, wie durch die beiden Doppelpfeile schematisch angedeutet. Die Fig. 2j zeigt schematisch die Halterung eines Behälters 2 in einer Haltestruktur, wie in der Fig. 2f gezeigt.

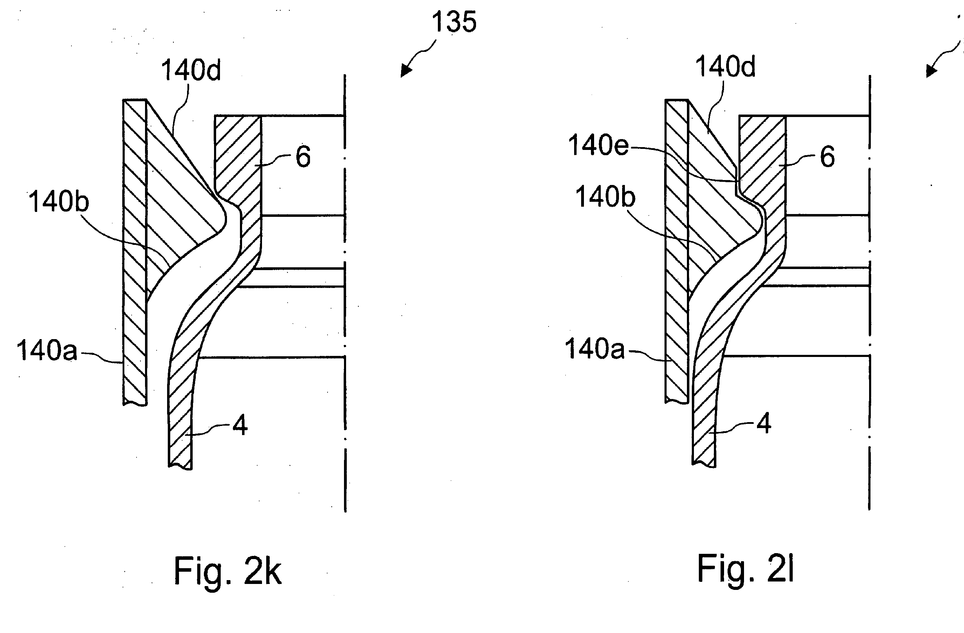

Die Fig. 2l zeigt eine weitere Variante zu der Ausführungsform nach der Fig. 2f mit modifizierter Ausbildung der fähnchenartigen elastischen Haltezungen 140. Während bei der Ausführungsform nach der Fig. 2f der Übergangsbereich zwischen den beiden Einführschrägen 140b und 140d abgeflacht oder auswärts abragend ausgebildet ist, ragt bei der Ausführungsform nach der Fig. 2k die untere Einführschräge 140b weiter in die Öffnung 135 hinein als die obere Einführschräge 140d. Der Übergangsbereich 140c' erstreckt sich im Wesentlichen senkrecht oder verläuft relativ steil abwärts geneigt. Der obere Rollrand 6 des Fläschchens 2 kann lose auf diesem geneigten Übergangsbereich 140c' aufliegen oder auf einer Stufe, die von der Oberseite der unteren Einführschräge 140b ausgebildet wird. In jedem Fall sind die elastischen Haltezungen 140 so ausgelegt, dass zwischen den vorderen Enden der Haltezungen 140 und dem von diesen gehaltenen Fläschchen 2 ein gewisses radiales Spiel besteht, sodass insbesondere Fertigungstoleranzen der Fläschchen 2 ausgeglichen werden können.

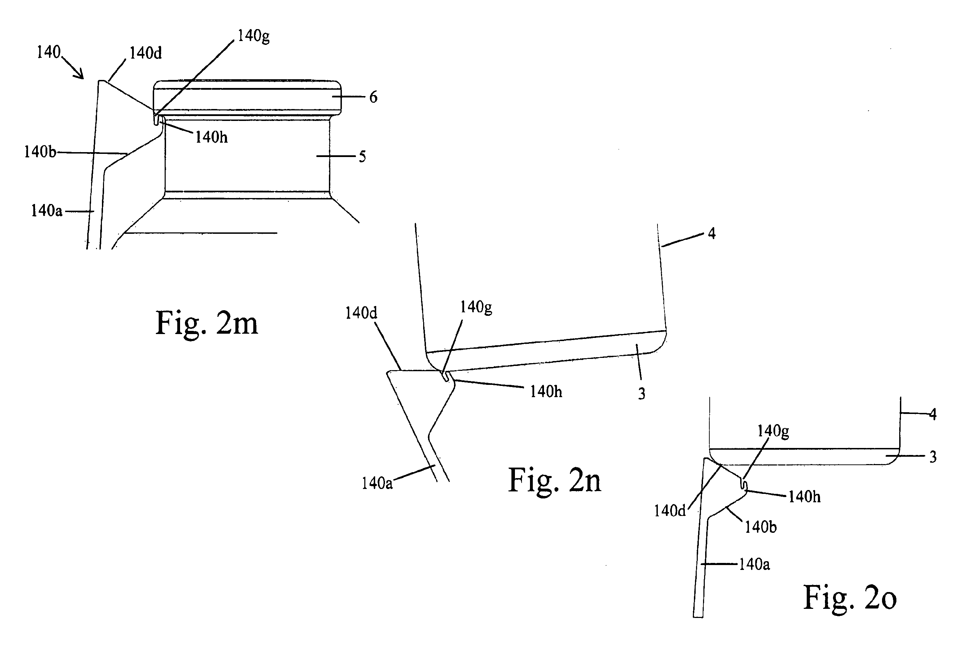

Die Figuren 2m bis 2o zeigen in einer Bewegungssequenz das Einführen eines Behälters in eine Haltestruktur gemäß einer weiteren Variante einer Haltestruktur. Gemäß der Fig. 2m ist an dem der zugeordneten Öffnung zugewandten vorderen Ende der oberen Einführschräge 140d eine Einkerbung 140g ausgebildet, der sich ein Vorsprung 140h anschließt, der eine vergleichsweise geringe Stärke aufweist und sich somit relativ leicht verbiegen lässt. In der Halteposition gemäß der Fig. 2m liegt die Unterseite des Rollrands 6 des Fläschchens lose auf diesem Vorsprung 140h auf. Dabei kann zwischen dem Rollrand 6 und dem der Öffnung zugewandten vorderen Ende der oberen Einführschräge ein radiales Spiel bestehen, wie vorstehend beschrieben. Die Fig. 2o zeigt, wie der Boden 3 des Fläschchens beim Einführen von oben her in die zugeordnete Öffnung entlang der oberen Einführschräge 140d hinabgleitet, bis schließlich gemäß der Fig. 2n der Vorsprung 140h erreicht ist. Sobald der

Boden 3 mit seinem abgeschrägten Umfangsrand den Vorsprung 140h berührt, bewirkt die Kontaktkraft ein Eindrücken des Vorsprungs 140h, wodurch sich der Kontaktwinkel verändert (er wird steiler) und das weitere Abrutschen des Fläschchens über den vorderen Bereich der oberen Einführschräge 140d hinaus begünstigt wird. Beim Durchdrücken des Rollrands 6 übt dieser eine Kraft auf den Vorsprung 140h aus, sodass ein Moment auf die ganze Haltezunge 140 einwirkt, das die Haltezunge 140 nach innen biegen würde. Der Vorsprung 140h biegt sich jedoch in Richtung des Fläschchens und der Kontaktwinkel wird so verändert, dass die Kraft nun mehr in die Richtung des Fusses der Haltezunge wirkt, sodass das Moment, das die Haltezunge 140 nach innen biegt, vorteilhaft verringert werden kann.

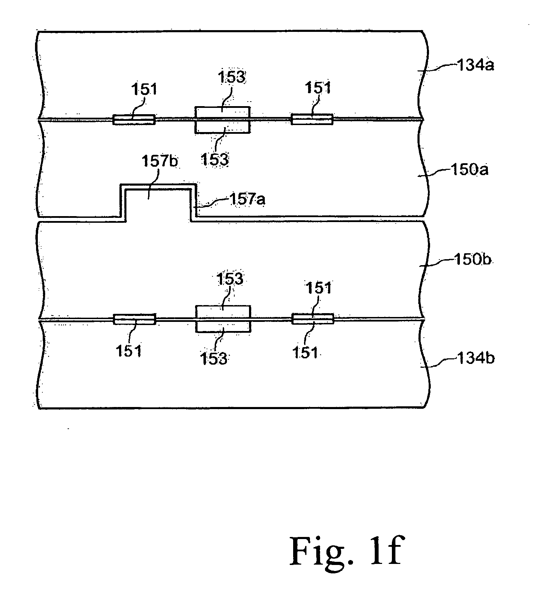

Die Fig. 1f zeigt in einem stark vergrößerten Teilschnitt und in Draufsicht eine weitere Variante der Haltestruktur gemäß der Fig. 1b, wobei Ränder 150a, 150b des plattenförmigen Trägers 134a, 134b wegeklappt werden können, um die Grundfläche des jeweiligen Trägers weiter zu reduzieren, beispielsweise dann, wenn dieser mit den Behältern an eine beengte Weiterverarbeitungsstation übergeben werden soll, beispielsweise an einen Gefriertrockenschrank mit begrenzter Grundfläche. Zu diesem Zweck sind die Ränder 150a, 150b über Scharniere 151 mit dem jeweiligen Träger verbunden. Insbesondere können die Scharniere 151 als Filmscharniere oder Schnapp- bzw. Federscharniere aus einem Kunststoff einstückig mit dem Träger 134 ausgebildet sein.

Gemäß der Fig. 1f sind an den abnehmbaren oder wegschwenkbaren Elementen 150a, 150b Aussparungen 157a und/oder Vorsprünge 157b ausgebildet. Die Aussparungen 157a und/oder Vorsprünge 157b der abnehmbaren oder wegschwenkbaren Elemente 150 eines Trägers sind korrespondierend zu den Aussparungen 157a und/oder Vorsprüngen 157b der abnehmbaren oder wegschwenkbaren Elemente 150 eines unmittelbar benachbarten flächigen Trägers ausgebildet, sodass ein Formschluss zwischen diesen Aussparungen 157a und/oder Vorsprüngen 157b ausgebildet werden kann, um die gegenseitige Lage der Träger festzulegen und zu stabilisieren.

Auf der Oberseite der Träger 134a, 134b und der Ränder 150a, 150b sind an einander entsprechenden Positionen blockförmige Anschläge 153 vorgesehen, die im gegenseitigen Anschlag eine koplanare Ausrichtung der Ränder 150a, 150b und des Trägers 134 festlegen und ein Hochklappen der Ränder 150a, 150b verhindern. Die Träger können deshalb auch nur an den Rändern in einem Transport- und Verpackungsbehälter (vgl. Fig. 2a) abgelegt werden.

Gemäß einer weiteren Ausführungsform (nicht dargestellt) können die Ränder 150 auch von dem Träger 134 abgenommen werden. Die Ränder 150 können selbstverständlich entlang von allen vier Längsseiten des Trägers 134 vorgesehen sein.

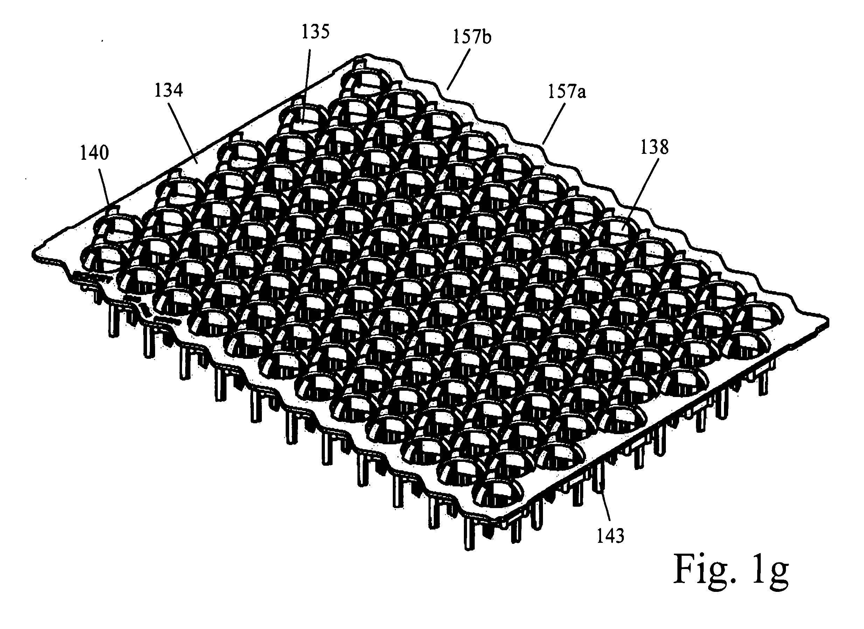

Die Fig. 1g zeigt eine weitere Variante der vorgenannten Haltestruktur gemäß der Fig. 1f, wobei die vorgenannten Vorsprünge 157b und Aussparungen 157a unmittelbar am Rand des flächigen Trägers 134 ausgebildet sind.

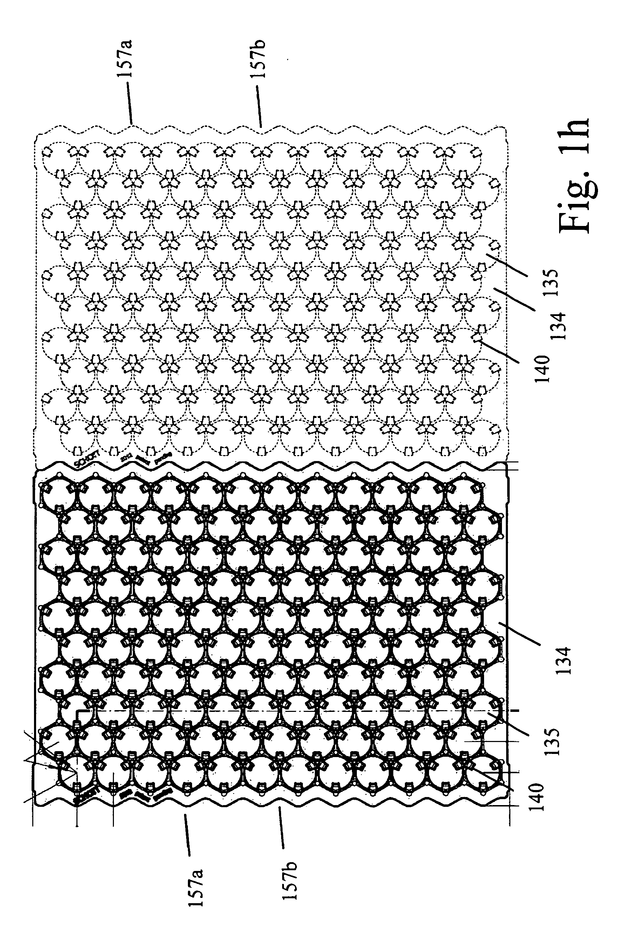

Die Fig. 1h zeigt in einer schematischen Draufsicht das Zusammenwirken von zwei Haltestrukturen gemäß der Fig. 1g. Die wellenförmig ausgebildeten Vorsprünge 157b und Aussparungen 157a von zwei benachbarten Trägern 134 sind korrespondierend zueinander ausgebildet, sodass die Ränder der Träger 134 unmittelbar formschlüssig ineinander greifen können, was eine gegenseitige Stabilisierung der Positionen der Träger 134 bei der Prozessierung oder Handhabung ermöglicht. Die Träger 134 können nach dieser Ausführungsform auch entlang der Ränder um einen Vorsprung 157b weiter verschoben werden und erneut in einen formschlüssigen Eingriff gebracht werden, sodass die beiden Träger dann um einen Vorsprung 157b versetzt zueinander angeordnet sind.

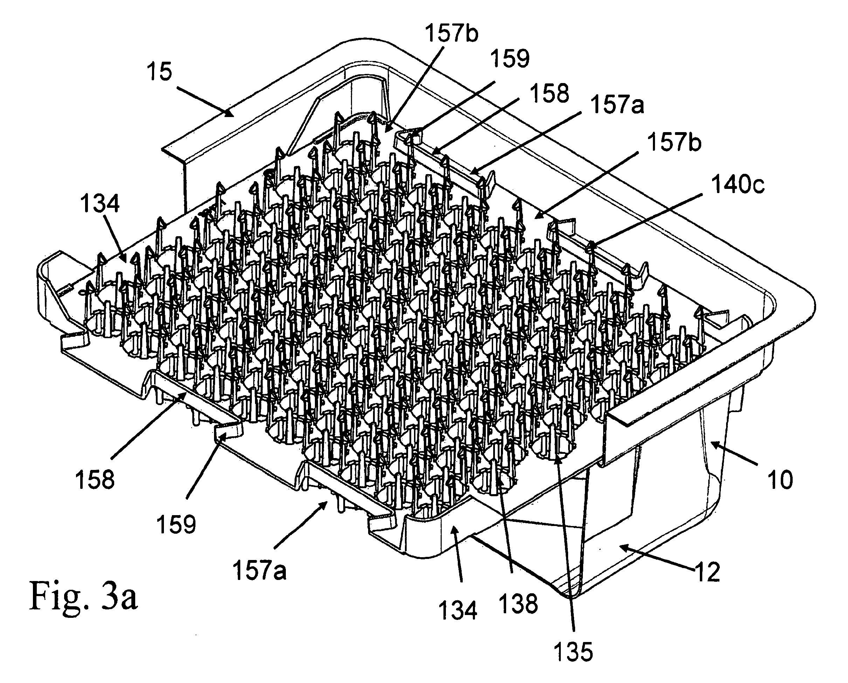

Die Fig. 3a zeigt eine Haltestruktur gemäß einer weiteren, auch unabhängig beanspruchbaren Ausführungsform der vorliegenden Erfindung in einer perspektivischen Draufsicht. Gemäß der Fig. 3a sind entlang der beiden Längsseiten der Halteplatte 134 alternierend und unter regelmäßigen Abständen zueinander eine Mehrzahl von Vorsprüngen 157b und Aussparungen 157a ausgebildet. Diese weisen, jeweils in Draufsicht betrachtet, eine insgesamt dreieckförmige oder polyedrische Grundfläche auf und sind korrespondierend zueinander ausgebildet, sodass diese unmittelbar miteinander verhakt werden können.

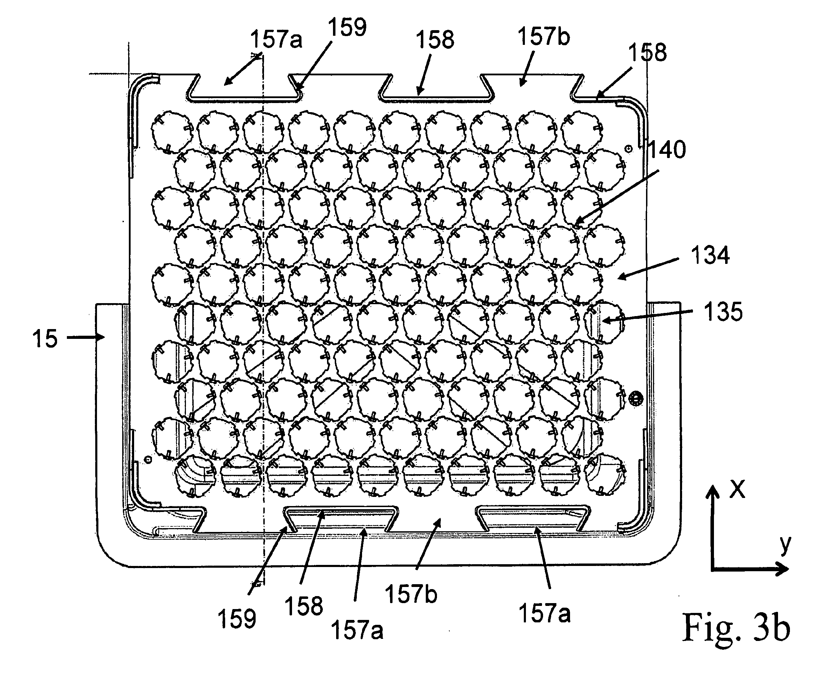

Wie man der Draufsicht gemäß der Fig. 3b entnehmen kann, können zwei Haltestrukturen so miteinander verhakt werden, dass diese in der Querrichtung (x) fluchtend angeordnet sind. Zu diesem Zweck ist im rechten unteren Eckbereich der Halteplatte 134 die Aussparung 157a nur halb ausgebildet. Im gegenüber liegenden rechten oberen Eckbereich der Halteplatte 134 ist hingegen der korrespondierende Vorsprung 157b ebenfalls nur halb ausgebildet und geht über in eine abgerundete Ecke der Halteplatte 134.

Durch die vorgenannte Auslegung der Vorsprünge 157b und Aussparungen 157a können zwei Haltestrukturen jedoch grundsätzlich auch so miteinander verhakt werden, dass diese in der Querrichtung (x) versetzt zueinander, also nicht-fluchtend, angeordnet sind.

Zum Verhaken zweier Haltestrukturen kann eine der Haltestrukturen mittels einer Hubeinrichtung in einer Richtung senkrecht zur Ebene der Halteplatte 134 angehoben werden. Anschließend werden die beiden Haltestrukturen aufeinander zubewegt, bis schließlich, in Draufsicht betrachtet, die Vorsprünge 157b und Aussparungen 157a der benachbarten Haltestrukturen miteinander überlappen. Durch anschließendes absenken der Halteplatte 134 senkrecht zur Ebene der Halteplatte 134 greifen schließlich die Vorsprünge 157b und Aussparungen 157a formschlüssig ineinander ein. Diese Vorgehensweise kann manuell aber auch voll- oder halbautomatisch erfolgen. Dabei können die Halteplatten 134 bereits mit Fläschchen bestückt sein. Grundsätzlich kann die Bestückung der Halteplatten 134 mit Fläschchen jedoch auch erst dann erfolgen, wenn die Halteplatten 134 miteinander verbunden sind.

Durch die vorgenannte Auslegung der Vorsprünge 157b und Aussparungen 157a wird insgesamt eine einhakende Wirkung in der Art einer Schwalbenschwanzverbindung realisiert. Wie dem Fachmann beim Studium der vorstehenden Beschreibung ohne weiteres ersichtlich sein wird, können grundsätzlich auch beliebige andere form- oder kraftschlüssige Verbindungstechniken zur vorübergehenden, lösbaren Verbindung zweier Haltestrukturen eingesetzt werden.

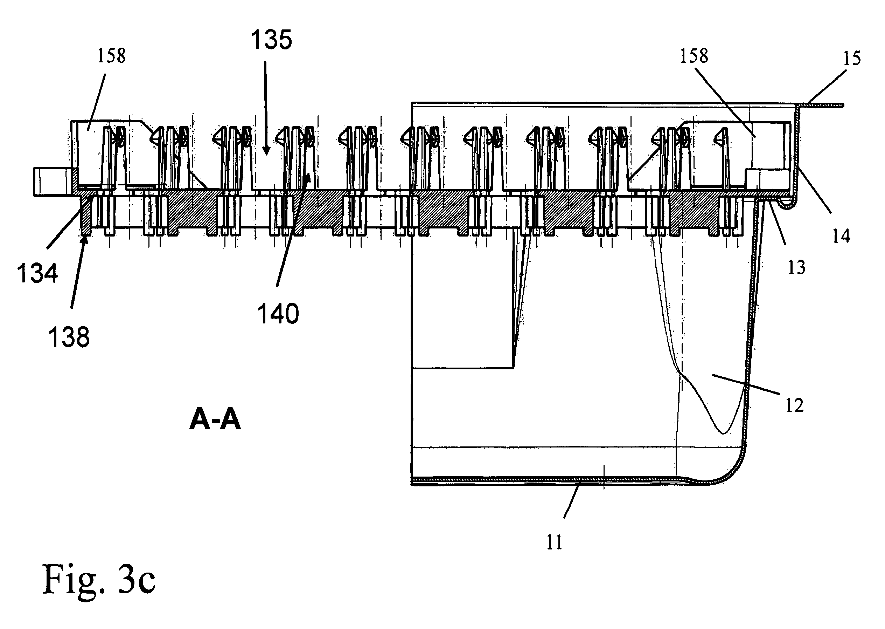

Wie man der perspektivischen Draufsicht gemäß der Fig. 3a entnehmen kann, sind entlang den Rändern der Vorsprünge 157b und Aussparungen 157a zumindest abschnittsweise Seitenwände 158, 159 ausgebildet, die rechtwinklig von der Oberfläche der Halteplatte 134 abragen. Diese Seitenwände 158, 159 folgen der Kontur der zugeordneten Aussparung 157a bzw. des zugeordneten Vorsprungs 157b und wirken als Anschlag- und Führungsfläche, die ein Überreinandergleiten bzw. Aufschieben der Halteplatten 134 verhindern. Genauer gesagt ist gemäß der Fig. 3b entlang der Vorderseite der Vorsprünge 157b am oberen Rand der Halteplatte 134 eine Seitenwand 158 ausgebildet, der sich im Bereich der benachbarten Aussparungen 157a eine Seitenwand 159 anschließt, die sich jedoch nicht über die gesamte Tiefe der Aussparungen (in x-Richtung) erstreckt. Am gegenüber liegenden unteren Rand der Halteplatte 134 sind die Seitenwände 158 dagegen entlang der Basis der Aussparungen 157a ausgebildet, während sich die abgewinkelten Seitenwände 159a entlang der abgewinkelten Seiten der Aussparungen 157a erstrecken, nicht jedoch über deren gesamte Tiefe (in x-Richtung). Gemäß der Fig. 3c ragen die Seitenwände 158, 159 weiter von der Oberseite der Halteplatte 134 ab als die Haltemittel 140, sodass, wenn die Halteplatten 134, wenn von den Haltemitteln 140 keine Behälter gehalten werden, unmittelbar aufeinander gestapelt werden können.

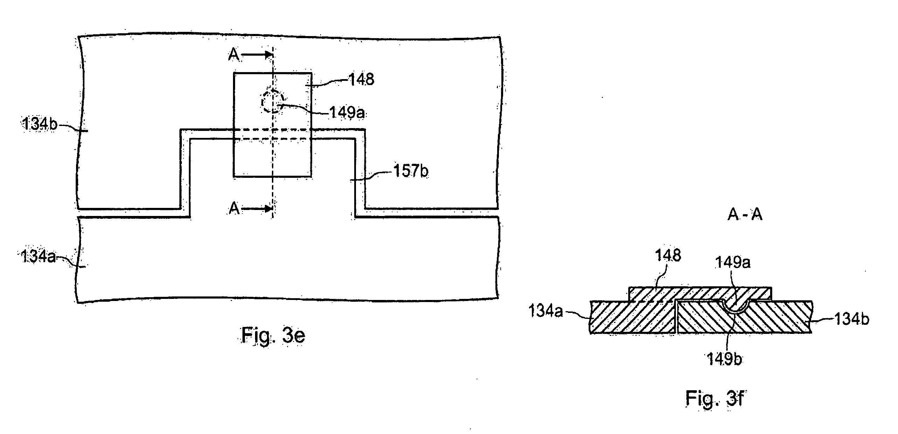

Wie in der stark vergrößerten Teildraufsicht gemäß der Fig. 3e dargestellt, liegen im verhakten Zustand die Seitenwände 158a der unteren Halteplatte 134a unmittelbar an den Seitenwänden 158b der obere Halteplatte 134b an. Ferner liegen auch die abgewinkelten Seitenwände 159b der oberen Halteplatte 134b unmittelbar an den abgewinkelten Seitenwände 159a der unteren Halteplatte 134a an.

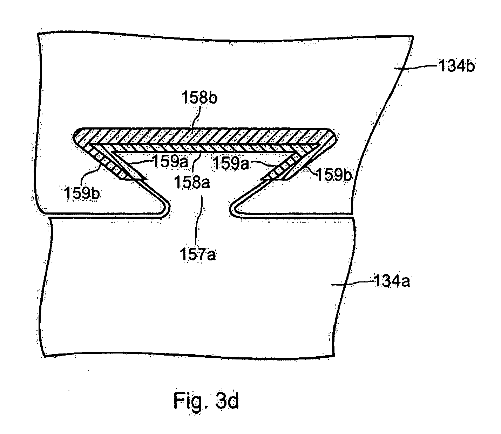

Die Figuren 3e und 3f zeigen als weiteres Beispiel für eine formschlüssige Verbindung in einer stark vergrößerten Teildraufsicht und in einem Teilschnitt entlang der Linie A-A gemäß der Fig. 3e die Verbindung zweier Halteplatten 134a, 134b nach einer weiteren Ausführungsform. Gemäß der Fig. 3e ragt von den hier rechteckförmig ausgebildeten Vorsprüngen 157b der unteren Halteplatte 134a eine elastische Zunge 148 in Richtung der zugeordneten Aussparung der oberen Halteplatte 134b rechtwinklig ab. Am vorderen Ende der elastischen Zunge 148 ist ein kugelförmiger Vorsprung 149a ausgebildet, der in eine korrespondierende Aufnahme 149b auf der Oberseite der oberen Halteplatte 134b eingreift. Die Halteplatten 134a, 134b können zur Verbindung aufeinander zu geschoben werden, bis das vordere Ende der elastischen Zunge 148 mit dem Vorsprung 149a schließlich in Anlage mit der Oberseite der oberen Halteplatte 134b gelangt. Um ein Aufgleiten der beiden Halteplatten 134a, 134b zu verhindern, können auch nach dieser Ausführungsform Anschlag- und Führungsflächen vorgesehen sein, insbesondere in Gestalt von Seitenwänden, die rechtwinklig von der Oberseite der Halteplatten 134a, 134b abragen, wie vorstehend anhand der Fig. 3a beschrieben. Bei der Ausführungsform nach der Fig. 3d wären solche Seitenwände insbesondere seitlich neben den elastischen Zungen 148 vorzusehen.

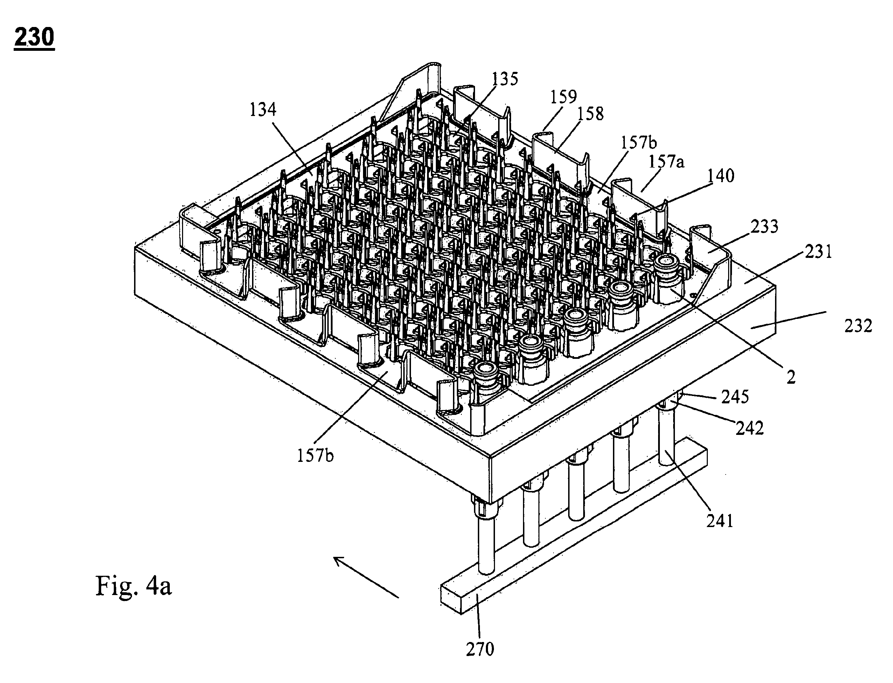

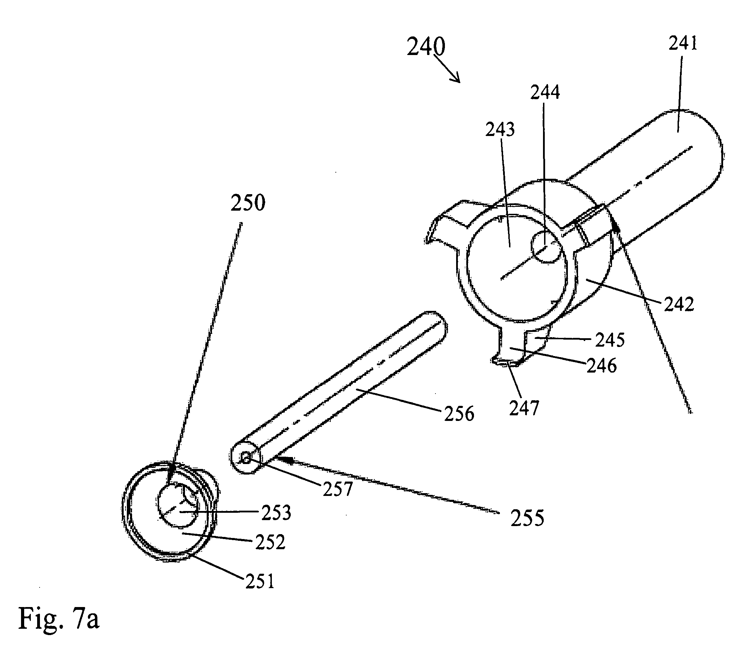

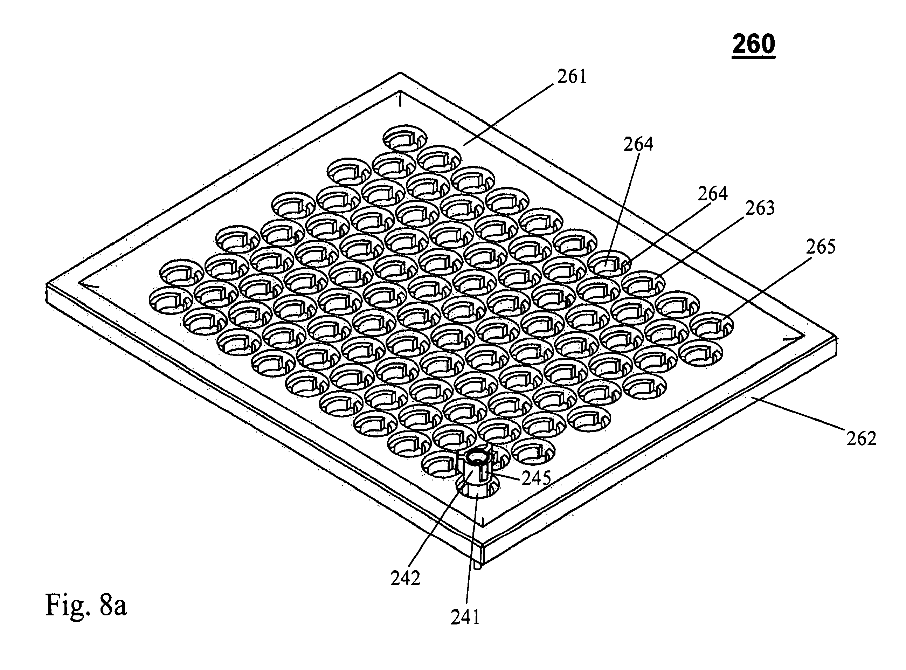

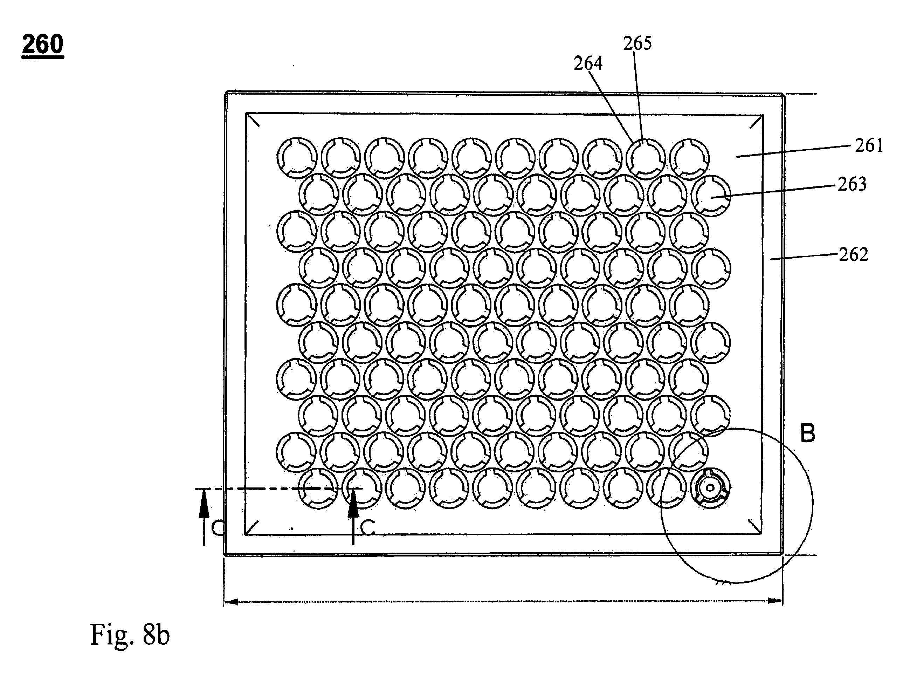

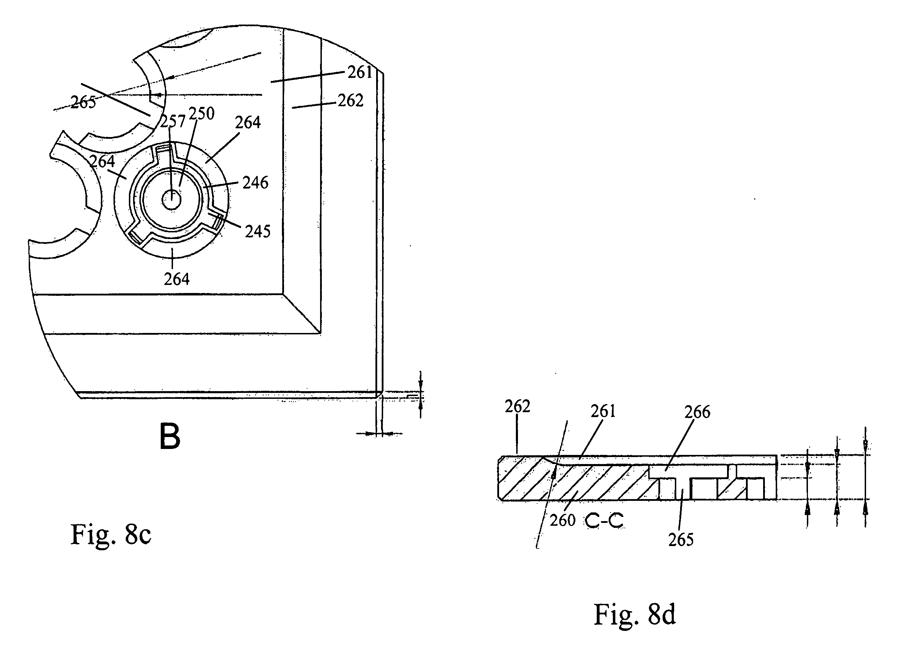

Die Fig. 4a zeigt einen Ausschnitt aus einer Bearbeitungsstation zur Bearbeitung oder Verarbeitung von Fläschchen, während diese in einer angehobenen Stellung in einer Haltestruktur gemäß der Fig. 2d gehalten sind. Die Bearbeitungsstation weist eine Verstelleinrichtung 230 mit einem Ausheber 270 auf, der mehrere Stößel (vgl. Fig. 7a) trägt und führt. Die Halteplatte 134 mit Ihren am Rand ausgebildeten Vorsprüngen 157b und Aussparungen 157a ist in der korrespondierend dazu ausgebildeten Führungsplatte 231 aufgenommen. In der von der Seitenwand 232 ausgebildeten Aufnahme kann eine Lochplatte, wie in der Fig. 8a dargestellt, exakt positioniert aufgenommen sein, um die Stößel exakt relativ zu der Halteplatte mit den daran gehaltenen Fläschchen 2 zu positionieren.

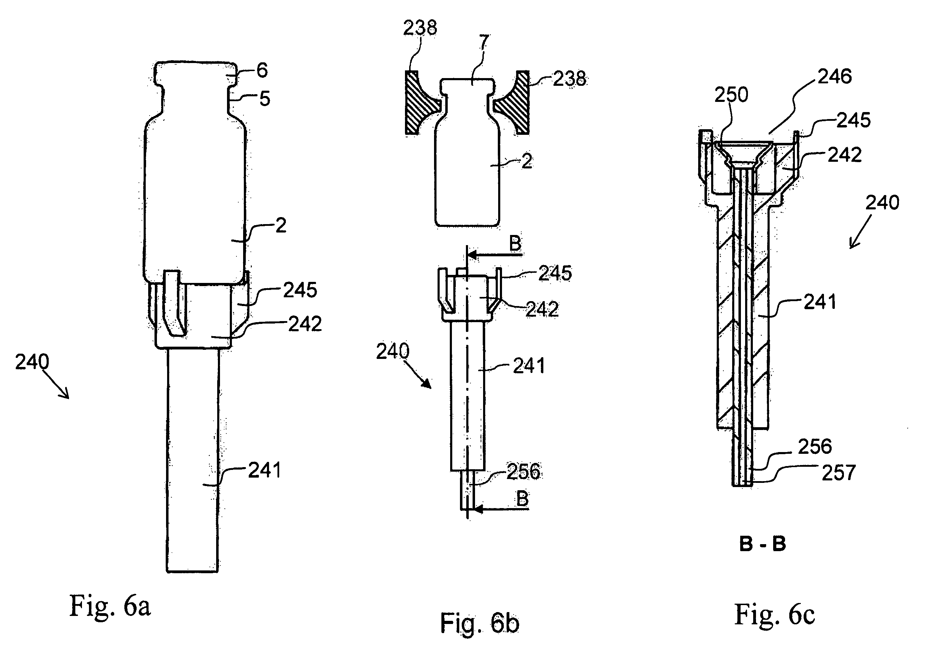

Gemäß der Fig. 7a weist jeder als Vertikal-Verstelleinrichtung wirkende Stößel 240 einen Schaft 241 mit einem Haltering 242 auf, von dessen Umfangsrand unter gleichmäßigen Winkelabständen zueinander drei Haltearme 245 abragen, deren Oberseiten gemeinsam eine Ebene aufspannen, die als Auflagefläche 246 dient, auf welcher der Boden des zu haltenden Fläschchens aufliegt, wie in der Fig. 6a dargestellt. Die Fläschchen werden von den Vorsprüngen 247, die senkrecht von den Haltearmen 245 abragen, gehalten. Dabei wird der untere Rand der Fläschchen von den Vorsprüngen 247 umgriffen, wodurch die Position der Fläschchen auf dem zugeordneten Stößel exakt vorgegeben werden kann und ein seitliches Verrutschen der Fläschchen, während diese von einem Stößel abgestützt sind, verhindert werden kann. Zu diesem Zweck können die Vorsprünge 247 den unteren Rand eines Fläschchens jeweils eng anliegend umgreifen, insbesondere klemmen. Zweckmäßig sind hierzu die Haltearme 246 und/oder Vorsprünge 247 ausreichend elastisch, beispielsweise aus einem Kunststoff ausgebildet, wodurch Toleranzen am unteren Ende der Fläschchen ohne weiteres kompensiert werden können.

Ein solcher Stößel kann ein Fläschchen gegen die von den Haltearmen der Halteplatte ausgeübte Haltekraft vertikal relativ zu der Halteplatte verstellen, um diese zur Bearbeitung oder Verarbeitung in einer Prozessstation in eine angehobene Position zu verschieben.

Wie in der Fig. 7a gezeigt, ist in dem Schaft 241 eine axiale Durchgangsbohrung 244 ausgebildet, die als axiale Führung für die rohrförmige Stange 256 eines Saugers 250 dient. In der Stange 256 ist eine axiale Durchgangsbohrung 257 ausgebildet, die mit einem Unterdruck-Erzeuger, beispielsweise einer Saugpumpe, verbunden ist. Die Stange 256 kann in dem Schaft 241 axial verstellt werden.

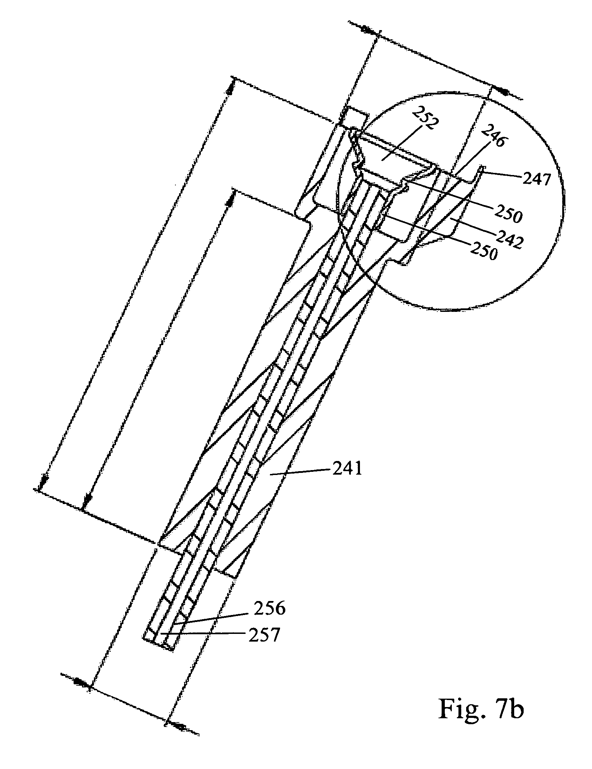

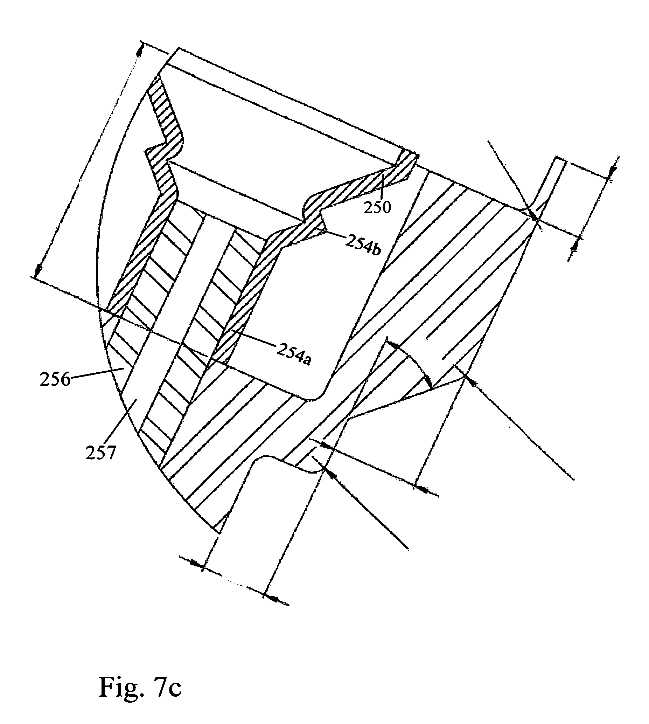

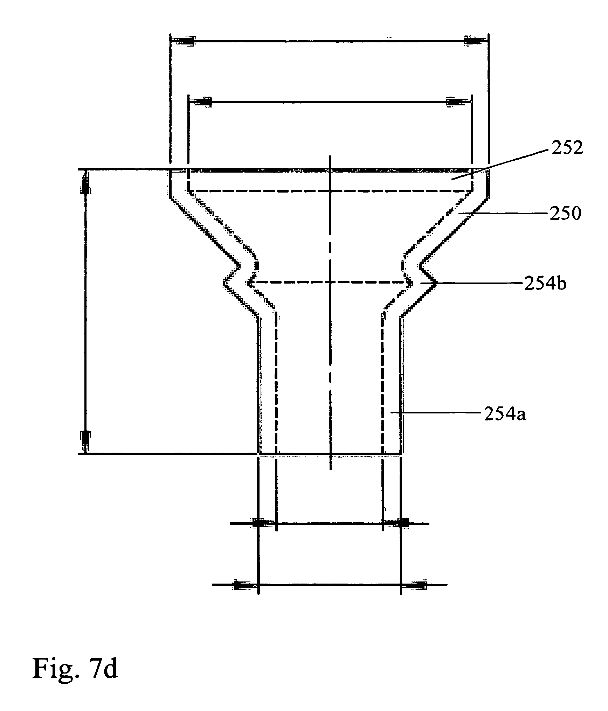

Auf das vordere freie Ende der Stange 256 ist eine Saugglocke 250 aus einem elastischen Material, beispielsweise Gummi oder Silikon, aufgesteckt. In der Saugglocke 250 ist ein trichterförmiger Hohlraum 252 ausgebildet, der über die Öffnung 253 und die Durchgangsbohrung 257 der Stange 256 mit dem Unterdruck-Erzeuger (nicht dargestellt) kommuniziert. Die Saugglocke 250 ist mit einer oder mehreren Wellungen 254b (vgl. Fig. 7c und 7d) versehen und kann sich durch Hochschieben der Stange 256 eng anliegend an den Boden des zugeordneten Fläschchens anschmiegen. Wie man der Fig. 7b entnehmen kann, kann die Saugglocke 250 vollständig in einer Aufnahme aufgenommen werden, die in dem Haltering 242 am vorderen Ende des Stößels ausgebildet ist. Die Saugglocke 250 kann in dieser Aufnahme vollständig versenkt werden, sodass diese nicht über die Auflagefläche 246 des Halterings 242 vorsteht. Allerdings kann die Saugglocke 250 durch Hochschieben der Stange 256 soweit vorgeschoben werden, dass diese schließlich in Kontakt mit dem Boden eines zu verstellenden Fläschchens gelangt. Dies erfordert eine gewisse Verschieblichkeit der Stange 256 relativ zu dem Schaft 241 des Stößels 240.

Nachfolgend wird anhand der Figuren 6a bis 6c ein Verfahren gemäß der vorliegenden Erfindung zur Bearbeitung oder Verarbeitung von Fläschchen zunächst grundlegend beschrieben.

Gemäß der Fig. 6a wird der Schaft 240 zum Anheben eines Fläschchens 2 in eine angehobene Position ausgehend von der Ruhestellung (vergleichbar zur Stellung gemäß der Fig. 6b) solange angehoben, bis der Haltering 242 am vorderen Ende des Schafts 241 in Anlage mit dem Boden eines Fläschchens 2 gelangt. In dieser Stellung ist der Boden des Fläschchens auf der Auflagefläche am vorderen Ende des Halterings 242 abgestützt und wird der untere Rand des Fläschchens von den Haltearmen 245 und den daran ausgebildeten Vorsprüngen 247 (vgl. Fig. 7a) umgriffen. In dieser Stellung kann die Saugglocke 250 in die Aufnahme 243 im Haltering 242 zurückgefahren sein.

In der angehobenen Position wird das Fläschchen entweder an weitere Fördermittel (nicht dargestellt) übergeben oder verbleibt in unmittelbarer Nähe des Stößels 240. Zur Bearbeitung oder Verarbeitung der Fläschchen können diese an oder in einer Prozessstation grundsätzlich mittels gesonderter Haltemittel 238, wie schematisch in der Fig. 6b gezeigt, gehalten werden. In einem solchen Fall kann der Stößel die Fläschchen 2 zur Bearbeitung oder Verarbeitung freigeben, beispielsweise nach unten oder seitlich weggefahren oder weggeschwenkt werden, wie in der Fig. 6b gezeigt.

Selbstverständlich kann jedoch der Stößel 240 auch alleinig zum Halten oder Abstützen der Fläschchen 2 während der Bearbeitung oder Verarbeitung verwendet werden. In einem solchem Fall verbleibt der Stößel dann zur Bearbeitung oder Verarbeitung an oder in der Prozessstation auf einer geeigneten Höhenposition und fixiert dabei die Position der Fläschchen 2 relativ zu der Prozessstation. Während der Bearbeitung oder Verarbeitung kann der Stößel dabei weiterhin zu einer geeigneten Verstellung der Fläschchen 2 in Axialrichtung und/oder zum Drehen der Fläschchen (der Stößel 240 wirkt gleichzeitig als Drehteller) und/oder Neigen oder Verschwenken der Fläschchen (der Stößel 240 wird verkippt) verwendet werden. Hierzu ist eine geeignete Haltekraft des Halterings 242 zum Halten der Fläschchen 2 vorzusehen.

Während der Bearbeitung oder Verarbeitung der Fläschchen 2 können diese dabei grundsätzlich auch weiterhin in den Öffnungen oder Aufnahmen der Haltestruktur aufgenommen oder zumindest geführt sein, beispielsweise um deren Position während der Bearbeitung oder Verarbeitung weiter zu stabilisieren. Grundsätzlich können die Fläschchen 2 während der Bearbeitung oder Verarbeitung jedoch auch vollständig aus den Öffnungen oder Aufnahmen der Haltestruktur herausgehoben und freigegeben sein.

Nach erfolgter Bearbeitung oder Verarbeitung in oder an einer Prozessstation sollen die Fläschchen wieder in eine tiefere Stellung zurückgezogen werden, insbesondere in die Öffnungen oder Aufnahmen einer Haltestruktur, wie vorstehend beschrieben. Hierzu wird, wie in dem Teilschnitt gemäß der Fig. 6c dargestellt, die Saugglocke 250 so positioniert, dass diese bündig zur Auflagefläche 246 des Halterings 242 ist oder geringfügig über diese hinausragt oder zu dieser zurückgefahren ist. Der Stößel 240 wird zum Absenken der Fläschchen 2 so positioniert, dass die Auflagefläche 246 erneut in die Kontakt mit dem Boden des Fläschchens 2 gelangt. Sofern der Stößel 240 während Bearbeitung oder Verarbeitung der Fläschchen 2 nicht zurückgefahren wurde, ist hierzu keine erneute Verstellung erforderlich. Durch Anlegen eines Unterdrucks an die Saugglocke 250 schmiegt sich diese dem Boden des Fläschchens 2 an und kann dieser hin zu der Auflagefläche 246 des Stößels gesaugt werden. Ggf. kann nun das Haltemittel 238 der Prozessstation (vgl. Fig. 6b) das Fläschchen 2 wieder freigeben. Anschließend wird der Stößel 240 abgesenkt. Das Fläschchen folgt der Absenkbewegung des Stößels 240.

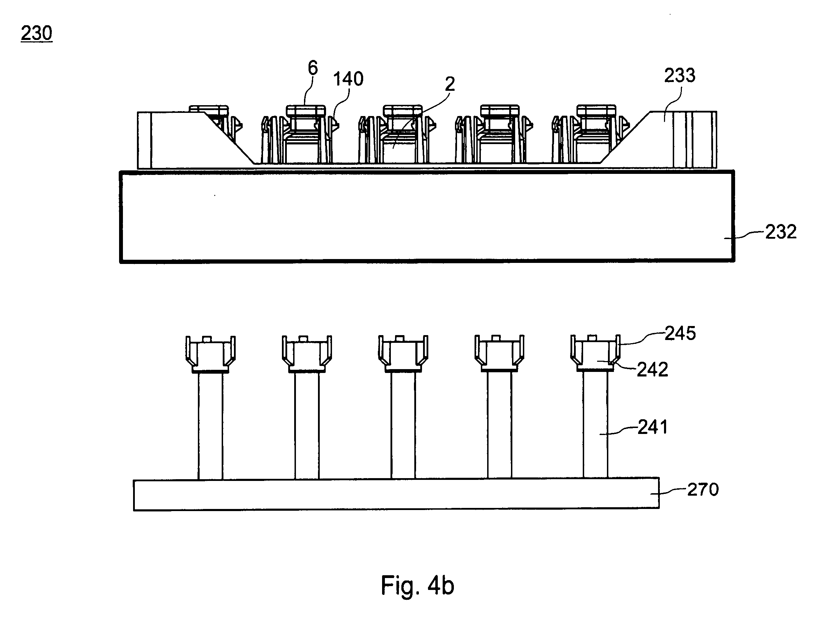

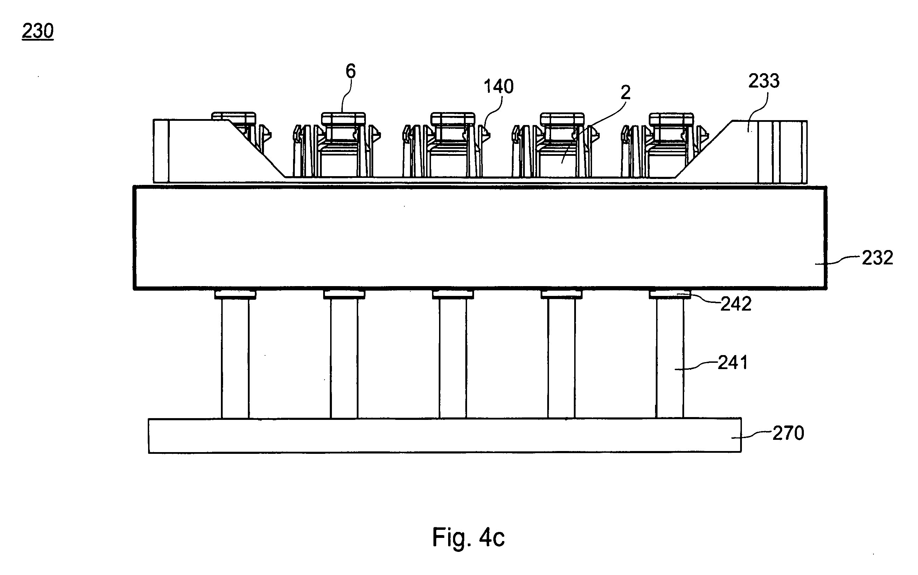

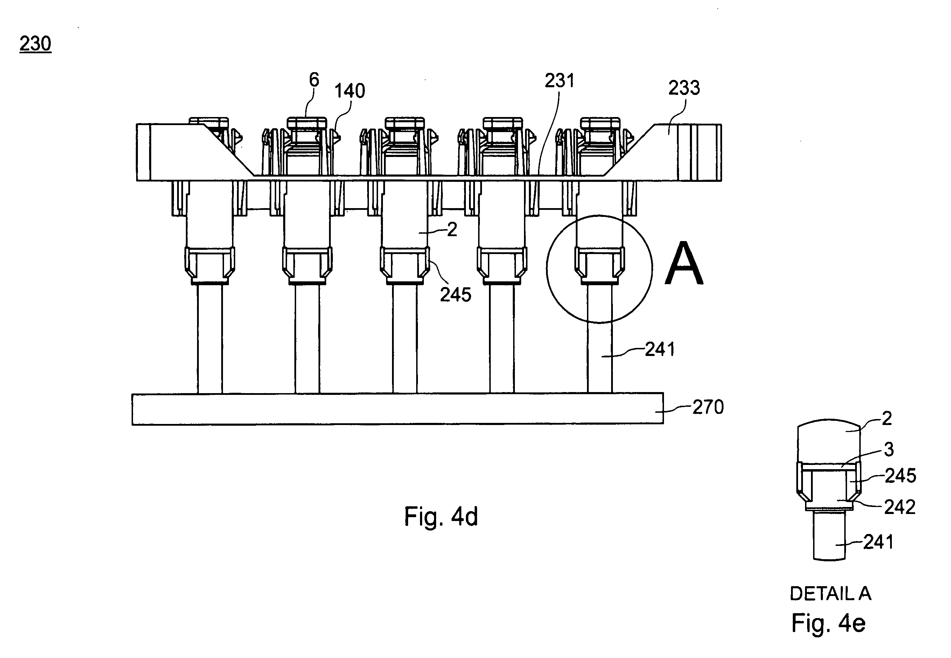

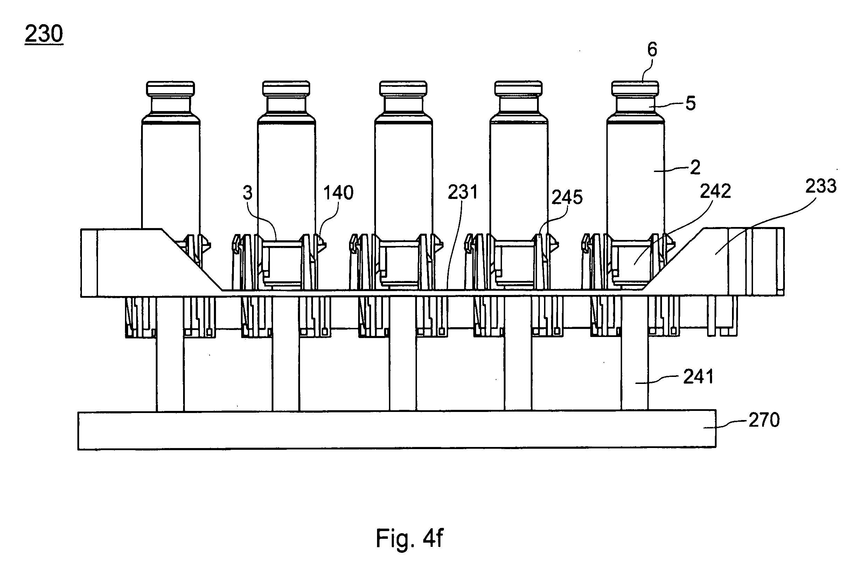

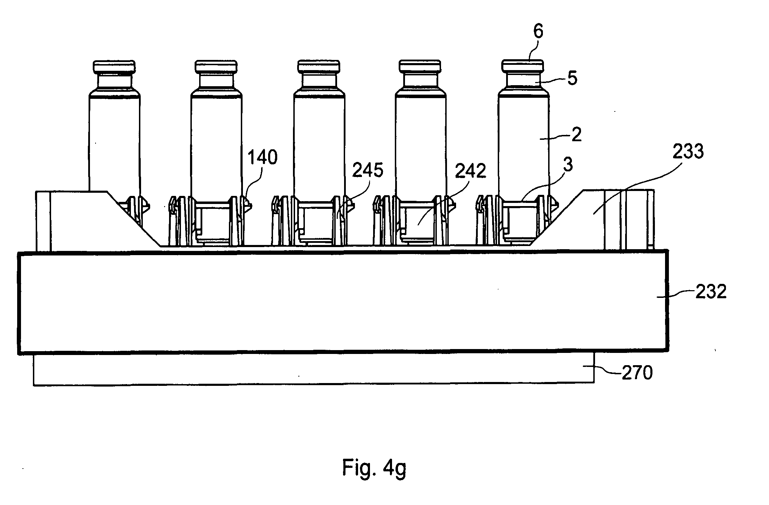

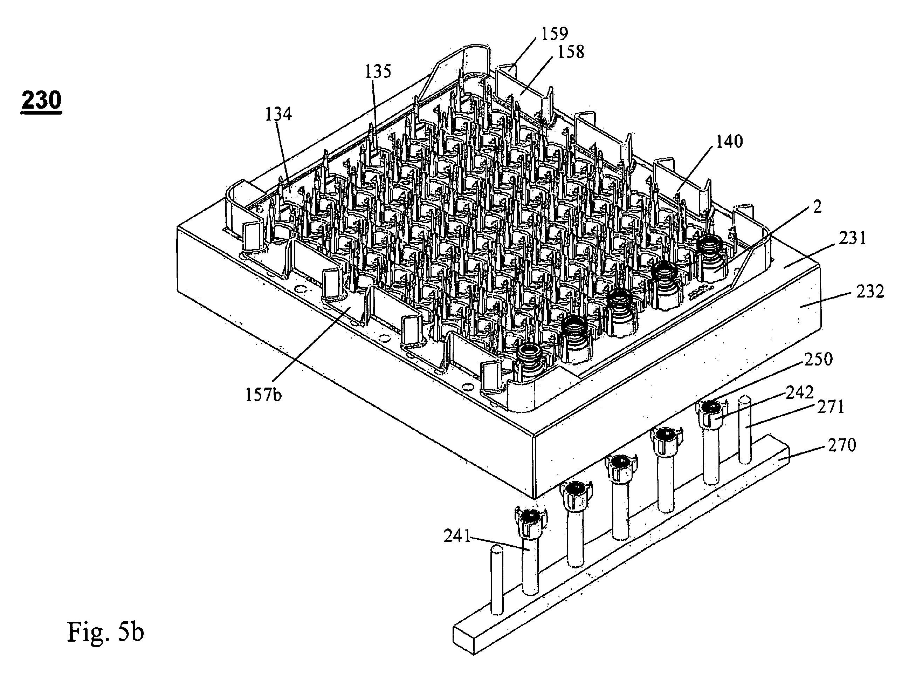

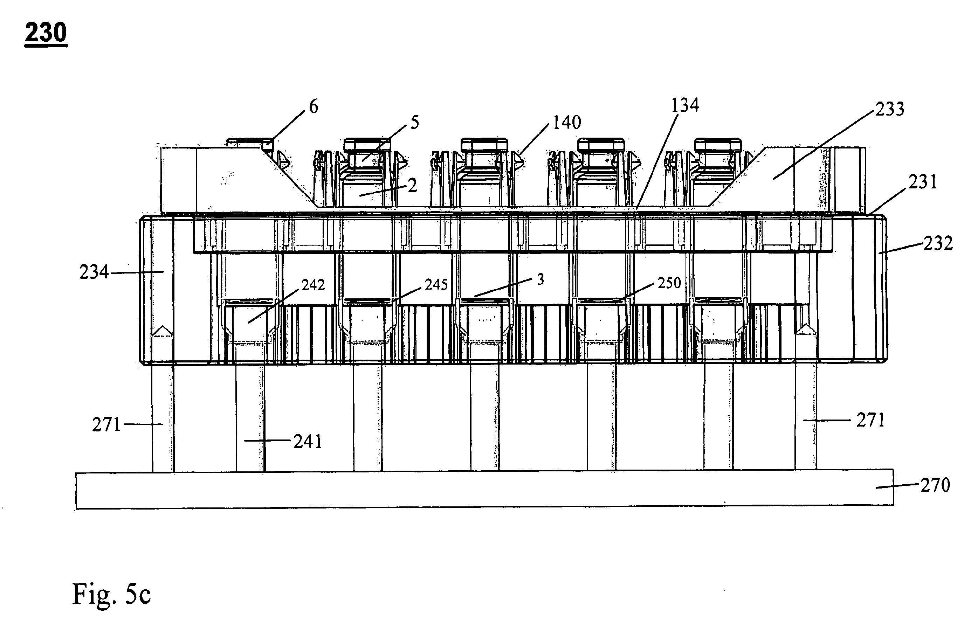

Die Figuren 4b bis 4g zeigen die vorstehend beschriebene Sequenz von Prozessschritten zum Überführen der Fläschchen in der Bearbeitungsstation gemäß der Fig. 4 aus einer abgesenkten Ausgangsstellung (Fig. 4b) in eine angehobene Stellung (Fig. 5h*) und umgekehrt. Den Figuren 4b bis 4g liegt dabei eine Vertikal-Verstelleinrichtung 270 zugrunde, die eine Mehrzahl von Stößeln 241, wie vorstehend beschrieben, aufweist, die entsprechend der regelmäßigen Anordnung der Fläschchen 2 in der Haltestruktur angeordnet sind. Gemäß der Fig. 4b sind insgesamt fünf Stößel unter gleichmäßigen Abständen zueinander und parallel zu einer Längsseite der Haltestruktur verlaufend angeordnet, in Zuordnung zu den fünf Fläschchen 2, die in der Haltestruktur gehalten sind. Somit können durch gleichzeitiges Anhaben aller Stößel die von der Haltestruktur gehaltenen Fläschchen 2 reihenweise verstellt werden.

Zunächst werden die Fläschchen von einem Greifer gegriffen, wie beispielsweise in der Fig. 2j oder 2k dargestellt, oberhalb der Halteplatte gegriffen und dort gehalten. Oder die Fläschchen werden lose in die Öffnungen der Halteplatte eingesteckt oder eingelegt, wie in der Fig. 4b dargestellt. Die Stößel 240 fahren nun von unten vertikal hoch, wie in der den Figuren 4c und 4d (ohne Seitenwand 232) sowie in der stark vergrößerten Darstellung gemäß der Fig. 4e dargestellt. Dabei können die Haltearme 245 der Stößel 240 im Zusammenwirken mit den Ausspamngen 265 einer Lochplatte 260 (vgl. Fig. 8a), wie nachfolgend beschrieben, für eine Zentrierung der Böden der Fläschchen 2 auf den Stößeln 240 sorgen. Anschließend werden die Stößel 240 weiter vertikal nach oben verstellt, um die Fläschchen 2 vertikal anzuheben. Ausgehend von der Stellung gemäß der Fig. 4c, in welcher die Fläschchen 2 von den elastischen Haltearmen 140 unterhalb des oberen Rands 6 gehalten werden, wie vorstehend beschrieben, werden beim Hochschieben der Fläschchen 2 die elastischen Haltearme 140 dabei aufgespreizt oder seitlich weggeschwenkt und gleiten dann entlang der Seitenwand der Fläschchen 2. Wie vorstehend beschrieben, können die Fläschchen 2 zur Bearbeitung oder Verarbeitung in einer Prozessstation vollständig über die Haltearme 140 hinaus angehoben werden, können jedoch auch grundsätzlich noch in einem Eingriff mit diesen stehen.

Das Anlegen eines Unterdrucks an die Saugglocke der Stößel bewirkt im Anschluss an die Bearbeitung oder Verarbeitung der Fläschchen 2 das Ansaugen der zugeordneten Fläschchen 2 an den Stößel 240. Durch Herunterziehen der Stößel 240 kann die von den Haltearmen 140 ausgeübte Halte- oder Rückstellkraft überwunden werden und die Fläschchen 2 so erneut in die abgesenkte Haltestellung in der Halteplatte überführt werden. Durch geeignete Ausgestaltung der Wellungen der Saugglocke (vgl. Fig. 7b) und geeignete Wahl deren Materials kann ein Abreißen des Kontakts zwischen Saugglocke und Fläschchenboden wirkungsvoll verhindert werden. Beim Absenken des Stößels wirkt dieser somit als elastischer Sauger. Der Greifer (vgl. Fig. 2j und 2k) kann nun entfernt werden; die Fläschchen können nach unten in die Haltestruktur (Nest) bewegt werden und wird dann dort erneut von den Haltemitteln an der Haltestruktur gehalten werden, wie vorstehend beschrieben.

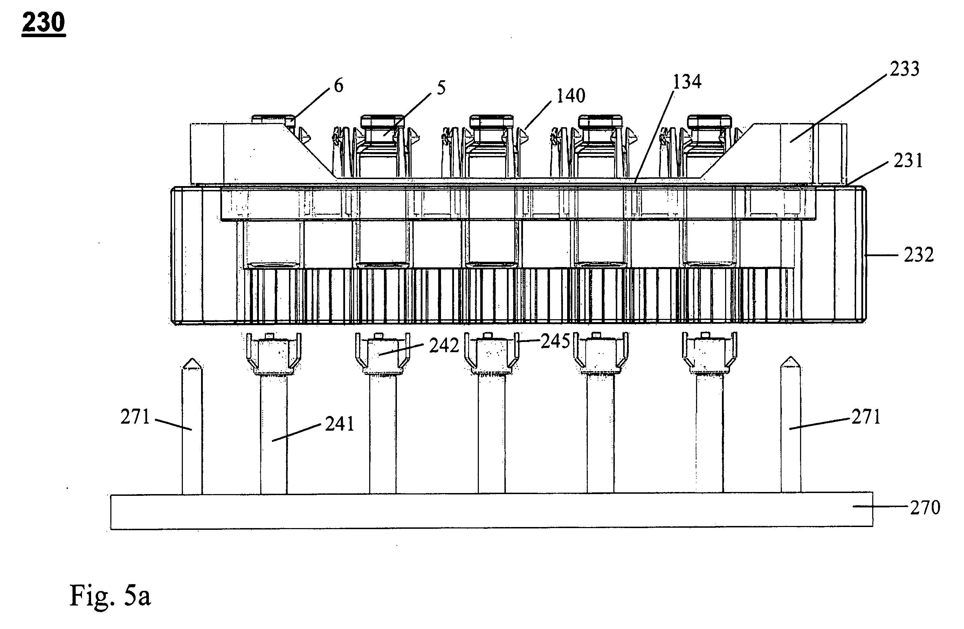

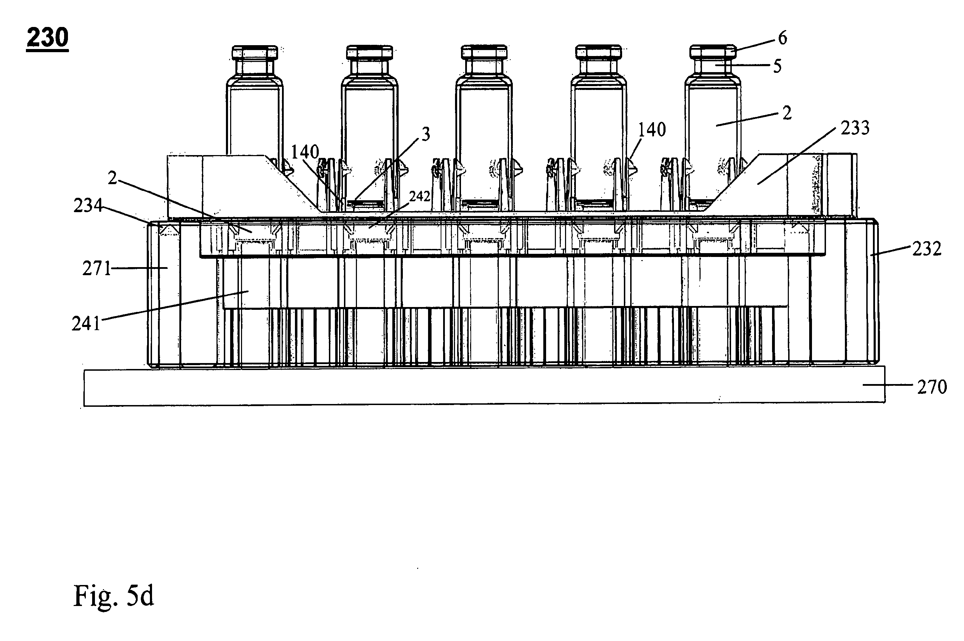

Die Figuren 5a bis 5e zeigen eine entsprechende Sequenz von Prozessschritten für eine weitere Ausführungsform, bei der an der Vertikal-Verstelleinrichtung 270 ferner mehrere zylindrische Positionierungszapfen 271 vorgesehen sind, die im Zusammenwirken mit einer korrespondierend ausgebildeten Positionierungshülse 234 in der Seitenwand 232 der Prozessstation 230 oder der Haltestruktur 134 für eine präzise Relativ-Positionierung von Vertikal-Verstelleinrichtung 270 und Haltestruktur 134 sorgen. In der angehobenen Stellung der Stößel 240 gemäß der Fig. 5d gelangt das vordere Ende der Positionierungszapfen 271 in Anlage zu dem Träger 134, wodurch gleichzeitig auch ein weiteres Anheben der Stößel 240 unterbunden werden kann.

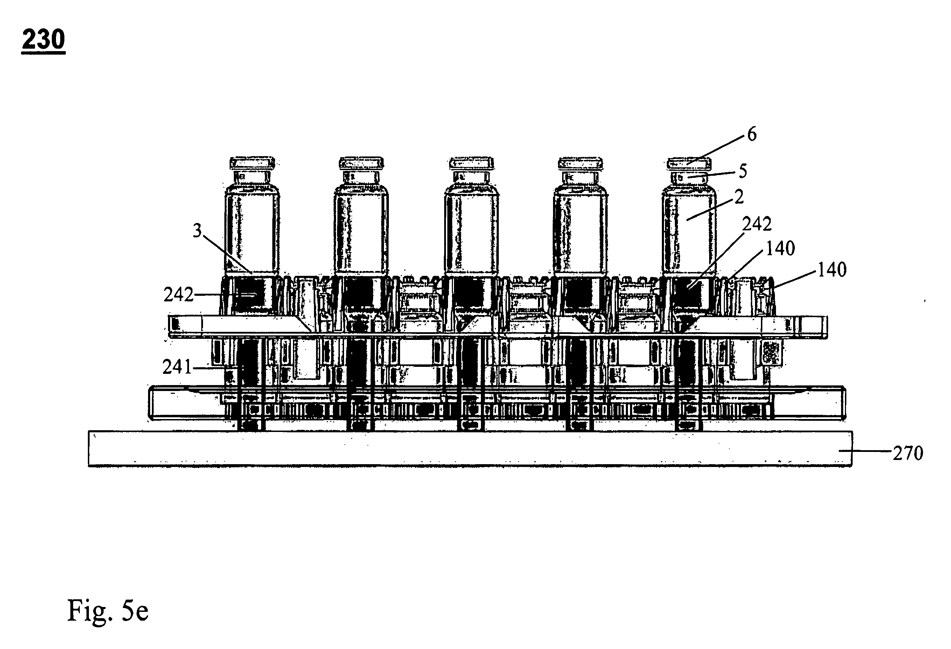

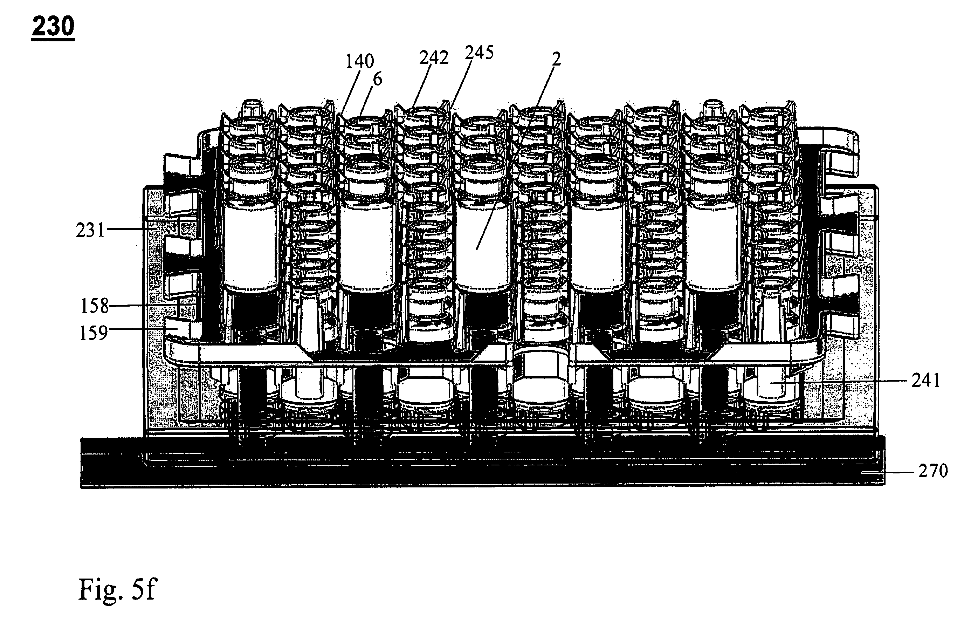

Die Fig. 5e zeigt dabei die Prozessstation 230 in der Stellung gemäß der Fig. 5d, jedoch ohne den Blick auf die Fläschchen und Stößel verdeckende Seitenwände. Schließlich zeigt die Fig. 5f die Prozessstation in einer Stellung der Vertikal-Verstelleinrichtung 270, in welcher der Eingriff sämtlicher Behälter 2 mit den Haltearmen 140 der Haltestruktur vollständig gelöst ist. Dabei ist in der Fig. 5f jede zweite Reihe von Fläschchen 2 nicht dargestellt, um den Blick auf die oberen Enden der Stößel 241 mit dem Haltering 242 und den Haltearmen 245 in der angehobenen Stellung der Stößel 241 freizugeben.

Zum Ausheben der Fläschchen braucht kein Unterdruck an die Saugglocken der Stößel angelegt zu werden. Bei ganz ausgefahrener Position (oberste Stellung) der Stößel kann allerdings durch Unterdruck eine zusätzliche Sicherung der Fläschchen ermöglicht werden.

Zur exakten Positionierung der Stößel 240 in Übereinstimmung mit der Anordnung der Fläschchen in der Halteplatte kann eine in der Fig. 8a dargestellte Lochplatte 260 vorgesehen sein. In der Lochplatte 260 ist für jedes Fläschchen ein Loch 263 mit ringförmiger Auflage 266 vorgesehen, die durch eine Mehrzahl von unter gleichen Winkelabständen zueinander in dem Loch 263 angeordneten gekrümmten Halteteilen 264 ausgebildet wird, die von Aussparungen 265 unterbrochen sind, durch welche die jeweiligen Haltearme 245 der Stößel 240 nach oben geführt werden können. Diese Lochplatte 260 kann geeignet unterhalb der Haltestruktur mit den von dieser gehaltenen Fläschchen angeordnet sein und die Stößel und deren Haltearme beim Anheben so führen, dass die Haltearme den unteren Rand der Fläschchen präzise umgreifen.

Gemäß einer weiteren Ausführungsform kann die in der Fig. 8a abgebildete Lochplatte 260 auch als Haltestruktur zum Halten der Fläschchen (nicht gezeigt) verwendet werden, in welchem Fall die Boden der Fläschchen in den kreisförmigen Aussparungen 265 aufgenommen sind und unmittelbar auf den gekrümmten Halteteilen 264 aufliegen, um an der Lochplatte abgestützt zu sein.

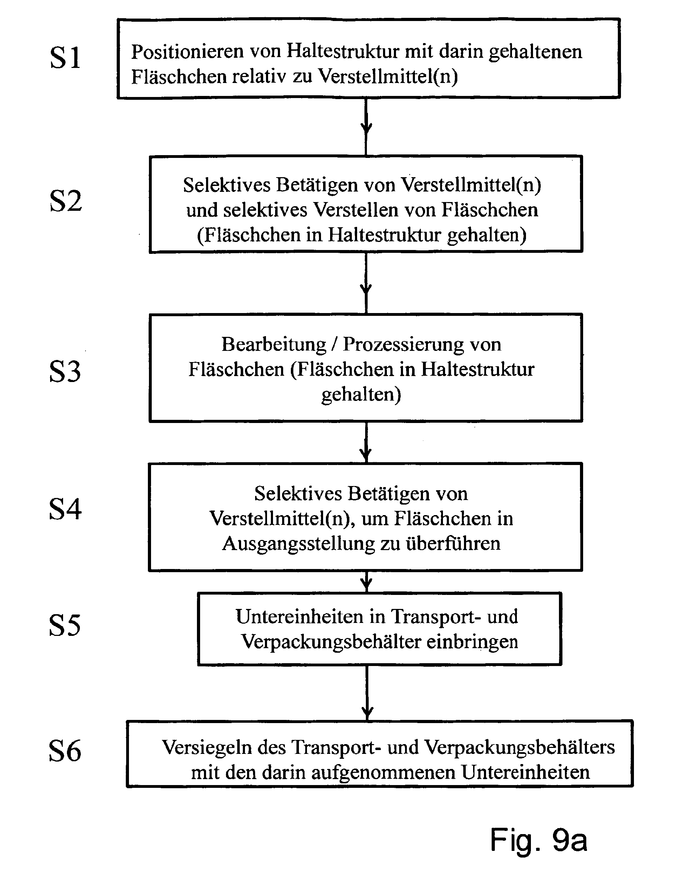

Das schematische Flussdiagramm gemäß der Fig. 9a fasst die Schritte zur Prozessierung oder Bearbeitung von Fläschchen mittels einer Bearbeitungsstation gemäß der Fig. 4a nochmals zusammen.

Somit wird die jeweilige Haltestruktur mit den daran gehaltenen Behältern, insbesondere Fläschchen (vials), Ampullen, Karpullen oder Spritzenkörpern, in dem Prozessschritt S1 relativ zu der Vertikal-Verstelleinrichtung geeignet positioniert. Diese Positionierung kann bereits dann erfolgen, wenn die Haltestruktur noch in dem Transport- und Verpackungsbehälter aufgenommen ist, wie beispielhaft in der Fig. 2a dargestellt. In einem solchen Fall könnte die Haltestruktur 134 beispielsweise mittels eines Greifers oder dergleichen über die Zugriffsöffnungen 29 (vgl. Draufsicht gemäß der Fig. 2b) gegriffen und fixiert und dann der Transport- und Verpackungsbehälter 1 nach unten abgezogen werden. Anschließend kann die Vertikal-Verstelleinrichtung, beispielsweise wie in der Fig. 4a dargestellt, die Behälter reihenweise in die angehobene Position anheben (Prozessschritt S2), in welcher die Behälter dann bearbeitet oder weiter verarbeitet werden (Prozessschritt S3).

Bei dieser Bearbeitung oder Verarbeitung kann es sich um beliebige Prozessschritte handeln. Beispielhaft seien aufgeführt: ein Füllen der Behälter mit einer Substanz oder substanzhaltigen Lösung; eine Sterilisierung oder Wärmebehandlung der Behälter; ein Waschvorgang; eine Wärmbehandlung oder anderweitige Behandlung des Inhalts der Behälter, beispielsweise Bestrahlung; ein Verschließen der Behälter, beispielsweise mit einem Stopfen; das Aufbringen eines Metalldeckels auf einem Verschluss am oberen Rand der Behälter, beispielsweise durch Bördeln oder Crimpen einer Metallkappe; ein Beschriften oder Labeln der Behälter; ein Wiegen der Behälter. Diese Bearbeitung oder Verarbeitung der Behälter in der angehobenen Position im Prozessschritt S3 kann erfolgen, wenn die Behälter vollständig von den Haltemitteln der Haltestruktur freigegeben sind, kann jedoch grundsätzlich auch erfolgen, während die Behälter auch weiterhin in den Öffnungen oder Aufnahmen der Haltestruktur aufgenommen oder zumindest geführt oder in ihrer Position geführt oder fixiert sind.

Anschließend wird eine Vertikal-Verstelleinrichtung, die identisch zu der zuvor verwendeten Vertikal-Verstelleinrichtung sein kann aber auch eine andere Vertikal-Verstelleinrichtung sein kann, in dem Prozessschritt S4 betätigt, um die Behälter in ihre abgesenkte Ausgangsstellung zu überführen, also insbesondere durch Herunterziehen in die Öffnungen oder Aufnahmen der Haltestruktur, wie vorstehend beschrieben. In der abgesenkten Ausgangsstellung können die Behälter erneut in die Haltemittel der Haltestruktur eingreifen, um von diesen erneut gehalten oder abgestützt zu sein, was jedoch nicht zwingend erforderlich ist.

Anschließend kann die Haltestruktur mit den daran gehaltenen Behältern optional in den zuvor verwendeten Transport- und Verpackungsbehälter oder einen anderen Transport- und Verpackungsbehälter eingebracht werden und dieser für einen sterilen Transport in der üblichen Weise verschlossen oder versiegelt werden (Prozessschritt S6), beispielsweise durch Aufkleben einer sterilen Schutzfolie, beispielsweise einer Tyvek®-Folie.

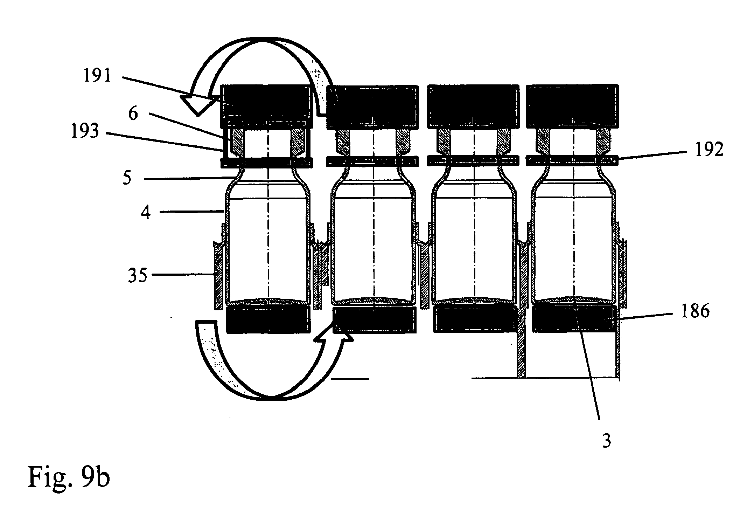

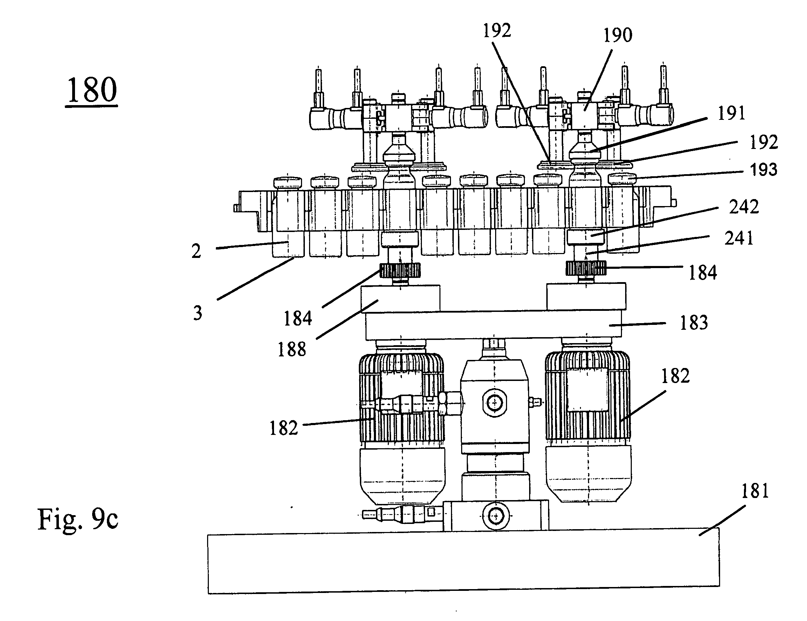

Die Fig. 9b zeigt als Beispiel für eine Bearbeitung der Behälter in der angehobenen Stellung das Aufbringen eines Metalldeckels auf den oberen Rand der Behälter. Wie man der vergrößerten Darstellung gemäß der Fig. 9b entnehmen kann, ist in der angehobenen Stellung der obere Rand der Behälter 2 mit dem darin eingesetzten Stopfen und einem Metalldeckel 193, beispielsweise einer Aluminiumkappe, in einer zugeordneten Zentrierscheibe 191 aufgenommen, um die Drehbewegung des Behälters beim Drehen des Drehtellers 186 zu zentrieren. Dieser Drehteller 186 kann Bestandteil der vorgenannten Vertikal-Verstelleinrichtung sein oder erst nach Anheben der Behälter in die angehobene Stellung mit diesen in Eingriff gebracht werden. Beim Drehen des Behälters 2 gelangt eine an dem Arm 190 gelagerte Bördelscheibe 192 in Anlage mit dem Metalldeckel 193 und bördelt diesen durch Umformung geeignet um, um so den Behälter steril zu versiegeln. Wenn sämtliche Behälter 2 eines Trägers in der vorstehend beschriebenen Weise verarbeitet worden sind, wird der Träger aus dem Bereich der Bördelstation 180 (vgl. Fig. 9c) abtransportiert und in der Prozessanlage (nicht dargestellt) weiter gefördert. Hierzu können die Behälter in den Öffnungen oder Aufnahmen des Trägers erneut in deren Normalstellung zurückgezogen und die Träger erneut in die Stellung gemäß der Fig. 2b überführt werden, beispielsweise durch Einsetzen in einen Transport- und Verpackungsbehälter, weiter gefördert zu werden. Die Verarbeitung in der Bördelstation erfolgt somit chargenweise, ohne dass die Behälter jedenfalls vollständig aus dem Träger entnommen zu werden brauchen.

Bei der Kappe, die gemäß der Fig. 9b auf den oberen Rand der Fläschchen aufgesetzt ist, kann es sich auch um sog. pre-fit caps handeln, wie nachfolgend beschrieben.

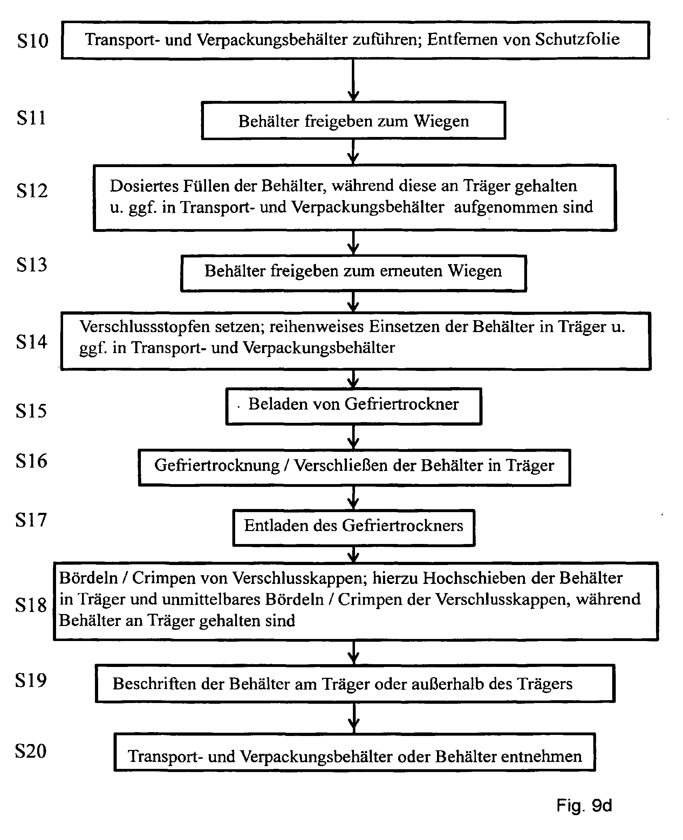

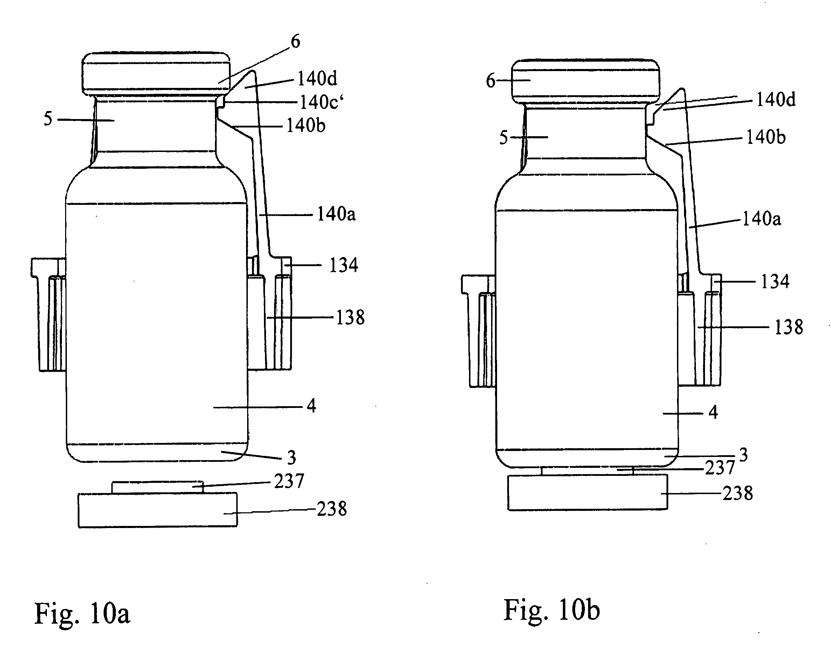

Die Fig. 9d zeigt als weiteres Beispiel ein schematisches Flussdiagramm eines Verfahrens gemäß einem weiteren Ausführungsbeispiel nach der vorliegenden Erfindung. Bei diesem Verfahren werden die Behälter vor dem Befüllen (Prozessschritt S11) und nach dem Befüllen (Prozessschritt S13) gewogen, sodass man eine genaue Information über die eingefüllte Menge erhält, die den einzelnen Behältern beispielsweise mittels einer Software oder durch Beschriftung oder Markierung rückverfolgbar zuordnen kann. Zum Wiegen der Behälter kann eine Wiegezelle verwendet werden, wie diese beispielhaft in den Figuren 10a und 10b dargestellt ist.

Die Wiegezelle umfasst eine Abstützfläche 238 und einen darauf vorgesehenen Gewichtssensor 237. Die Wiegezelle wird in den Prozessschritten S 11 und S 13 unter den Behältern positioniert, sodass der Gewichtssensor 237 das Gewicht des jeweils zugeordneten Behälters messen kann. Grundsätzlich kann diese Abstützfläche 238 identisch zu der Ablagefläche 246 am oberen Ende der Vertikal-Verstelleinrichtung sein (vgl. Fig. 7a) und der Gewichtssensor 237 ebenfalls in die Ablagefläche 246 der Vertikal-Verstelleinrichtung integriert sein, d.h. die Wiegezelle kann in die Vertikal-Verstelleinrichtung integriert sein. Alternativ kann die Wiegezelle auch als separate Messeinheit zu einem geeigneten Zeitpunkt unter die Behälter gefahren werden, um diese zu vermessen.

Wie in der Fig. 10b gezeigt, kann das Wiegen der Behälter grundsätzlich erfolgen, während die Behälter in den Öffnungen oder Aufnahmen der Haltestruktur aufgenommen sind, nämlich dann, wenn diese mit radialem und axialem Spiel oder mit radialem Spiel und axial frei verschiebbar von den Haltearmen 140a gehalten werden, wie vorstehend anhand der Figuren 2a bis 2o beschrieben, und die Behälter somit nur lose auf den Haltearmen 140 aufliegen, jedoch nicht in irgendeiner Weise von den Haltearmen geklemmt werden, was die Ergebnisse der Gewichtsmessung verfälschen würde. Natürlich kann das Wiegen in den Prozessschritten S11 und S 13 auch erfolgen, wenn die Behälter vollständig aus den Öffnungen oder Aufnahmen der Haltestruktur herausgenommen sind.

Den Figuren 2c und 2j kann man entnehmen, dass bei den meisten Ausführungsformen einer Haltestruktur im Sinne der vorliegenden Erfindung die Böden der Behälter von der Unterseite der Haltestruktur ungehindert und vollflächig zugänglich sind. Dies ermöglicht einen unmittelbaren Kontakt der Böden der Behälter mit einer Kühlfläche oder einem Kühlfinger eines Gefriertrockners, um in dem Prozessschritt S16 an den Behältern eine Gefriertrocknung vorzunehmen. Hierzu ist, anders als nach dem Stand der Technik, keine aufwändige Entnahme der Behälter aus der Haltestruktur und Vereinzelung der Behälter erforderlich. Vielmehr kann die Gefriertrocknung ausgeführt werden, während die Behälter an der Haltestruktur gehalten sind.

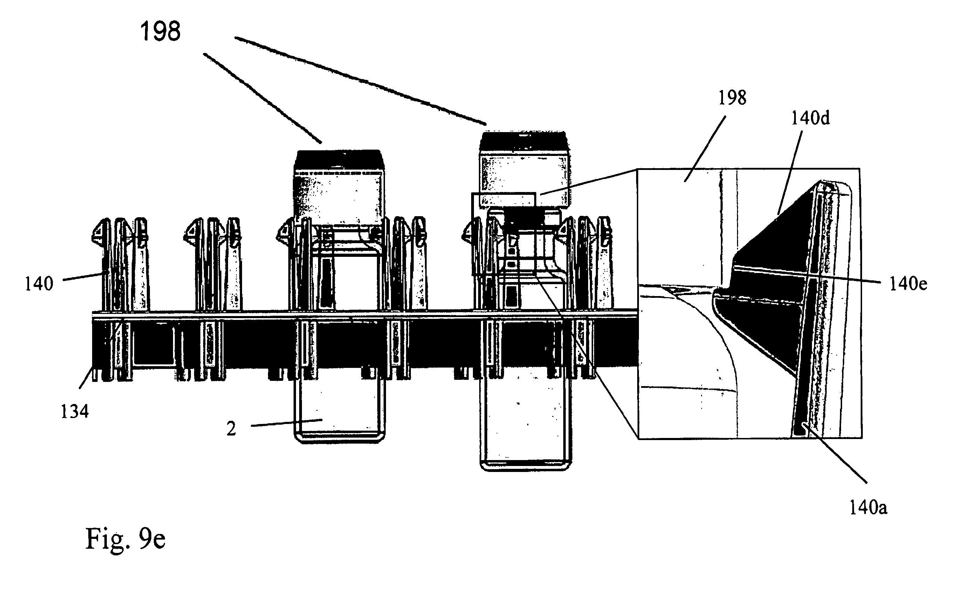

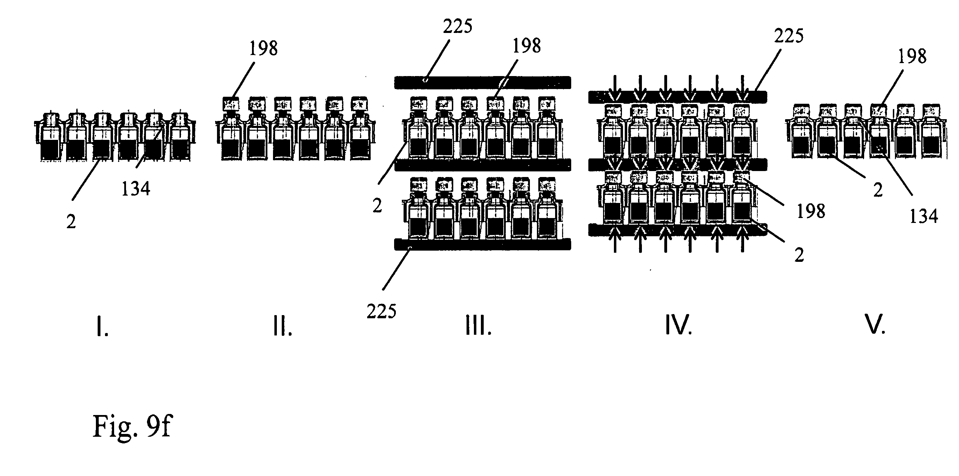

Die Figuren 9e und 9f zeigen ein weiteres Beispiel für die Verwendung eines solchen Verfahrens gemäß der vorliegenden Erfindung zum Verschließen von Fläschchen mit Hilfe von steril verschließenden Kappen, die unmittelbar auf den oberen Rand der Fläschchen aufgedrückt werden. Während die Fläschchen 2 in einer angehobenen Position der Kappe 198, also wie für das rechte Fläschchen 2 in der Fig. 9e dargestellt, über die bereits auf den oberen Rand des Fläschchens 2 aufgesetzte Kappe 198 befüllt werden kann oder zumindest weiter bearbeitet oder behandelt werden können, beispielsweise gefriergetrocknet werden können, weil das Innenvolumen der Fläschchen 2 mit der Umgebung kommuniziert, sind die Fläschchen 2 nach Herunterdrücken der Kappen 198, also wie für das linke Fläschchen 2 in der Fig. 9e dargestellt, steril verschlossen, ohne dass hierzu ein weiteres Bördeln oder Crimpen eines Metalldeckels erforderlich wäre. Zu diesem Zweck eignen sich insbesondere sog. pre-fit caps. Die vergrößerte Teildarstellung im rechten Bildteil der Fig. 9e zeigt die Halterung des Fläschchens 2 an den elastischen Haltezungen 140a des Trägers. Wie hier dargestellt, ist der untere Rand der Kappe 198 in der angehobenen Position am unteren Rand der Halteaufnahme 140e mit radialem Spiel abgestützt, liegt also locker auf der elastischen Haltezunge 140a auf. In dieser Position kann das Gewicht des Fläschchens 2 gemessen werden, beispielsweise mittels einer Wiegezelle, wie anhand der Figuren 10a und 10b beschrieben.

Wie man der Darstellung für die Halterung des linken Fläschchens 2 in der Fig. 9e entnehmen kann, ist der untere Rand der Kappe 198 in der herabgedrückten Position der Kappe 198 auch weiterhin am unteren Rand der Halteaufnahme 140e mit radialem Spiel abgestützt. Auch in dieser Position kann das Gewicht des Fläschchens 2 gemessen werden, beispielsweise mittels einer Wiegezelle, wie anhand der Figuren 10a und 10b beschrieben.

Zum sterilen Verschließen der Fläschchen 2 ist es somit wichtig, dass Kappe 198 und Fläschchen 2 relativ zueinander axial verstellt werden können. Diese axiale Verstellung kann erfindungsgemäß auch erfolgen, während die Fläschchen 2 an einem Träger (Englisch "nest") gehalten oder zumindest in dessen Öffnungen oder Aufnahmen geführt aufgenommen sind. Zusätzlich kann erfindungsgemäß auch ein Wiegen der Fläschchen 2 erfolgen, während diese an dem Träger gehalten sind.

Die Fig. 9f fasst fünf aufeinanderfolgende Prozessschritte (I. bis V.) zum Verschließen der Fläschchen gemäß einer weiteren Variante des Verfahrens nach der Fig. 9d zusammen. Zunächst werden die Fläschchen 2 in dem Prozessschritt I. befüllt, während diese an dem Träger 134 gehalten sind. Nach dem Befüllen werden die Kappen 198 in dem Prozessschritt II. auf den oberen Rand der Fläschchen 2 aufgedrückt. In dieser angehobenen Position der Kappen 198 kann eine Gefriertrocknung des Inhalts der Fläschchen 2 erfolgen, oder auch eine beliebige andere Behandlung oder Weiterverarbeitung der Fläschchen 2. Beispielhaft ist dies in dem Prozessschritt III. durch die Bezugszeichen 225 angedeutet, die Kühlböden eines nicht näher dargestellten Gefriertrockenschranks symbolisieren sollen. In dem Prozessschritt IV. werden die Kühlböden 225, oder andere Ablageflächen, auf denen die Böden der Fläschchen 2 unmittelbar aufliegen, gegeneinander gedrückt, wie durch die Pfeile angedeutet. Dadurch werden die Kappen 198 auf die Fläschchen 2 herabgedrückt und diese somit steril verschlossen. Die vorgenannten Prozessschritte können ausgeführt werden, während die Fläschchen 2 an einem Träger (Englisch "nest") gehalten oder zumindest in dessen Öffnungen oder Aufnahmen geführt aufgenommen sind. Anschließend folgt im Prozessschritt V. die Entnahme der Träger 134 mit den daran gehaltenen Fläschchen 2 aus der Prozessstation.

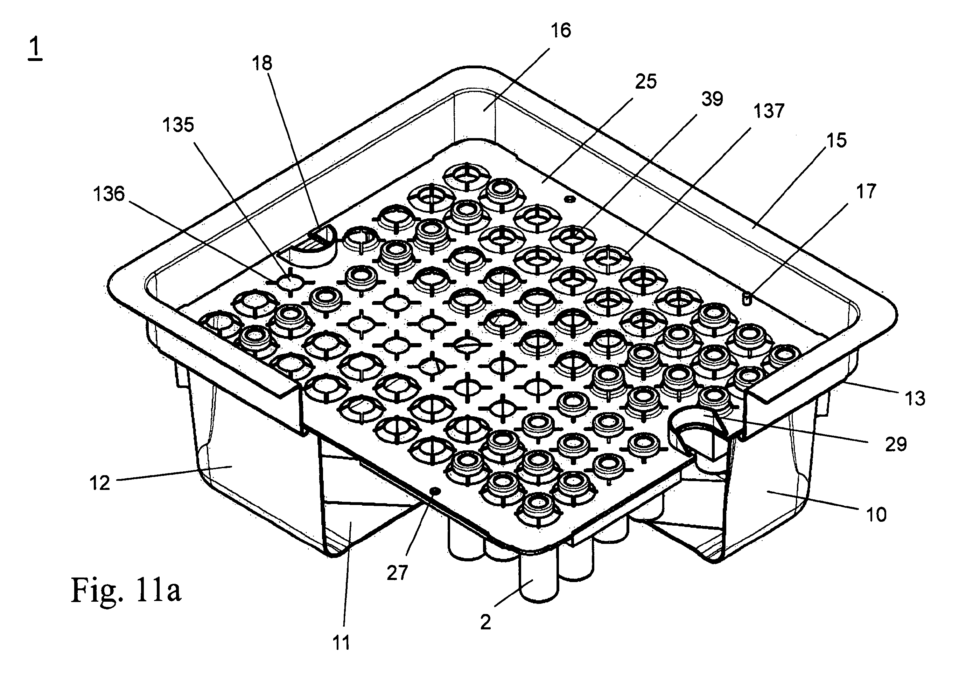

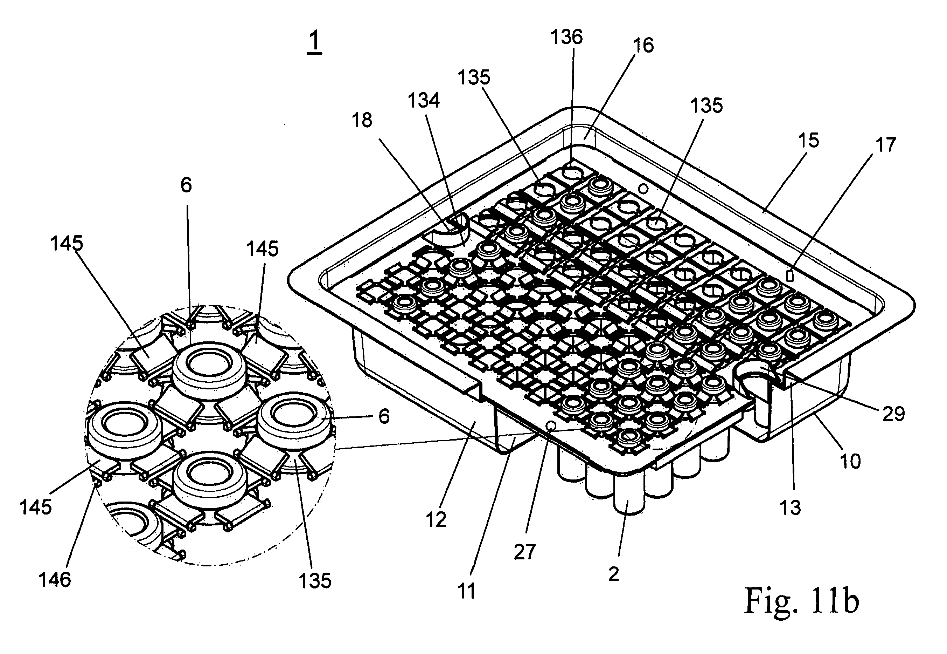

Die Figuren 11a und 11b zeigen zwei weitere Beispiele für Haltestrukturen, die bei einem Verfahren nach der vorliegenden Erfindung grundsätzlich eingesetzt werden können.

Gemäß der Fig. 11a wird zum gleichzeitigen Halten einer Mehrzahl von Behältern eine flächige Transportplatte 25 verwendet, die aus einem Kunststoff ausgebildet ist, beispielsweise ausgestanzt oder spritzgegossen ist, und die eine Mehrzahl von Öffnungen 39 zur Aufnahme der Fläschchen 2 aufweist. Die Öffnungen 39 sind in ringförmigen Formschlusselementen 137 ausgebildet, die als Klappen wirken, die entweder in die Öffnungen 39 eingesteckt sind, insbesondere in deren Umfangsrand eingerastet oder eingeclipst sind, oder die einstückig mit dem flächigen Träger 25 ausgebildet sind, beispielsweise durch 1K- oder 2K-Kunststoff-Spritzgussverfahren. Die Behälter 2 können von oben oder von unten her in die Öffnungen 39 der Formschlusselemente 137 eingeführt werden. Dadurch können eine Mehrzahl von Behälter 2 im Bereich ihrer verengten Halsabschnitte 5 formschlüssig fixiert werden.

Die Fig. 11b zeigt eine weitere Ausführungsform einer Haltestruktur, bei der die Fixierung der Behälter 2 mit Hilfe von schwenkbaren Haltezungen oder Klappen 145 realisiert ist, die schwenkbeweglich an dem flächigen Träger 134 gelagert sind. Die Haltezungen 145 weisen an ihrem unteren Ende jeweils einen seitlich abstehenden Zapfen 146 auf, der in eine korrespondierend ausgebildete Aufnahme des Trägers 134 eingeclipst oder eingedrückt ist. Die Haltezungen oder Klappen 145 können somit zwischen einer ersten Stellung, in welcher die Öffnungen 135 des Trägers freigegeben sind und die Behälter ungehindert eingeführt werden können, und einer zweiten Stellung geschwenkt werden, in welcher die Behälter formschlüssig fixiert sind. Die zum Verschwenken der Klappen bzw. Haltezungen 145 erforderliche Kraft kann insbesondere durch die Lagerung der Zapfen 146 in den hierzu vorgesehenen Aufnahmen geeignet vorgegeben werden. Es kann auch vorgesehen sein, dass die Haltezungen 145 elastisch in die zweite Stellung vorgespannt sind. Die Behälter 2 können in eine solche Haltestruktur in einfacher Weise von unterhalb des Trägers 134 in die Öffnungen 135 eingeführt werden, bis der obere Rand 6 der Behälter 2 jeweils auf den einer jeweiligen Öffnung 135 zugeordneten Haltezungen 145 aufliegt. Grundsätzlich können die Behälter 2 jedoch auch von oberhalb des Trägers 134 her in die Öffnungen 135 eingeführt werden, ohne dass Verunreinigungen in die Behälter über deren Befüllöffnung eindringen können. Zu diesem Zweck kann beispielsweise die Vertikal-Verstelleinrichtung so ausgelegt sein, dass die Haltezungen 145 gemeinsam, beispielsweise in einer angehobenen Position der Vertikal-Verstelleinrichtung, soweit aufgespreizt werden, dass die Behälter 2 von oben her in die Öffnungen 135 eingeführt werden, beispielsweise um dann jeweils auf einer Auflagefläche am oberen der Vertikal-Verstelleinrichtung abgestützt zu werden. Beim Absenken der Vertikal-Verstelleinrichtung kann dann die Aufspreizung der Haltezungen 145 wieder freigegeben werden.

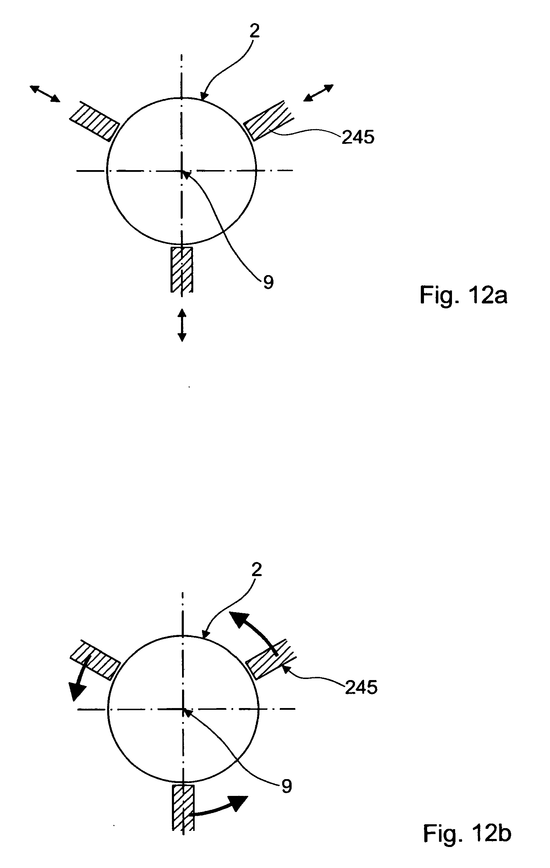

Nachfolgend werden anhand der Figuren 12a bis 13c weitere Ausführungsformen einer Vertikal-Verstelleinrichtung und des Zusammenwirkens einer solchen Vertikal-Verstelleinrichtung mit Haltemitteln an einer Haltestruktur beschrieben werden, wie diese bei einem Verfahren gemäß der vorliegenden Erfindung eingesetzt werden können.

Die Fig. 12a zeigt einen Behälter 2 in Draufsicht, dessen unterer Rand der von den Haltearmen 245 einer nicht näher dargestellten Vertikal-Verstelleinrichtung umgriffen ist, wie vorstehend anhand der Fig. 6a beschrieben, um den Behälter 2 zu greifen und auf der Vertikal-Verstelleinrichtung zu fixieren. Die Haltearme 245 können radial auswärts verstellt werden, wie durch die Doppelpfeile angedeutet, um die Behälter freizugeben. Diese Verstellung kann aktiv erfolgen, also beispielsweise elektrisch, magnetisch, pneumatisch oder mechanisch angetrieben sein, zu welchem Zweck am oberen Ende der Vertikal-Verstelleinrichtung ein geeigneter Antrieb vorgesehen sein kann. Oder die Haltearme sind elastisch radial einwärts oder auswärts vorgespannt und werden bei Erreichen einer vorbestimmten Höhe der Vertikal-Verstelleinrichtung freigegeben oder zusammen- oder auseinandergedrückt. Zu diesem Zweck kann ein Steuernocken oder dergleichen mit der Vertikal-Verstelleinrichtung zusammenwirken. Um die Behälter zuverlässig zu greifen, ist dabei auf eine gemeinsame und koordinierte Verstellung der Haltearme 245 zu achten, wobei die Verstellbewegung auf den Mittelpunkt 9 des Behälterbodens zentriert sein muss. Nach Aufspreizen der Haltearme 245 ist der Behälter 2 von der Vertikal-Verstelleinrichtung freigegeben und kann dann an ein Haltemittel oder eine Prozessstation übergeben werden.

Die Fig. 12b zeigt eine weitere Ausführungsform, bei der diese Verstellbewegung der Haltearme 245 nicht radial erfolgt, sondern sowohl radial einwärts oder auswärts und als auch mit einer Bewegungskomponente in Umfangsrichtung des Behälters, wie durch die drei gekrümmten Pfeile angedeutet.

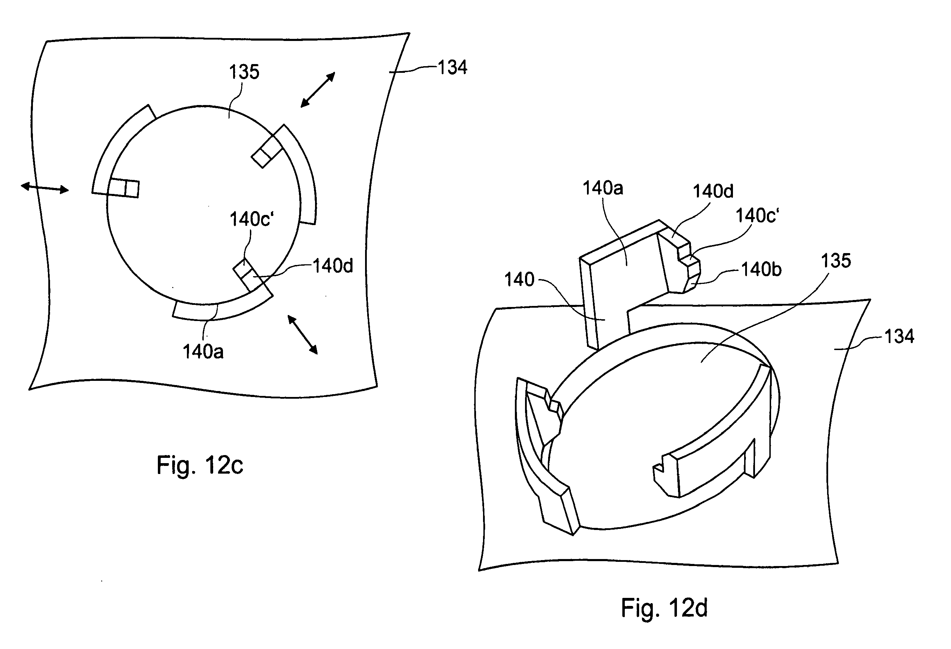

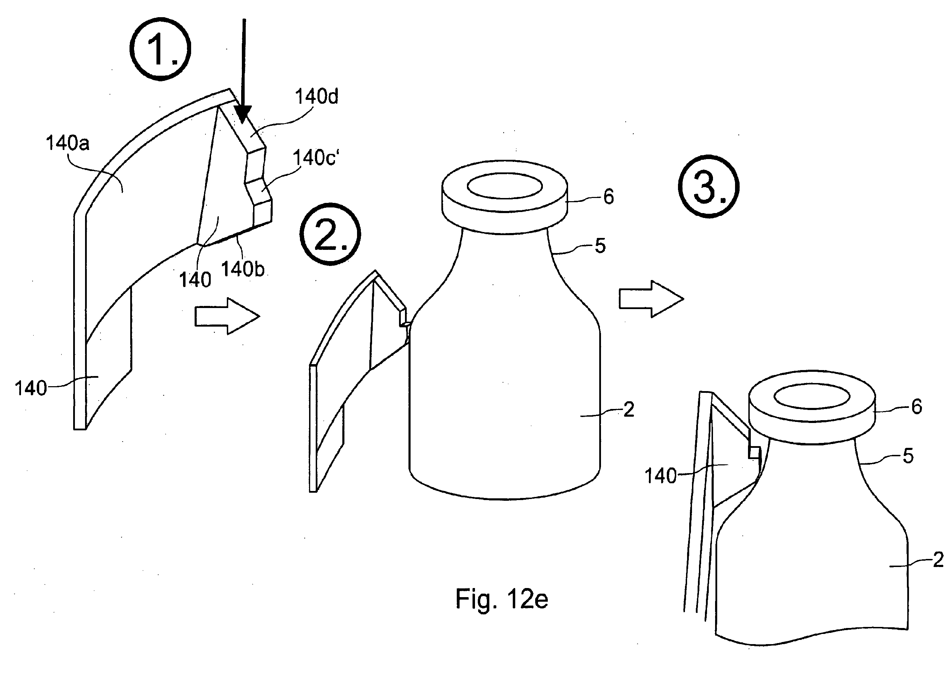

Die Figuren 12c und 12d zeigen eine weitere Variante einer Haltestruktur 134, bei der eine vergleichbare Aufspreizung der elastischen Haltearme 140 zum Freigeben und Halten der Behälter an der Haltestruktur 134 statt an der Vertikal-Verstelleinrichtung realisiert ist. Gemäß der Draufsicht nach der Fig. 12c sind um die Öffnung 135 in dem flächigen Träger 134 herum drei elastische Haltearme 140a angeordnet, vergleichbar zu dem vorstehend anhand der Fig. 2l beschriebenen Haltearm. Gemäß der Fig. 12d ist die Basis des Haltearms 140 vergleichsweise schmal ausgebildet, während der der eigentlichen Abstützung der Behälter dienende Teil an einem rechteckförmigen, vergleichsweise großen Abschnitt 140a ausgebildet ist, einschließlich der oberen und unteren Einführschrägen 140d, 140b und der stufenförmigen Haltenase 140c', auf welcher der obere Rand des Behälters (nicht dargestellt) lose oder jedenfalls mit radialem Spiel aufliegt. Beim Einführen des Behälters in die Öffnung 135 gelangt die obere Einführschräge 140d und die Haltenase 140c' in Anlage mit der Seitenwand des Behälters, wobei der Haltearm hierbei nicht nach hinten, also radial auswärts, aufgebogen wird sondern in einer Drehbewegung aufschwenkt, um den Behälter passieren zu lassen. Ist der Behälter schließlich soweit in die Öffnung eingeführt, dass der Nackenabschnitt in den Bereich der Haltenase 140c' gelangt, schwenkt der Haltearm 140 in die Ausgangsposition zurück und hält den Behälter am Hals, wie vorstehend beispielsweise anhand der Fig. 2l beschrieben. Dies ist in der Sequenz der Schritte 1. bis 3. der Fig. 12e dargestellt, nämlich zunächst die Ausgangsstellung (Schritt 1.), dann das Einführen des Behälters 2 von oben her (Schritt 2.) und dann die Abstützung des Behälters 2 (Schritt 3.) am Haltearm 140.

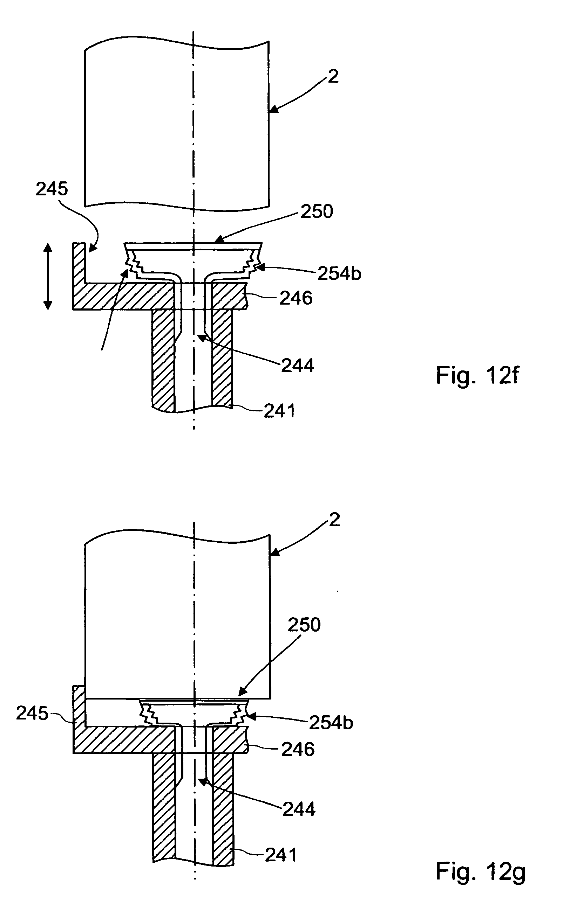

Die Figuren 12f und 12g zeigen eine weitere Ausführungsform einer Vertikal-Verstelleinrichtung, wenn der der Behälter 2 freigegeben ist (Fig. 12f) und wenn der Behälter 2 durch Anlegen eines Unterdrucks an die Saugglocke 250 nach unten gegen die Haltearme 245 gezogen wird. Im Unterschied zur Ausführungsform nach der Fig. 7a ist die Saugglocke 250 aus einem elastischen Kunststoff oder Gummi unmittelbar in die axiale Durchgangsbohrung 244 des Stößelschafts 241 eingesteckt. Der obere Rand der Saugglocke 250 bildet gemeinsam mit Randabschnitten der Haltearme 245 unmittelbar eine Auflagefläche zur Abstützung des Bodens des Behälters 2. Steigt der Innenrand der Haltearme geneigt nach oben an, so können Toleranzen des Durchmessers der Behälter 2 in einfacher Weise kompensiert werden. Die Saugglocke 250 ist einerseits ausreichend elastisch, um für eine ausreichende Abdichtung beim Anlegen des Unterdrucks zu sorgen, andererseits ausreichend steif, um eine zuverlässige und gleichmäßige Abstützung des Behälters 2 auf dem Stößelschaft 241 zu ermöglichen.

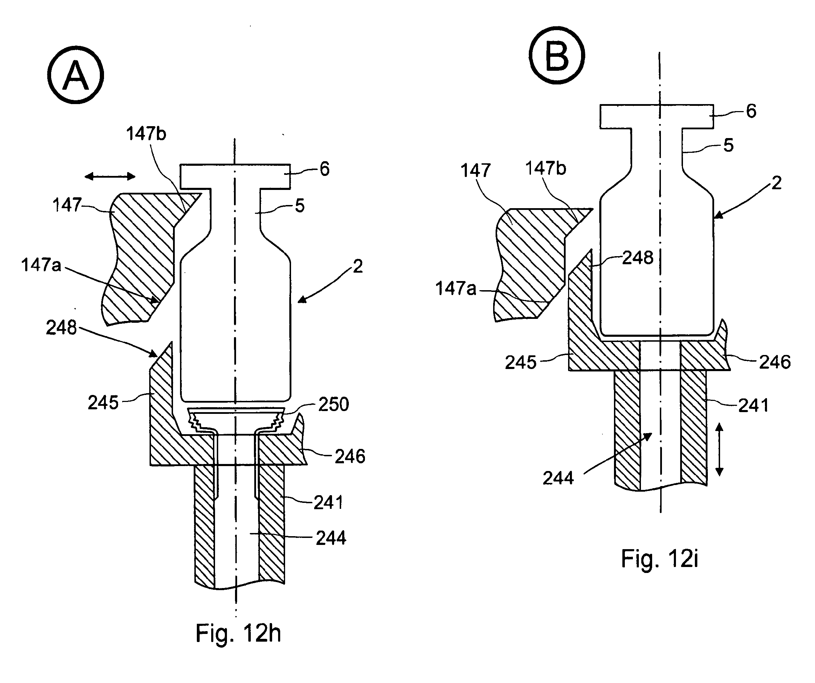

Die Figuren 12h und 12i zeigen eine weitere Ausführungsform, bei der die Vertikal-Verstelleinwirkung mit den Haltemitteln der Haltestruktur zusammenwirkt, um diese zur Freigabe der Behälter zu lösen und wieder in Eingriff mit den Behältern zu bringen. Das Haltemittel 147 der hier nicht dargestellten Haltestruktur ist zu diesem Zweck radial einwärts und auswärts verstellbar, beispielsweise radial verschwenkbar, wie durch den Doppelpfeil in der Fig. 12h dargestellt. Am unteren Rand des Haltemittels ist eine Schräge 147a ausgebildet, die mit einer gegenüberliegenden Schräge 248 am oberen Rand der Haltearme 245 (oder ausgebildet in einem anderen Bereich der Vertikal-Verstelleinrichtung) zusammenwirkt. Zum Anheben der Fläschchen 2 aus der Stellung gemäß der Fig. 12h in eine angehobene Position wird zunächst der Stößel 241 nach oben angehoben und schiebt dabei das Fläschchen 2 mit nach oben, bis die Schräge 248 an den Haltearmen 245 in Anlage mit der Schräge 147a am unteren Rand der Haltemittel 147 gelangt. In dieser Stellung gelangt der Übergangsbereich zwischen der zylindrischen Seitenwand des Fläschchens 2 und dem verengten Nackenbereich 5 in die Nähe der vorstehenden Nase 147b des Haltemittels 147. Beim weiteren Anheben des Stößels gleiten die Schrägen 147a und 248 aneinander ab, was zu einem Zurückstellen des Haltemittels 147 auswärts und somit zu einer vollständigen Freigabe des Fläschchens 2 führt (vgl. Fig. 12i), welches dann weiter aus der Haltestruktur nach oben herausgehoben werden kann. Die Haltearme 245 der Vertikal-Verstelleinrichtung wirken dabei als Nocken zum Steuern der Stellung der Haltemittel 147 der Haltestruktur. Selbstverständlich kann eine entsprechende koordinierte Verstellung der Haltemittel der Haltestruktur auch mittels einer gesondert im Bereich der Prozessstation oder der Vertikal-Verstelleinrichtung vorgesehenen Verstelleinrichtung realisiert werden.

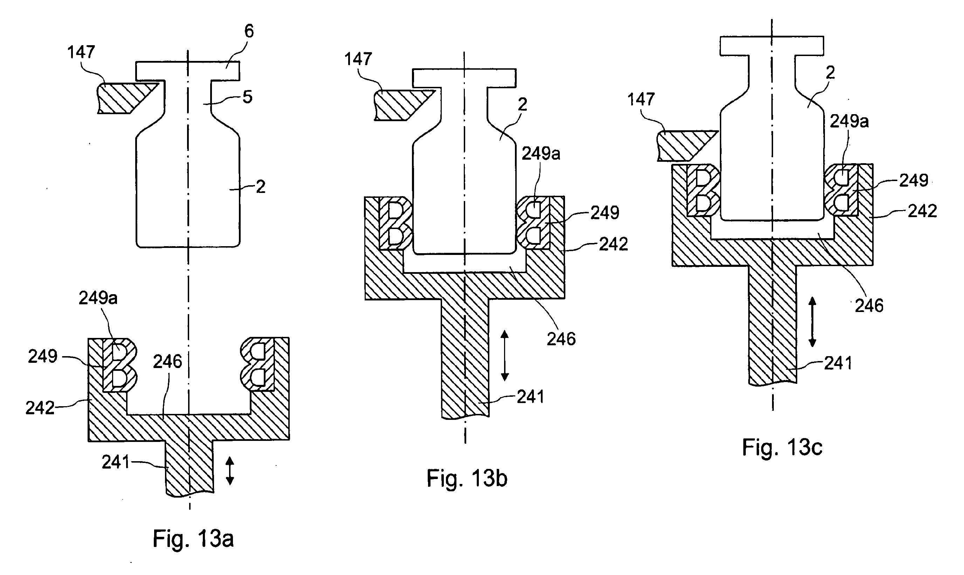

Anhand der Figuren 13a bis 13c wird nachfolgend eine weitere Ausführungsform einer Vertikal-Verstelleinrichtung beschrieben werden, bei der Fläschchen 2 zum Ziehen nach unten an deren unterem Rand geklemmt werden. Zu diesem Zweck ist gemäß der Fig. 13a auf der Innenseite des Halterings 242 des Stößelschafts 241 eine durch ein Fluid aufblasbare Druckmanschette 249 mit zumindest einer Druckkammer 249a vorgesehen, die in ihrer entspannten Ruhestellung ein ungehindertes oder jedenfalls reibungsarmes Einführen des Fläschchens in die Aufnahme in dem Haltering 242 bis zum Erreichen der Auflagefläche 246 ermöglicht. Um ein Fläschchen 2 zu greifen, werden die Druckkammern 249a der Druckmanschette mit einem Druck beaufschlagt, sodass diese sich definiert radial einwärts vergrößern, bis schließlich der untere Rand des Fläschchens 2 geklemmt ist, wie in der Fig. 13b dargestellt. In dieser Stellung ist das Fläschchen 2 auf der Vertikal-Verstelleinrichtung fixiert, ein seitliches Verrutschen der Fläschchen 2 ist verhindert. In dieser Stellung kann das Fläschchen 2 nach oben angehoben werden (vgl. Fig. 13c) oder nach unten gezogen werden.

Wie dem Fachmann beim Studium der vorstehenden Beschreibung ohne weiteres ersichtlich sein wird, können die vorstehend beispielhaft beschriebenen Haltestrukturen und Vorgehensweisen zum Verstellen der Vertikal-Verstelleinrichtung und der Haltemittel auch in anderer Weise miteinander kombiniert werden, als vorstehend beschrieben.

Wie dem Fachmann beim Studium der vorstehenden Beschreibung ohne weiteres ersichtlich sein wird, ist der vorgenannte Gesichtspunkt der form- oder kraftschlüssigen Verbindung von unmittelbar benachbarten Haltestrukturen grundsätzlich unabhängig von der konkreten Ausführung der Halterung der Fläschchen an solchen Haltestrukturen, sodass dieser Gesichtspunkt grundsätzlich auch als unabhängiger Gesichtspunkt der vorliegenden Erfindung unabhängig von der konkreten Realisierung der Halterung der Fläschchen an solchen Haltestrukturen beansprucht werden kann.

Die von den Haltemitteln jeweils auf die Behälter ausgeübte Haltekraft ist ausreichend, um die Behälter zuverlässig an der Haltestruktur zu halten. Insbesondere ist die ausgeübte Haltekraft größer als die Gewichtskraft der Behälter, ggf. mit Inhalt und Verschlussstopfen. Dadurch wird eine zuverlässige Halterung der Behälter an der Haltestruktur gewährleistet. Gleichzeitig können die Behälter in den Öffnungen oder Aufnahmen der Haltestruktur ohne größeren Kraftaufwand verstellt werden, insbesondere axial vorgeschoben oder gedreht werden.

Selbstverständlich kann die Haltestruktur (der Träger) im Sinne der vorliegenden Erfindung auch aus einem thermoplastischen, duroplastischen oder elastomeren Kunststoff ausgebildet sein, wobei zumindest Abschnitte der Haltestruktur bzw. des Trägers mit einer reibreduzierenden Beschichtung versehen sind, um das Einführen und die Entnahme der Behälter zu erleichtern.

Wie dem Fachmann beim Studium der vorstehenden Beschreibung ohne Weiteres ersichtlich sein wird, können die einzelnen Gesichtspunkte und Merkmale der vorstehend beschriebenen Ausführungsbeispiele auch in beliebiger Weise miteinander kombiniert werden, was in zahlreichen weiteren Ausführungsformen und Modifikationen resultiert. Wie dem Fachmann beim Studium der vorliegenden Beschreibung ohne weiteres ersichtlich sein wird, sollen sämtliche solche weiteren Ausführungsformen und Modifikationen von der vorliegenden Erfindung mit umfasst sein, solange diese nicht von dem allgemeinen Lösungsgedanken und dem Schutzbereich der vorliegenden Erfindung abweichen, wie in den beigefügten Patentansprüchen festgelegt.

## Patentansprüche

1. Verfahren zur Behandlung oder Verarbeitung von Behältern (2), die zur Aufbewahrung von Substanzen für medizinische, pharmazeutische oder kosmetische Anwendungen dienen oder diese enthalten, wobei die Behälter mittels einer Fördereinrichtung zur Behandlung oder Verarbeitung automatisiert an zumindest einer Prozessstation vorbeigeführt werden oder diese durchlaufen, bei welchem Verfahren
eine Mehrzahl von Behältern (2) von der Fördereinrichtung gefördert werden, während diese an einem Träger (25; 134) gemeinsam in einer regelmäßigen Anordnung gehalten werden,
die an dem Träger gehaltenen Behälter zur Behandlung oder Verarbeitung an oder in der jeweiligen Prozessstation in eine angehobene Position angehoben werden, und
nach der Behandlung oder Verarbeitung abgesenkt werden, um an dem Träger erneut in der regelmäßigen Anordnung gehalten zu werden, wobei
die Behälter (2) mittels einer Vertikal-Verstelleinrichtung (240) vertikal aufwärts in die angehobene Position angehoben werden, **gekennzeichnet dadurch, dass**
die Behälter nach der Behandlung oder Verarbeitung mittels eines Unterdrucks an den Träger zurückgezogen werden, wobei
der Unterdruck über eine Auflagefläche, die an einem oberen Ende der Vertikal-Verstelleinrichtung vorgesehen ist und auf welcher der jeweilige Bodenbereich (3) der Behälter (2) beim Anheben der Behälter in die angehobene Position aufliegt, und eine in der Auflagefläche vorgesehene Saugkappe, die an dem Bodenbereich (3) der Behälter (2) anliegt, an den Bodenbereich (3) der Behälter (2) angelegt wird.

2. Verfahren nach Anspruch 1, wobei die Vertikal-Verstelleinrichtung eine seitliche Ausweichbewegung der Behälter zumindest beim Anheben der Behälter in die angehobene Position verhindert.

3. Verfahren nach Anspruch 2, wobei die Vertikal-Verstelleinrichtung auf einen Bodenbereich (3) der Behälter (2) einwirkt und dabei einen unteren Rand der Behälter (2) zumindest abschnittsweise umgreift, wobei der untere Rand der jeweiligen Behälter (2) bevorzugt mittels einer Mehrzahl von Haltearmen (245) umgriffen wird, wobei Öffnungen oder Aufnahmen in dem Träger, in welchem die Behälter in deren Ausgangsposition an dem Träger gehalten werden, von der Vertikal-Verstelleinrichtung beim Anheben der Behälter in die angehobene Position durchgriffen werden.

4. Verfahren nach einem der vorhergehenden Ansprüche, wobei die Vertikal-Verstelleinrichtung eine Mehrzahl von Hubstangen umfasst, die entlang einer Linie fluchtend angeordnet sind und eine Reihe von Behältern gleichzeitig in die angehobene Position anheben,
bevorzugt weiterhin umfassend eine Relativpositionierung des Trägers (25; 134) mit den daran gehaltenen Behältern (2) und der Vertikal-Verstelleinrichtung.

5. Verfahren nach einem der vorhergehenden Ansprüche, wobei die Behälter in der angehobenen Position weiterhin in Öffnungen oder Aufnahmen, die in dem Träger vorgesehen sind und die regelmäßige Anordnung vorgeben, gehalten oder zumindest geführt werden, um an oder in der Prozessstation behandelt oder verarbeitet zu werden.

6. Verfahren nach Anspruch 5, wobei die Behälter in der angehobenen Position weiterhin in den Öffnungen oder Aufnahmen des Trägers aufgenommen sind, jedoch auf einer zusätzlichen Abstützfläche abgestützt werden oder mittels einer zusätzlichen Halte- oder Greifeinrichtung gehalten werden, um an oder in der Prozessstation behandelt oder verarbeitet zu werden, wobei
die zusätzliche Abstützfläche bevorzugt zumindest einen drehbeweglich gelagerten und angetriebenen Drehteller aufweist, auf welchem die Behälter während der Behandlung oder Verarbeitung an oder in der Prozessstation gedreht werden, während diese weiterhin in den Öffnungen oder Aufnahmen des Trägers aufgenommen sind.

7. Verfahren nach einem der Ansprüche 1 bis 4, wobei die Behälter vollständig aus Öffnungen oder Aufnahmen, die in dem Träger vorgesehen sind und die regelmäßige Anordnung vorgeben, herausgehoben werden, um an oder in der Prozessstation behandelt oder verarbeitet zu werden.

8. Verfahren nach einem der vorhergehenden Ansprüche, wobei zumindest einige der Prozessstationen unter sterilen Umgebungsbedingungen angeordnet und betrieben werden.

9. Verfahren nach einem der vorhergehenden Ansprüche, wobei
die an dem Träger gehaltenen Behälter zur Behandlung oder Verarbeitung an oder in der jeweiligen Prozessstation in eine angehobene Position angehoben werden und
mittels einer Wiegeeinrichtung (237) zwischen zwei Verfahrensschritten gewogen werden, wobei
die Wiegeeinrichtung (237) bevorzugt in eine zum Anheben und Absenken der Behälter (2) verwendete Vertikal-Verstelleinrichtung integriert ist.

10. Verfahren nach einem der vorhergehenden Ansprüche, weiterhin umfassend einen Schritt zu Befüllen der Behälter (2) mit der Substanz, während diese an dem Träger gehalten, bei welchem Schritt
Kappen (198) auf die oberen Ränder der Behälter aufgedrückt werden, während diese an dem Träger gehalten,
die Kappen (198) und die Behälter (2) relativ zueinander axial verstellt werden, um die Kappen auf die Behälter (2) herabzudrücken, während Böden der Behälter (2) auf einer Ablagefläche aufliegen, um die Behälter mit den Kappen (198) zu verschließen, wobei
die Innenvolumen der Behälter (2) über die bereits auf die oberen Ränder der Behälter (2) aufgesetzte Kappen mit der Umgebung kommunizieren, die Behälter über die bereits auf die oberen Ränder der Behälter (2) aufgesetzte Kappen mit der Substanz befüllt werden und die Behälter (2) nach Herunterdrücken der Kappen (198) verschlossen sind, oder
das Befüllen der Behälter (2) mit der Substanz vor einem Aufsetzen der Kappen (198) auf die oberen Ränder der Behälter erfolgt und die Kappen (198) nach dem Befüllen der Behälter (2) auf die oberen Ränder der Behälter derart aufgedrückt werden.

11. Verfahren nach Anspruch 10, wobei
die Behälter (2) nach dem Befüllen einer Gefriertrocknung unterzogen werden, während diese an dem Träger gehalten werden, indem die Böden der Behälter (2) mit einer Kühlfläche oder einem Kühlfinger eines Gefriertrockners in einen unmittelbaren Kontakt gebracht werden, wobei die Innenvolumen der Behälter (2) während der Gefriertrocknung über die bereits auf die oberen Ränder der Behälter (2) aufgesetzte Kappen mit der Umgebung kommunizieren, wobei
mehrere Kühlflächen oder Kühlfinger des Gefriertrockners oder Ablageflächen mit den darauf unmittelbar aufliegenden Behältern gestapelt übereinander angeordnet sind und die Kühlflächen oder Kühlfinger des Gefriertrockners oder Ablageflächen gegeneinander gedrückt werden, um die Kappen auf die Behälter (2) herabzudrücken und die Behälter mit den Kappen (198) zu verschließen

12. Vorrichtung zur Behandlung oder Verarbeitung von Behältern (2), die zur Aufbewahrung von Substanzen für medizinische, pharmazeutische oder kosmetische Anwendungen dienen oder diese enthalten, mit
zumindest einer Prozessstation, in oder an der die Behälter (2) behandelt oder verarbeitet werden;
einer Fördereinrichtung, um einen Träger (25; 134), an welchem die Behälter gehalten sind, zu fördern, sodass dieser zur Behandlung oder Verarbeitung an zumindest einer Prozessstation vorbeigeführt wird oder diese durchläuft; und
einer Vertikal-Verstelleinrichtung, die ausgelegt ist, um die an dem Träger gehaltenen Behälter zur Behandlung oder Verarbeitung an oder in der Prozessstation vertikal aufwärts in eine angehobene Position anzuheben und um die Behälter nach der Behandlung oder Verarbeitung an oder in der Prozessstation abzusenken, sodass diese an dem Träger erneut in der regelmäßigen Anordnung gehalten werden;
**dadurch gekennzeichnet, dass** an einem oberen Ende der Vertikal-Verstelleinrichtung eine Auflagefläche vorgesehen ist, auf welcher der jeweilige Bodenbereich (3) der Behälter (2) beim Anheben der Behälter in die angehobene Position aufliegt, wobei die Vertikal-Verstelleinrichtung weiterhin ein Unterdruck-Erzeugungsmittel aufweist, das so mit der Vertikal-Verstelleinrichtung gekoppelt ist, dass der Unterdruck über die Auflagefläche (246) und eine in der Auflagefläche vorgesehene Saugkappe (250), die ausgelegt ist, um an dem Bodenbereich (3) der Behälter (2) anzuliegen, auf den Bodenbereich (3) der Behälter (2) einwirkt.

13. Vorrichtung nach Anspruch 12, wobei die Vertikal-Verstelleinrichtung ausgelegt ist, um auf einen Bodenbereich (3) der Behälter (2) einzuwirken und dabei einen unteren Rand der Behälter (2) zumindest abschnittsweise zu umgreifen.

14. Vorrichtung nach Anspruch 13, wobei die Vertikal-Verstelleinrichtung eine Mehrzahl von Haltearmen (245) aufweist, um den unteren Rand der jeweiligen Behälter (2) zu umgreifen, wobei Öffnungen oder Aufnahmen in dem Träger, in welchem die Behälter in deren Ausgangsposition an dem Träger gehalten werden, von der Vertikal-Verstelleinrichtung beim Anheben der Behälter in die angehobene Position durchgriffen werden.

15. Vorrichtung nach einem der Ansprüche 12 bis 14, wobei die Vertikal-Verstelleinrichtung eine Mehrzahl von Hubstangen (240) umfasst, die entlang einer Linie fluchtend angeordnet sind und eine Reihe von Behältern gleichzeitig in die angehobene Position anheben.

16. Vorrichtung nach Anspruch 15, weiterhin umfassend ein Positionierungsmittel (260, 271), um die Hubstangen relativ zu dem Träger (25; 134) mit den daran gehaltenen Behältern (2) zu positionieren.

17. Verwendung einer Vertikal-Verstelleinrichtung zum Anheben und Absenken von Behältern (2), die zur Aufbewahrung von Substanzen für medizinische, pharmazeutische oder kosmetische Anwendungen dienen oder diese enthalten, in einem Verfahren nach einem der Ansprüche 1 bis 11 oder einer Vorrichtung nach einem der Ansprüche 12 bis 16, um
die Behälter (2) mittels der Vertikal-Verstelleinrichtung (240) vertikal aufwärts in die angehobene Position anzuheben und
nach der Behandlung oder Verarbeitung mittels des Unterdrucks an den Träger zurückzuziehen, wobei
der Unterdruck über die Auflagefläche und die in der Auflagefläche vorgesehene Saugkappe an den Bodenbereich (3) der Behälter (2) angelegt wird, wenn die Saugkappe an dem Bodenbereich (3) der Behälter (2) anliegt.

## Claims

1. A process for the treatment or processing of containers (2) that serve for storing, or contain, substances for medical, pharmaceutical or cosmetic applications, wherein the containers are conveyed automatically, by means of a conveyor, past at least one processing station or pass it for the treatment or processing, in which process
a plurality of containers (2) is conveyed by the conveyor while being held by a carrier (25; 134) in a regular arrangement,
the containers held on the carrier are raised to a raised position for the treatment or processing at or in the respective processing station, and
are lowered after the treatment or processing, to be held again on the carrier in the regular arrangement, wherein
the containers (2) are raised vertically upward to the raised position by means of a vertical displacement device (240),
**characterized in that** the containers are retracted to the carrier by means of a negative pressure after the treatment or processing, wherein
the negative pressure acts on the bottom portions (3) of the containers (2) via supporting surfaces provided at an upper end of the vertical displacement device, on which the bottom portions (3) of the containers (2) rest in the raised position when raising the containers, and via suction caps provided in the supporting surface, which abut against the bottom portions (3) of the containers (2).

2. The process according claim 1, wherein the vertical displacement device prevents a lateral displacement of the containers at least during raising of the containers to the raised position.

3. The process according to claim 2, wherein the vertical displacement device acts on bottom portions (3) of the containers (2) and embraces bottom edges of the containers (2) at least partially, wherein the bottom edge of the respective container (2) is embraced by a plurality of holding arms (245), wherein the vertical displacement device extends through apertures or receptacles in the carrier, in which the containers are held in their initial position on the carrier, during raising of the containers to the raised position.

4. The process according to any of the preceding claims, wherein the vertical-displacement device comprises a plurality of lifting rods that are aligned along a line and simultaneously raise a row of containers to the raised position,
preferably further comprising positioning the carrier (25; 134) together with the containers (2) held thereon relative to the vertical displacement device.

5. The process according to any of the preceding claims, wherein the containers continue to be held or at least guided in apertures or receptacles in the raised position, which are provided in the carrier and define the regular arrangement, to be treated or processed at or in the processing station.

6. The process according claim 5, wherein the containers continue to be held or at least guided in apertures or receptacles in the raised position, but are supported on an additional supporting surface or are held by means of an additional holding or gripping device, to be treated or processed at or in the processing station, wherein
the additional supporting surface preferably comprises at least one turntable, which is rotatably mounted and driven, on which the containers are rotated during the treatment or processing at or in the processing station while they are still accommodated in the apertures or receptacles of the carrier.

7. The process according to any of claims 1 to 4, wherein the containers are completely removed from the apertures or receptacles, which are provided in the carrier and define the regular arrangement, to be treated or processed at or in the processing station.

8. The process according to any of the preceding claims, wherein at least some of the processing stations are arranged and operated under sterile environmental conditions.

9. The process according to any of the preceding claims, wherein
the containers held on the carrier are raised to the raised position for the treatment or processing at or in the respective processing station and
weighed by a weighing device (237) between two process steps, wherein
the weighing device (237) is preferably integrated in the vertical displacement device used for raising and lowering the containers (2).

10. The process according to any of the preceding claims, further comprising a step of filing the containers (2) with the substance while being held at the carrier, in which step
caps (198) are pushed onto the upper rims of the containers while being held by the carrier, and
the caps (198) and the containers are displaced in axial direction relative to each other to press down the caps onto the containers (2) while the bottoms of the containers (2) are supported on a supporting surface, for sealing the containers with the caps (198), wherein
the internal volumes of the containers (2) communicate with the environment via the caps positioned on the upper rims of the containers (2), the containers are filled with the substance via the caps positioned on the upper rims of the containers (2) and the containers (2) are sealed after pushing down the caps (198), or
the filling of the containers (2) with the substances is performed disposing the caps (198) on the upper rims of the containers and the caps (198) are pushed onto the upper rims of the containers after filling the containers (2).

11. The process according to claim 10, wherein
the containers (2) are subjected to a freeze-drying process after the filling while being held by the carrier by bringing the bottoms of the containers (2) into direct contact with a cooling surface or a cooling finger of a freeze-dryer, wherein the internal volumes of the containers (2) communicate with the environment via the caps positioned on the upper rims of the containers (2) during the freeze-drying, wherein
a plurality of cooling surfaces or cooling fingers of the freeze-dryer or of supporting surfaces together with the containers directly supported thereon are stacked one above the other and the cooling surfaces or cooling fingers of the freeze-dryer or the supporting surfaces are pressed against each other for pushing down the caps onto the containers (2) and sealing the containers with the caps (198).

12. An apparatus for the treatment or processing of containers (2) that serve for storing, or contain, substances for medical, pharmaceutical or cosmetic applications, comprising
at least one processing station, in or at which the containers (2) are treated or processed;
a conveyor for conveying a carrier (25; 134) on which the containers are held so that it is conveyed past or passes through at least one processing station for the treatment or processing; and
a vertical displacement device, which is configured for raising the containers held on the carrier for the treatment or processing at or in the processing station vertically upward to a raised position and for lowering the containers after the treatment or processing at or in the processing station, so that they are held again on the carrier in the regular arrangement;
**characterized in that** a supporting surface is provided at an upper end of the vertical displacement device on which the respective bottom portion (3) of the container (2) rests during raising of the containers to the raised position, wherein the vertical displacement device further comprises a negative pressure generating device, which is coupled to the vertical displacement device in such manner that the negative pressure acts on the bottom portions (3) of the containers (2) via the supporting surfaces (246) and via suction caps (250) provided in the supporting surfaces to abut against the bottom portions (3) of the containers (2).

13. The apparatus according claim 12, wherein the vertical displacement device is configured to act on bottom portions (3) of the containers (2) and to thereby embrace bottom edges of the containers (2) at least partially.

14. The apparatus according claim 13, wherein the vertical displacement device comprises a plurality of holding arms (245) for embracing the bottom edge of the respective containers (2), wherein the vertical displacement device extends through apertures or receptacles in the carrier, in which the containers are held in their initial position on the carrier, during raising of the containers to the raised position.

15. The apparatus according to any of claims 12 to 14, wherein the vertical displacement device comprises a plurality of lifting rods (240) that are aligned along a line and simultaneously raise a number of containers to the raised position.

16. The apparatus according claim 15, further comprising a positioning device (260, 271) for positioning the lifting rods relative to the carrier (25; 134) together with the containers (2) held thereon.

17. A use of a vertical displacement device for raising and lowering containers (2) that serve for storing, or contain, substances for medical, pharmaceutical or cosmetic applications in a process according to any of claims 1 to 11 or in an apparatus according to any of claims 12 to 16, for
raising the containers (2) vertically upwards to the raised position by means of the vertical displacement device and
retracting the containers to the carrier by means of the negative pressure, wherein
the negative pressure acts on the bottom portions (3) of the containers (2) via the supporting surfaces and via the suction caps provided in the supporting surfaces, when the suction caps abut against the bottom portions (3) of the containers (2).

## Revendications

1. Un procédé pour le traitement ou la manipulation de récipients (2) servant au stockage ou contenant des substances cosmétiques, pharmaceutiques ou médicales, dans lequel les récipients sont transportés automatiquement, au moyen d'un convoyeur, à la suite d'au moins une station de traitement ou passant par elle pour traitement ou manutention, dans lequel procédé :
une pluralité de récipients (2) sont pris en charge par le convoyeur tout en étant maintenus par un support (25; 134) suivant une disposition régulière,
les récipients maintenus sur le support sont soulevés vers une position élevée pour le traitement ou la manutention dans la station de traitement respective, et
sont redescendus à la suite du traitement ou de la manutention, pour être maintenus à nouveau sur le support au sein de la disposition régulière, dans lequel
les récipients (2) sont soulevés verticalement vers la position supérieure au moyen d'un dispositif de déplacement vertical (240),
**caractérisé en ce que** les récipients sont retirés du support au moyen d'une pression négative à la suite du traitement ou de la manutention, dans lequel
la pression négative agit sur les parties inférieures (3) des récipients (2) via des surfaces de support fournie à une extrémité supérieure du dispositif de déplacement vertical, sur lequel les parties inférieures (3) des récipients (2) reposent dans la position relevée lors du levage des récipients (2), et via des capuchons d'aspiration disposés dans la surface de support , venant en bout des parties inférieures (3) des récipients (2).

2. Le procédé selon la revendication 1, dans lequel le dispositif de déplacement vertical empêche tout déplacement latéral des récipients au moins durant le levage des récipients vers la position relevée.

3. Le procédé selon la revendication 2, dans lequel le dispositif de déplacement vertical agit sur des parties inférieures (3) des récipients (2) et entoure au moins partiellement des bords inférieurs des récipients (2), dans lequel le bord inférieur du récipient respectif (2) est entouré par une pluralité de bras de retenue (245), dans lequel le dispositif de déplacement vertical s'étend via des ouvertures ou des réceptacles dans le support, dans lequel les récipients sont maintenus dans leur position initiale sur le support, durant le levage des récipients dans la position relevée.

4. Procédé selon l'une quelconque des revendications précédentes, dans lequel le dispositif de déplacement vertical comprend une pluralité de tiges de levage qui sont alignées le long d'une ligne et qui simultanément soulèvent une rangée de récipients vers la position relevée,
et comportant de préférence le positionnement du support (25, 134) ensemble avec les récipients (2) maintenus vis à vis du dispositif de déplacement vertical.

5. Le procédé selon l'une quelconque des revendications précédentes, dans lequel les récipients restent maintenus ou au moins guidés dans des ouvertures ou des réceptacles dans la position relevée, qui sont fournis dans le support et qui définissent une disposition régulière, pour le traitement ou la manutention au sein de la station de traitement.

6. Le procédé selon la revendication 5, dans lequel les récipients sont en outre maintenus ou au moins guidés dans les ouvertures ou les réceptacles en position relevée, mais sont supportés sur une surface de support supplémentaire ou sont maintenus au moyen d'un dispositif de maintien additionnel ou par un dispositif de préhension, pour être traités dans la station de traitement, dans lequel
la surface de support supplémentaire comporte de préférence au moins un plateau tournant, entraîné en rotation, sur lequel les récipients sont mis en votation durant le traitement au sein de la station de traitement alors qu'il sont logés dans les ouvertures ou les réceptacles du support.

7. Le procédé selon l'une des revendications 1 à 4, dans lequel les récipients sont complètement enlevés des ouvertures ou des réceptacles, qui sont fournis sur le support et qui définissent une disposition régulière, pour le traitement au sein de la station de traitement.

8. Le procédé selon l'une quelconque des revendications précédentes, dans lequel au moins quelques unes des stations de traitement sont disposées et fonctionnent dans un environnement stérile.

9. Le procédé selon l'une quelconque des revendications précédentes, dans lequel
les récipients maintenus sur le support sont relevés dans la position relevée pour traitement au sein de la station de traitement respective et pesée au moyen d'un dispositif de pesée (237) entre deux étapes de traitement, dans lequel
le dispositif de pesée (237) est de préférence intégré au sein du dispositif de déplacement vertical utilisé pour relever et abaisser les récipients.

10. Le procédé selon l'une quelconque des revendications précédentes, comprenant en outre une étape de remplissage des récipients (2) avec une substance lors du maintien sur le support, dans laquelle étape :
des capuchons (198) sont poussés sur des bords supérieurs des récipients pendant le maintien sur le support, et
les capuchons (198) et les récipients sont déplacés suivant une direction axiale l'un par rapport à l'autre pour appuyer sur les capuchons des récipients (2) alors que les fonds des récipients (2) sont maintenus sur une surface d'appui, pour le scellement des récipients au moyen des capuchons (198), dans lequel
les volumes internes des récipients (2) communiquent avec l'environnement via les capuchons positionnés sur les bords supérieurs des récipients (2), les récipients sont remplis avec la substance via les capuchons positionnés sur les bords supérieurs des récipients (2) et les récipients (2) sont scellés à la suite de l'appui sur les capuchons (198), ou
le remplissage des récipients (2) avec les substances est réalisé en disposant les capuchons (198) sur les bords supérieurs des récipients et les capuchons (198) sont poussés sur les bords supérieurs des récipients à la suite du remplissage des récipients (2).

11. Le procédé selon la revendication 11, dans lequel
les récipients (2) font l'objet d'un traitement de séchage par le froid à la suite du remplissage alors qu'il sont maintenus par le support en amenant le fond des récipients (2) en contact direct avec une surface de refroidissement ou un doigt de refroidissement d'un séchoir par le froid, dans lequel les volumes internes des récipients (2) communiquent avec l'environnement via les capuchons positionnés sur les bords supérieurs des récipients (2) durant le séchage par le froid, dans lequel
une pluralité de surface de refroidissement ou de doigts de refroidissement du séchoir par le froid ou des surfaces de support ayant les récipients directement en contact sur eux, sont empilés les uns sur les autres et les surfaces de refroidissement ou les doigts de refroidissement du séchoir par le froid ou des surfaces de support sont pressées les uns aux autres pour pousser les capuchons sur les récipients (2) de manière à sceller les récipients (2) avec les capuchons (192).

12. Un dispositif destiné au traitement de récipients (2) servant au stockage de substances à usage médical, pharmaceutique ou cosmétique, comprenant :
au moins une station de traitement, dans ou sur laquelle les récipients (2) font l'objet d'un traitement ;
un convoyeur pour le déplacement d'un porteur (25; 134) sur lequel les récipients sont maintenus pour traitement dans au moins une station de traitement ; et
un dispositif de déplacement vertical, configuré pour relever les récipients maintenus sur le porteur pour le traitement dans la station de traitement vers une position relevé et pour abaisser les récipients à la suite du traitement au sein de la station de traitement, de telle manière à ce qu'ils soient à nouveau sur le porteur dans une disposition régulière ;
**caractérisée en ce que** une surface de support est fournie à une extrémité supérieur du dispositif de déplacement vertical sur laquelle les parties inférieures respectives (3) des récipients (2) reposent durant le levage des récipients vers la position relevée, dans lequel le dispositif de déplacement vertical comporte en outre un dispositif de génération de pression négative, qui est couplée au dispositif de déplacement vertical de manière à ce que la pression négative agit sur les parties inférieures (3) des récipients (2) via des surfaces de support (246) et via des capuchons de succion (250) fourni dans les surfaces de support venant en contact contre les parties inférieures (3) des récipients (2).

13. Le dispositif selon la revendication 12, dans lequel le dispositif de déplacement vertical est configuré pour agir sur les parties inférieures (3) des récipients et pour entourer au moins partiellement les bords inférieurs des récipients (2).

14. Le dispositif selon la revendication 13, dans lequel le dispositif de déplacement vertical comporte une pluralité de bras de retenue (245) pour entourer le bord inférieur des récipients respectif (2), dans lequel le dispositif de déplacement vertical s'étend au travers d'ouvertures ou des réceptacles dans le porteur, dans lequel les récipients sont maintenus dans leur position initiale sur le porteur, durant le levage des récipients dans la position relevée.

15. Le dispositif selon l'une quelconque des revendications 12 à 14, dans lequel le dispositif de déplacement vertical comprend une pluralité de tiges de levage (240) qui sont alignées le long d'une ligne et qui simultanément soulèvent une rangée de récipients vers la position relevée.

16. Le dispositif selon la revendication 15, comprenant en outre un dispositif de positionnement (260, 271) pour le positionnement des tiges de levage relativement au porteur (25, 134) avec les récipients (2) retenus ensemble.

17. Une utilisation d'un dispositif de déplacement vertical pour le levage et l'abaissement de récipients (2) servant au stockage de substances pour des applications cosmétiques, pharmaceutiques ou médicales au sein d'un procédé selon l'une quelconque des revendications 1 à 11 ou au sein d'un dispositif selon une quelconque des revendications 12 à 16, pour
le levage des récipients (2) vers la position relevée au moyen du dispositif de déplacement vertical et
le retrait des récipients du porteur au moyen d'une pression négative, dans laquelle
la pression négative agit sur les parties inférieures (3) des récipients (2) via des surfaces de support et via des capuchons de succion fournis dans les surfaces de support, lorsque les capuchons de succion viennent en butée contre les parties inférieures (3) des récipients (2).
